# EUROPEAN PATENT APPLICATION

(11) **EP 4 678 087 A1**
(43) Date of publication of application: **14.01.2026**
(21) Application number: 24766807.2
(22) Date of filing: 15.02.2024
(51) Int. Cl.: A61B 1/00, A61B 1/045, A61B 1/06

(54) **MEDICAL OBSERVATION SYSTEM AND MEDICAL OBSERVATION METHOD**

(30) Priority: 03.03.2023 JP 2023033055; 29.03.2023 JP 2023054252
(71) Applicant: Sony Olympus Medical Solutions Inc., Hachioji-shi, Tokyo 192-0904 (JP)
(72) Inventor: DEGUCHI Tatsuya, Hachioji-shi, Tokyo 192-0904 (JP); YAMAMOTO Takahiro, Hachioji-shi, Tokyo 192-0904 (JP)
(74) Representative: D Young & Co LLP
(86) International application number: PCT/JP2024/005183
(87) International publication number: WO 2024/185435

(57) **Abstract**

Provided is a technique advantageous for observing an observation target via a plurality of types of observation light having different wavelength bands.

A medical observation system includes: a light source apparatus that emits broadband light of a first wavelength band, first narrowband light that excites a first substance that emits first fluorescence of a wavelength band included in the first wavelength band, and second narrowband light that excites a second substance that emits second fluorescence of a wavelength band not included in the first wavelength band; and a control section that controls the light source apparatus, in which the control section is configured to: control the light source apparatus such that the broadband light and the first narrowband light are emitted to an observation target in a time division manner in a first mode; and control the light source apparatus such that the broadband light and the second narrowband light are emitted to the observation target in a second mode different from the first mode.

## Description

### TECHNICAL FIELD

The present disclosure relates to a medical observation system and a medical observation method.

### BACKGROUND ART

Demand for fluorescence observation for determining a state of a biological tissue by irradiating the biological tissue (living tissue) containing a fluorescent substance with excitation light and observing fluorescence emitted from the biological tissue is expanding (see, for example, Patent Document 1).

According to such fluorescence observation, it is possible to grasp a tissue state that is difficult to recognize in a case of observing reflected light of visible light such as white light with which a biological tissue to be observed is irradiated via fluorescence. Therefore, fluorescence observation can be used for various purposes and applications such as identification of a lesion.

### CITATION LIST

### PATENT DOCUMENT

Patent Document 1: Japanese Patent Application Laid-Open No. 2021-132695

### SUMMARY OF THE INVENTION

### PROBLEMS TO BE SOLVED BY THE INVENTION

In the fluorescence observation, various kinds of agents according to the characteristics of the biological tissue to be observed can be used, and for example, an agent that emits fluorescence in a visible light wavelength band or an agent that emits fluorescence in a wavelength band other than the visible light wavelength band can be injected into the biological tissue.

In addition, the fluorescence observation is performed in combination with the reflected light observation of observing the reflected light of the visible light with which the observation target is irradiated, whereby the state of the observation target can be determined more accurately and easily.

By observing the target tissue using the observation light of various wavelength bands in this manner, it is possible to determine the state and characteristics of the observation target in a multifaceted and comprehensive manner.

The present disclosure provides a technique advantageous for observing an observation target via a plurality of types of observation light having different wavelength bands.

### SOLUTIONS TO PROBLEMS

An aspect of the present disclosure relates to a medical observation system including: a light source apparatus that emits broadband light of a first wavelength band, first narrowband light that excites a first substance that emits first fluorescence of a wavelength band included in the first wavelength band, and second narrowband light that excites a second substance that emits second fluorescence of a wavelength band not included in the first wavelength band; and a control section that controls the light source apparatus, in which the control section is configured to: control the light source apparatus such that the broadband light and the first narrowband light are emitted to an observation target in a time division manner in a first mode; and control the light source apparatus such that the broadband light and the second narrowband light are emitted to the observation target in a second mode different from the first mode.

Another aspect of the present disclosure relates to a medical observation method including: a step of emitting, from a light source apparatus, at least one of broadband light in a first wavelength band, first narrowband light that excites a first substance that emits first fluorescence in a wavelength band included in the first wavelength band, or second narrowband light that excites a second substance that emits second fluorescence in a wavelength band not included in the first wavelength band, in which in a first mode, the broadband light and the first narrowband light are emitted from the light source apparatus such that the broadband light and the first narrowband light are emitted to an observation target in a time division manner, and in a second mode different from the first mode, the broadband light and the second narrowband light are emitted from the light source apparatus such that the observation target is irradiated with the broadband light and the second narrowband light.

### BRIEF DESCRIPTION OF DRAWINGS

Fig. 1A is a diagram illustrating an example of a medical observation system.
Fig. 1B is a diagram illustrating another example of the medical observation system.
Fig. 2 is a diagram illustrating a schematic configuration of an example of a light source apparatus.
Fig. 3 is a diagram illustrating a schematic configuration of an example of an imaging system of a camera head.
Fig. 4 is a diagram illustrating a schematic configuration of another example of an imaging system of a camera head.
Fig. 5 is a diagram illustrating a schematic configuration of another example of an imaging system of a camera head.
Fig. 6 is a diagram illustrating a schematic configuration of another example of an imaging system of a camera head.
Fig. 7 is a block diagram illustrating a configuration example of a camera head and a control apparatus.
Fig. 8 illustrates an example of an output image displayed on a display apparatus.
Fig. 9 illustrates another example of the output image displayed on the display apparatus.
Fig. 10 illustrates another example of the output image displayed on the display apparatus.
Fig. 11 illustrates another example of the output image displayed on the display apparatus.
Fig. 12 is a diagram for explaining types of light incident on the imaging element (first imaging element and second imaging element) according to the first embodiment.
Fig. 13 illustrates an example of a timing chart of light source light emission and imaging element exposure in the first mode of the first embodiment.
Fig. 14 illustrates an example of a timing chart of light source light emission and imaging element exposure in the second mode of the first embodiment.
Fig. 15 illustrates another example of a timing chart of light source light emission and imaging element exposure in the second mode of the first embodiment.
Fig. 16 is a diagram for explaining types of light incident on the imaging element (first imaging element and second imaging element) according to the second embodiment.
Fig. 17 is a diagram for explaining types of light incident on the imaging element (first imaging element and second imaging element) according to the third embodiment.
Fig. 18 illustrates an example of a timing chart of light source light emission and imaging element exposure in the first mode of the third embodiment.
Fig. 19 is a diagram for explaining types of light incident on the imaging element (first imaging element and second imaging element) according to the fourth embodiment.
Fig. 20 is a diagram for explaining types of light incident on the imaging element (first imaging element and second imaging element) according to the fifth embodiment.
Fig. 21 is a diagram for explaining types of light incident on an imaging element (first imaging element, second imaging element, and third imaging element) according to the sixth embodiment.
Fig. 22 illustrates an example of a timing chart of light source light emission and imaging element exposure in the third mode of the seventh embodiment.
Fig. 23 is a diagram for explaining types of light incident on an imaging element (first imaging element, second imaging element, and third imaging element) according to the eighth embodiment.
Fig. 24 is a diagram for explaining types of light incident on an imaging element (first imaging element, second imaging element, and third imaging element) according to the ninth embodiment.
Fig. 25 illustrates an example of a timing chart of light source light emission and imaging element exposure in the fifth mode of the ninth embodiment.
Fig. 26 is a diagram for explaining types of light incident on an imaging element (first imaging element, second imaging element, and third imaging element) according to the 10th embodiment.
Fig. 27 is a flowchart illustrating an example of a medical observation method performed by the medical observation system.
Fig. 28 illustrates an example of a physical size relationship between an imaging region (effective pixel region) of a first imaging element and an imaging region (effective pixel region) of a second imaging element.
Fig. 29 is a schematic diagram illustrating an example of a first image generated on the basis of an image signal from the first imaging element illustrated in Fig. 28.
Fig. 30 is a diagram for explaining an example of generation processing of a first image, a second image, and a superimposed image in the first image generation example.
Fig. 31 illustrates an example of a first output image generated mainly from a first image (normal light captured image) which is a captured image by the first imaging element.
Fig. 32 illustrates an example of a second output image mainly generated from a second image (fluorescence captured image) which is a captured image by a second imaging element.
Fig. 33 is a diagram for explaining an example of output image generation processing in the first image generation example.
Fig. 34A is a flowchart illustrating a generation processing example of a superimposed output image.
Fig. 34B is a flowchart illustrating a generation processing example of a superimposed output image.
Fig. 34C is a flowchart illustrating a generation processing example of a superimposed output image.
Fig. 35 is a diagram for explaining an example of generation processing of a first image, a second image, and a superimposed image in the second image generation example.
Fig. 36 is a diagram for explaining an example of output image generation processing in the second image generation example.
Fig. 37 is a conceptual diagram illustrating an example of a medical observation system configured as an operative field illumination observation apparatus in which a ring light (open field illumination apparatus for living body observation) is connected to a light source apparatus.
Fig. 38 is a diagram illustrating an example of a medical observation system configured as a microscope system.

### MODE FOR CARRYING OUT THE INVENTION

Hereinafter, embodiments of the present disclosure will be described with reference to the drawings. The same or corresponding elements are denoted by the same reference signs, and the detailed description thereof will be omitted as appropriate. In addition, the terms "first", "second", and the like do not define a specific order, and do not represent importance, and are used merely for the purpose of distinction, unless otherwise noted.

Hereinafter, a case where the present disclosure technology is applied to an endoscope apparatus will be exemplified. However, an application target of the present disclosure technology is not limited, and the present disclosure technology can also be applied to a system, an apparatus, and a method for medical observation other than the endoscope apparatus. The term "medical care" as used herein is interpreted in a broad sense, and is a concept that can include not only treatment of diseases and injuries but also various actions for the purpose of maintaining, recovering, or promoting health, and an action for the main purpose of research is also included in the concept of "medical care".

### [Medical Observation System]

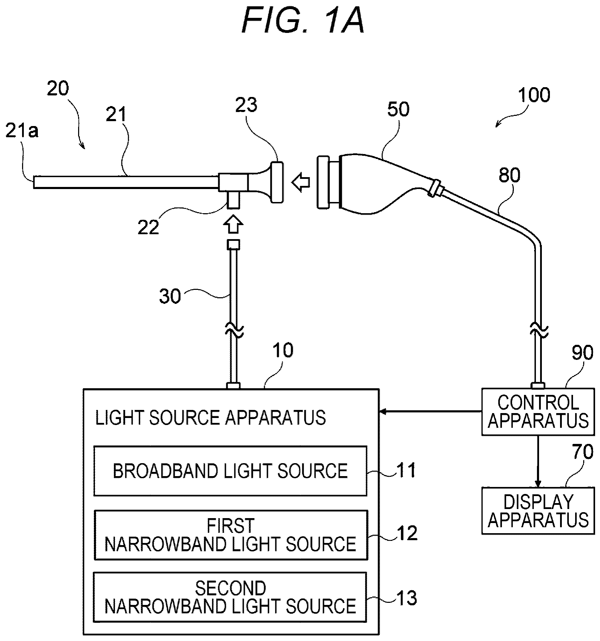

Fig. 1A is a diagram illustrating an example of a medical observation system 100.

A medical observation system 100 illustrated in Fig. 1A is configured as an endoscope apparatus (endoscope system) for observing light (that is, observation light) from a subject as an observation target via a captured image. However, the medical observation system 100 of the present example can directly visually recognize the observation light with the naked eye without passing through the captured image as described later.

The medical observation system 100 illustrated in Fig. 1A includes a light source apparatus 10, an insertion device 20 (endoscope main body), a light guide 30, a camera head (imaging section) 50, a display apparatus 70, a transmission cable 80, and a control apparatus 90.

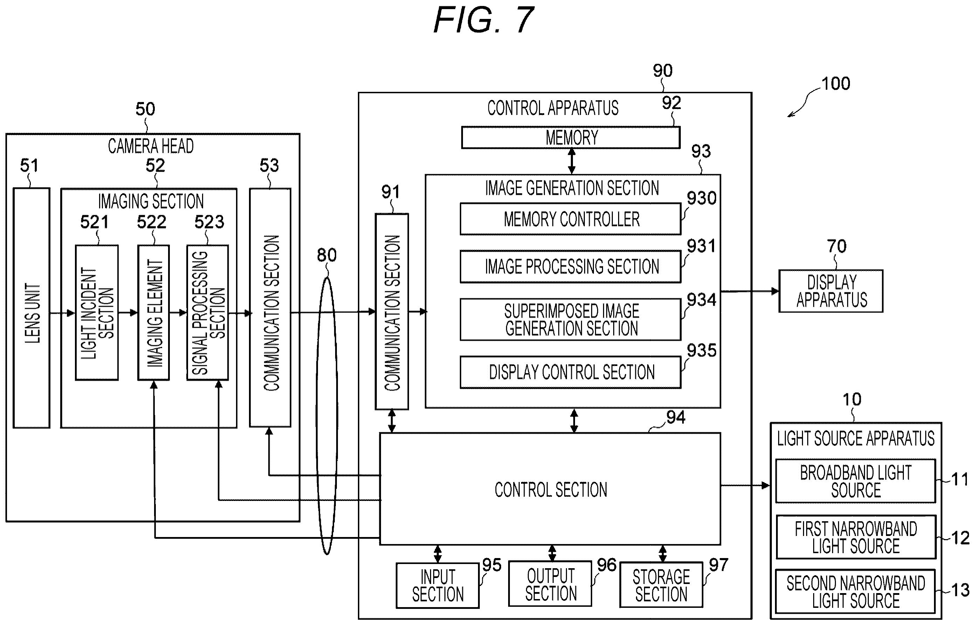

The light source apparatus 10 is an apparatus that emits light to be applied to a subject to be observed, and emits light under the control of the control apparatus 90 (particularly, a "control section" described later (see Fig. 7)). The light source apparatus 10 can emit a plurality of types of light having different wavelength bands, and can emit one or both of visible light (white light or the like) and invisible light (infrared light, ultraviolet light, or the like), for example.

The light source apparatus 10 illustrated in Fig. 1A includes a broadband light source (first light source) 11 that emits broadband light, a first narrowband light source (second light source) 12 that emits first narrowband light, and a second narrowband light source (third light source) 13 that emits second narrowband light.

The broadband light emitted from the broadband light source 11 includes light of a relatively wide wavelength band (that is, a first wavelength band) as a main light component with respect to the narrowband light. The first wavelength band of the broadband light may be a continuous single wavelength band or may include a plurality of discrete wavelength bands.

On the other hand, the first narrowband light and the second narrowband light emitted from each of the first narrowband light source 12 and the second narrowband light source 13 include light in a relatively narrow wavelength band as a main light component with respect to the broadband light. The first narrowband light is partially or entirely included in a first wavelength band that is a wavelength band of broadband light, has a bandwidth narrower than the first wavelength band, and can act as light that excites a first substance that emits first fluorescence. The first fluorescence also has a bandwidth narrower than the first wavelength band.

The entire wavelength band of the second narrowband light is not included in the first wavelength band and has a bandwidth narrower than the first wavelength band, and can act as light that excites the second substance that emits the second fluorescence. The wavelength band of the second narrowband light may be a wavelength band on a longer wavelength side or a wavelength band on a shorter wavelength side than the first wavelength band of the broadband light and the wavelength band of the first narrowband light. The second fluorescence also has a bandwidth narrower than the first wavelength band, and may be a wavelength band on a longer wavelength side or a wavelength band on a shorter wavelength side than the first wavelength band of the broadband light and the wavelength band of the first narrowband light.

The light source apparatus 10 is connected to the insertion device 20 via the light guide 30, and light emitted from the light source apparatus 10 is transmitted to the insertion device 20 via the light guide 30. The light guide 30 of the present example is detachably connected to the light source apparatus 10 and the insertion device 20.

The insertion device 20 includes an insertion portion 21, and an optical connection portion 22 and an imaging connection portion 23 provided on the proximal end side of the insertion portion 21. The insertion device 20 illustrated in Fig. 1A is configured as a rigid endoscope, and the insertion portion 21 has a rigid and elongated shape. However, the insertion device 20 can have any structure, and may be configured as, for example, a flexible endoscope having a flexible insertion portion 21.

A light transmission portion (light guide) and an objective lens are provided on an end surface of the distal end portion 21a of the insertion portion 21 located on the side opposite to the proximal end side. The light transmitted from the light source apparatus 10 to the insertion device 20 via the light guide 30 is emitted from the light transmission portion on the end surface of the distal end portion 21a of the insertion portion 21 toward the observation target. Then, light from the observation target enters the objective lens, and is guided to the imaging connection portion 23 through the inside of the insertion portion 21. In this manner, the entire light from the observation target guided to the imaging connection portion 23 via the objective lens and the inside of the insertion portion 21 is referred to as observation light, and for example, reflected light from the observation target and fluorescence emitted from the observation target can be included in the observation light.

The imaging connection portion 23 is detachably connected to the connection portion of the camera head 50. Observation light transmitted through the objective lens enters the camera head 50 through the imaging connection portion 23 and is received by the camera head 50.

Note that the imaging connection portion 23 can also function as an eyepiece portion. That is, in a state where the imaging connection portion 23 is detached from the camera head 50, the operator of the insertion device 20 can directly view the observation light via the imaging connection portion 23.

The camera head 50 is an imaging apparatus that is detachably connected to the insertion device 20 and receives observation light transmitted via the insertion device 20, and captures an image of an observation target irradiated with light emitted by the light source apparatus 10 to acquire an image. The camera head 50 is connected to the control apparatus 90 via the transmission cable 80.

The transmission cable 80 can transmit various signals (for example, an image signal, a control signal, a synchronization signal, and a clock signal) and power between the camera head 50 and the control apparatus 90. The camera head 50 outputs an image signal corresponding to the received observation light, and the image signal is transmitted from the camera head 50 to the control apparatus 90 via the transmission cable 80.

A signal transmission method in the transmission cable 80 is not limited, and various signals can be transmitted via the transmission cable 80 as an electric signal or an optical signal. In addition, in place of the wired signal transmission method via the transmission cable 80, various signals may be transmitted between the camera head 50 and the control apparatus 90 by a wireless signal transmission method (for example, wireless local area network (LAN), Bluetooth (registered trademark), infrared communication, and the like).

The control apparatus 90 is connected to the camera head 50, the light source apparatus 10, and the display apparatus 70 in a wired or wireless manner, and integrally controls the camera head 50, the light source apparatus 10, and the display apparatus 70. For example, the control apparatus 90 controls light emission of the light source apparatus 10 as described later, or generates an image from an image signal transmitted from the camera head 50 and causes the display apparatus 70 to display the image. Furthermore, the control apparatus 90 can also control the insertion device 20 connected to the camera head 50 via the camera head 50.

Note that the control apparatus 90 is provided separately from the light source apparatus 10, the camera head 50, and the display apparatus 70 in the example illustrated in Fig. 1A, but may be provided integrally with the light source apparatus 10, the camera head 50, and/or the display apparatus 70.

The display apparatus 70 includes an arbitrarily configured display (for example, a liquid crystal display or an organic electro-luminescence (EL) display), and displays an image on the display under the control of the control apparatus 90.

Although the display apparatus 70 is illustrated as a single block in Fig. 1A, one or a plurality of display apparatuses 70 can be connected to the control apparatus 90 (image generation apparatus). In a case where the plurality of display apparatuses 70 is connected to the control apparatus 90, the same output image may be synchronously displayed on the plurality of display apparatuses 70, or different output images may be synchronously displayed on the plurality of display apparatuses 70. For example, while an output image in which the entire observation target is captured in a bird's-eye view is displayed on a certain display apparatus 70, an output image in which the entire or a part of the observation target is enlarged and captured may be displayed on another display apparatus 70.

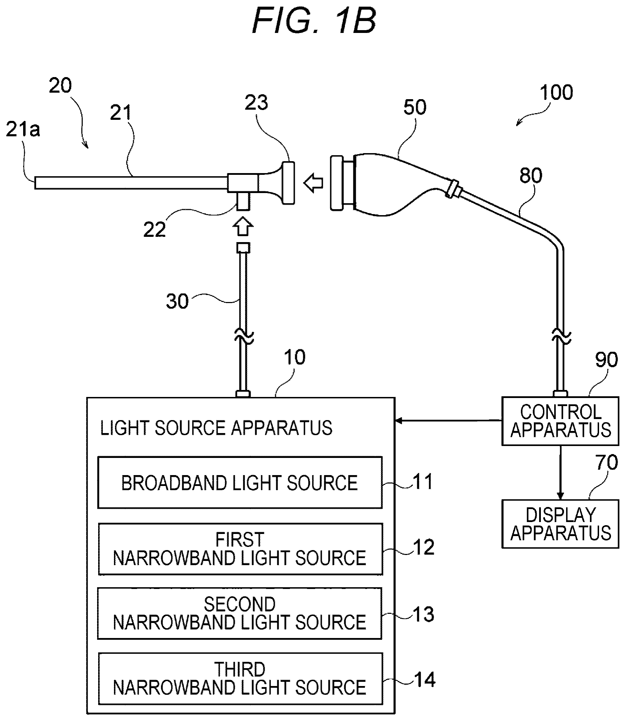

Fig. 1B is a diagram illustrating another example of the medical observation system 100. The medical observation system 100 illustrated in Fig. 1B further includes a third narrowband light source (fourth light source) 14 that emits third narrowband light in addition to the broadband light source 11, the first narrowband light source 12, and the second narrowband light source 13. Other configurations of the medical observation system 100 illustrated in Fig. 1B are similar to those of the medical observation system 100 illustrated in Fig. 1A described above.

The entire wavelength band of the third narrowband light is not included in the first wavelength band and has a bandwidth narrower than the first wavelength band, and can act as light that excites a third substance that emits third fluorescence in a wavelength band at least partially different from the wavelength band of the second fluorescence. The wavelength band of the third narrowband light may be a wavelength band on a longer wavelength side or a wavelength band on a shorter wavelength side than the first wavelength band of the broadband light and the wavelength band of the first narrowband light. The wavelength band of the second narrowband light and the wavelength band of the third narrowband light may partially overlap each other or may not overlap each other at all. The third fluorescence also has a bandwidth narrower than the first wavelength band, and may be a wavelength band on a longer wavelength side or a wavelength band on a shorter wavelength side than the first wavelength band of the broadband light and the wavelength band of the first narrowband light.

### [Light Source Apparatus]

Next, a device configuration example of the above-described light source apparatus 10 will be described.

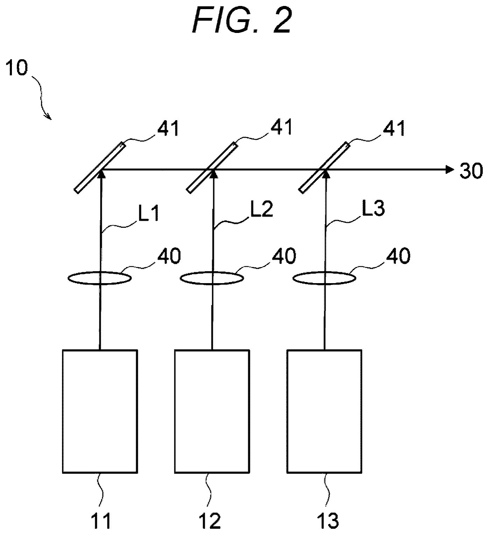

Fig. 2 is a diagram illustrating a schematic configuration of an example of the light source apparatus 10. In the light source apparatus 10 illustrated in Fig. 2, a lens optical system 40 and a mirror optical system 41 are provided for each of the broadband light source 11, the first narrowband light source 12, and the second narrowband light source 13.

The lens optical system 40 includes a collimator lens that converts incident light into parallel light. The mirror optical system 41 reflects light incident through the lens optical system 40 and transmits light incident through other mirror optical system 41.

The broadband light L1 emitted from the broadband light source 11 is converted into parallel light by the corresponding lens optical system 40, then reflected by the corresponding mirror optical system 41, transmitted through the mirror optical system 41 associated with the other light sources 12 and 13, and incident on the light guide 30. The first narrowband light L2 emitted from the first narrowband light source 12 is converted into parallel light by the corresponding lens optical system 40, then reflected by the corresponding mirror optical system 41, transmitted through the mirror optical system 41 associated with the second narrowband light source 13, and incident on the light guide 30. The second narrowband light L3 emitted from the second narrowband light source 13 is converted into parallel light by the corresponding lens optical system 40, then reflected by the corresponding mirror optical system 41, and incident on the light guide 30.

In particular, in the light source apparatus 10 of the present example, the broadband light L1, the first narrowband light L2, and the second narrowband light L3 emitted from the respective light sources 11, 12, and 13 are reflected by the corresponding mirror optical system 41, and then enter the light guide 30 through a common optical path. Therefore, in a case where light emission is simultaneously performed in two or more of the broadband light source 11, the first narrowband light source 12, and the second narrowband light source 13, a plurality of types of light is incident on the light guide 30, and the observation target is irradiated with the light.

Note that the light source apparatus 10 illustrated in Fig. 2 is merely an example, and the light source apparatus 10 can take any other configuration, and for example, the third narrowband light source 14 (see Fig. 1B) may be provided. Further, specific device configurations of the broadband light source 11, the first narrowband light source 12, the second narrowband light source 13, and the third narrowband light source 14 are not limited, and each of the light sources 11, 12, 13, and 14 may have a single light emitting device or may have a plurality of light emitting devices.

In addition, the wavelength bands of the broadband light L1, the first narrowband light L2, the second narrowband light L3, and the third narrowband light source 14 emitted by the broadband light source 11, the first narrowband light source 12, the second narrowband light source 13, and the third narrowband light source 14 are also not limited.

For example, the broadband light source 11 may emit visible light as the broadband light L1 or may emit light in a wavelength band corresponding to a color gamut displayable by the display apparatus 70. Visible light is light perceptible to healthy human eyes and may have a wavelength band of 380 to 780 nm as an example, with an upper wavelength limit of 760 to 830 nm and a lower wavelength limit of 360 to 400 nm. Note that, here, light other than visible light is referred to as invisible light, and a wavelength band other than the visible light wavelength band is referred to as an invisible light wavelength band.

For example, the broadband light source 11 may emit white light as the broadband light L1, and may be configured by a light-emitting diode (LED), a xenon lamp, or any other device. The broadband light source 11 may be configured by a white LED that is a monochromatic light emitting device, or may be configured by a plurality of colored LEDs (for example, a red LED, a green LED, and a blue LED) that emit light of different colors. In addition, the broadband light source 11 may include a white LED and a plurality of colored LEDs that emit colored light other than white light.

Furthermore, the light emitted from the broadband light source 11 may include at least a part of the light in the visible light wavelength band, and for example, may include light corresponding to the color gamut of the standard specification of the display apparatus 70, or may include light in a specific wavelength band (for example, violet light or green light).

The first narrowband light source 12 may emit, as the first narrowband light L2, light that excites a first substance that emits first fluorescence in a wavelength band included in a wavelength band (first wavelength band) of the broadband light L1 from the broadband light source 11. On the other hand, the second narrowband light source 13 may emit, as the second narrowband light L3, light that excites a second substance that emits second fluorescence in a wavelength band not included in the wavelength band (first wavelength band) of the broadband light L1 from the broadband light source 11. In addition, the third narrowband light source 14 may emit, as the third narrowband light, light that excites a third substance that emits third fluorescence in a wavelength band not included in the wavelength band (first wavelength band) of the broadband light L1 from the broadband light source 11.

Each of the first narrowband light source 12, the second narrowband light source 13, and the third narrowband light source 14 may include, for example, a laser light source, an LED light source, a xenon lamp, or any other light emitting device. As an example, each of the first narrowband light source 12, the second narrowband light source 13, and the third narrowband light source 14 may be configured by combining such a light emitting device (for example, a xenon lamp) and a filter (band pass filter) that transmits narrowband light of a desired wavelength band from light emitted from the light emitting device.

Note that the broadband light L1 from the broadband light source 11 may be visible light or visible light in a part of the visible light wavelength band. In addition, each of the first fluorescence to the third fluorescence emitted by the first substance to the third substance can emit light in a visible light or invisible light wavelength band (for example, infrared light or ultraviolet light).

Note that the substance excited by the light emitted from the light source apparatus 10 (for example, the first substance to the third substance described above) may be an agent or a fluorescent dye applied to the observation target, or may be a fluorescent substance constituting the observation target itself.

Examples of such an agent that can be imparted to the observation target include 5-ALA (PP-IX), ADS780WS, ADS830WS, aggregation-induced emission dots allophycocyanin (APC), boron-dipyrromethane (BODIPY), CLR 1502, Flavins, fluorescamine, Fluorescein, fluoro-gold, green fluorescence protein, ICG (indocyanine green), IRDye 78, IR-PEG nanoparticles, Isothiocyanate, rose Bengal, trypan blue, and SGM-101.

Examples of the fluorescent dye that can be imparted to the observation target include coumarine, Cy3, DyLight547, GE3126, metal nanoclusters, oxacarbocyanine, Rhodamine, Riboflavin, fluorescein, AlexaFluor 488, AlexaFluor660, AlexaFluor680, AlexaFluor700, Cy5, Cy5.5, Dy677, Dy682, Dy752, DyLight647, HiLyte Fluor 647, HiLyte Fluor 680, IRDye 700DX, methylene blue, Porphyrins, Porphysomes, VivoTag-680, VivoTag-S680, AlexaFluor750, AlexaFluor790, carbocyanine, conjugated copolymers, CW800-CA, Cy7, Cy7.5, cyanine dyes, Dy780, HiLyte Fluor 750, Indocarbocyanine, IR-786, IRDye 800CW, IRDye 800RS, IRDye 800BK, Nervelight^{™}, OTL-38, Polymethine, VivoTag-S750, ASP5354, and Xanthene.

Examples of the fluorescent substance derived from the observation target constituting the observation target itself include collagen, elastin, and NADH.

### [Camera Head]

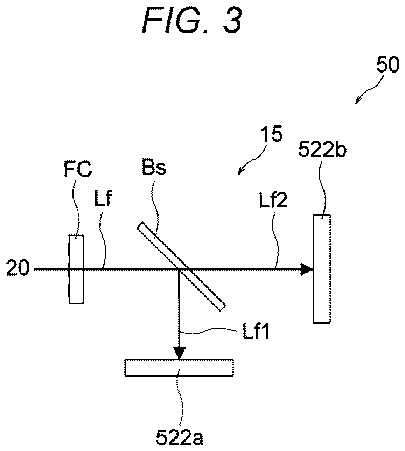

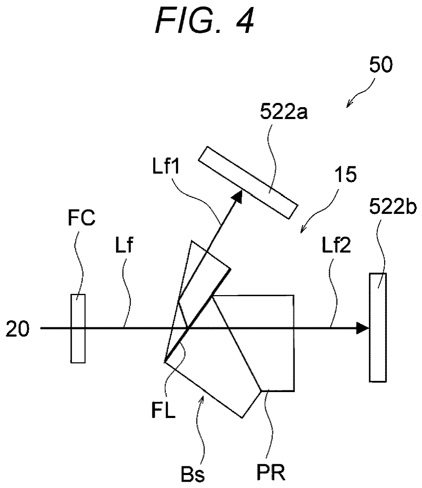

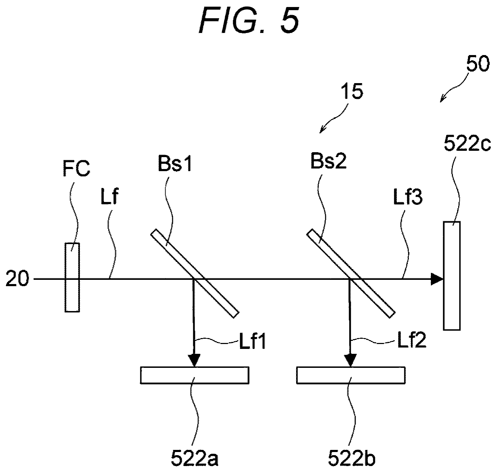

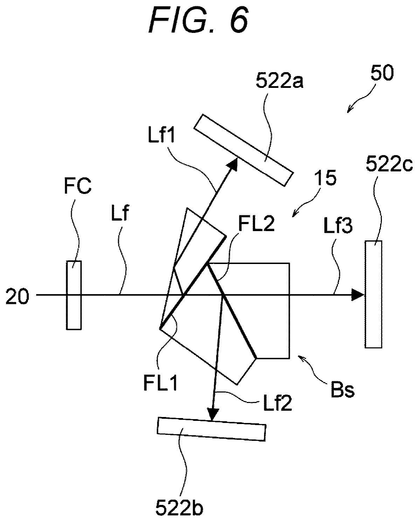

Next, a configuration example of an imaging system of the above-described camera head 50 will be described. Hereinafter, a typical example of a two-plate type imaging module (see Figs. 3 and 4) that performs imaging using two imaging elements and a typical example of a so-called three-plate type imaging module (see Figs. 5 and 6) that performs imaging using three imaging elements will be described.

Fig. 3 is a diagram illustrating a schematic configuration of an example of an imaging system of the camera head 50.

The camera head 50 illustrated in Fig. 3 includes an excitation light cut filter FC, a branching optical system Bs, a first imaging element 522a, and a second imaging element 522b.

The observation light Lf transmitted through the insertion device 20 is incident on the branching optical system Bs after light in a predetermined wavelength band is cut by the excitation light cut filter FC.

The wavelength band of the light cut by the excitation light cut filter FC includes the wavelength band of the excitation light that may be emitted to the observation target. For example, in a case where there is a possibility that the observation target is irradiated with the first narrowband light to the third narrowband light from the first narrowband light source 12 to the third narrowband light source 14 as the excitation light, the excitation light cut filter FC partially, substantially, or completely suppresses at least the light in the wavelength bands of the first narrowband light, the second narrowband light, and the third narrowband light. The excitation light cut filter FC can be configured by a known wavelength selection filter or the like, and prevents reflected light of the excitation light with which the observation target is irradiated from being received by the imaging element (in the present example, the first imaging element 522a and the second imaging element 522b).

Note that, although Fig. 3 illustrates the excitation light cut filter FC as a single unit, the excitation light cut filter FC may be configured by a single filter or a plurality of filters. For example, in a case where there is a possibility that the observation target is irradiated with a plurality of types of excitation light having different wavelength bands, the excitation light cut filter FC may be a single filter that partially, substantially, or completely suppresses light in the wavelength bands of these excitation lights, or may include a plurality of filters that partially, substantially, or completely suppresses light in the wavelength bands of the respective excitation lights.

Furthermore, the excitation light cut filter FC is not limited to the position illustrated in Fig. 3, and can be installed at an arbitrary position on the optical path of the observation light Lf (including the light flux after separation) from the observation target to the imaging element (the first imaging element 522a and the second imaging element 522b in the present example). That is, the excitation light cut filter FC may be provided in the insertion device 20, and for example, may be provided on the upstream side or the downstream side with respect to the traveling direction of the observation light Lf with respect to the objective lens of the end surface of the distal end portion 21a of the insertion portion 21. In addition, the excitation light cut filter FC may be provided separately from the medical observation system 100.

Note that the excitation light cut filter FC may not be provided. In a case where the influence of the reflected light of the excitation light on the captured image is sufficiently small, the system configuration may be simplified by not providing the excitation light cut filter FC.

The branching optical system Bs is an optical element 15 that separates the observation light Lf from the observation target into a first light flux Lf1 and a second light flux Lf2 (a plurality of light fluxes), guides the first light flux Lf1 to the first imaging element 522a, and guides the second light flux Lf2 to the second imaging element 522b. The branching optical system Bs illustrated in Fig. 3 reflects the first light flux Lf1 toward the first imaging element 522a and transmits the second light flux Lf2 to guide the second light flux Lf2 to the second imaging element 522b.

The branching optical system Bs can be configured on the basis of, for example, a combination of a dichroic mirror and a wavelength selection filter, but a specific configuration of the branching optical system Bs is not limited. The branching optical system Bs may have an optical device that separates (disperses) the incident light into a plurality of light fluxes on the basis of the wavelength, and for example, the light in the visible light wavelength band and the light in the invisible light wavelength band in the incident light may be separated into separate light fluxes. In addition, the branching optical system Bs may include an optical device that separates the incident light into a plurality of light fluxes not based on the wavelength, and for example, the incident light may be separated into a plurality of light fluxes having the same wavelength characteristic.

Note that, in a case where the branching optical system Bs separates the incident light into a plurality of light fluxes on the basis of the wavelength, each light flux after the separation may include light of a wavelength band (that is, an unintended wavelength band) different from a wavelength band of light intended to be included as a main light component in each light flux. For example, in the example illustrated in Fig. 3, in a case where the branching optical system Bs separates the observation light Lf into the first light flux Lf1 in the visible light wavelength band and the second light flux Lf2 in the invisible light wavelength band, the second light flux Lf2 may include visible light corresponding to about 3% to 20% of the light amount in the visible light wavelength band in the observation light Lf.

Note that in a case where the light flux after separation includes light of an unintended wavelength band (also referred to as "leakage light" here), the optical element 15 may include a filter that partially, substantially, or completely removes the leakage light from the light flux. For example, in a case where light included in the separated light flux is weak fluorescence, if the light flux includes leakage light, fluorescence that is an original light receiving target may not be appropriately received by the corresponding imaging element (for example, the second imaging element 522b). In such a case, the leakage light is partially, substantially, or completely removed from the light flux by the filter, whereby the fluorescent light to be received is more appropriately received by the corresponding imaging element.

In addition, in a case where the branching optical system Bs separates the incident light into a plurality of light fluxes without being based on the wavelength, the plurality of light fluxes after the separation may have substantially equal light amounts or unequal light amounts. For example, in the example illustrated in Fig. 3, each of the first light flux Lf1 and the second light flux Lf2 separated by the branching optical system Bs may have a light amount of about 50% of the light amount of the observation light Lf, or may have mutually different light amounts (for example, a light amount difference of about ±10% between the first light flux Lf1 and the second light flux Lf2). For example, the branching optical system Bs may be designed such that the light amount difference between the plurality of light fluxes is determined on the basis of the difference in sensitivity between the imaging elements from which the plurality of light fluxes after separation is received.

As an example, in a case where the ICG applied to the observation target is excited, the branching optical system Bs may include a wavelength selection filter that transmits light in another wavelength band while reflecting light on a shorter wavelength side than the vicinity of 820 to 870 nm, which is the fluorescence wavelength of the ICG.

Fig. 4 is a diagram illustrating a schematic configuration of another example of the imaging system of the camera head 50.

The branching optical system Bs included in the optical element 15 illustrated in Fig. 4 includes a color separation prism PR based on a combination of a plurality of (three) prisms and a wavelength selection filter FL. The wavelength selection filter FL is provided on the junction surface between the first prism located most upstream among the color separation prisms PR and the second prism adjacent to the first prism.

Other configurations are similar to those of the camera head 50 illustrated in Fig. 3 described above.

A part of the observation light Lf (first light flux Lf1) incident on the optical element 15 of the present example is reflected by the junction surface between the first prism and the second prism of the color separation prism PR, and then further reflected to be guided to the first imaging element 522a. On the other hand, at least a part (second light flux Lf2) of the other light of the observation light Lf is transmitted through the optical element 15 and guided to the second imaging element 522b. Note that, by providing an antireflection coating (AR coating) on the junction surface between the second prism and the third prism of the color separation prism PR, reflection of light on the junction surface can be effectively suppressed. Further, the color separation prism PR is not limited to the example illustrated in Fig. 4, and may have any configuration. For example, a two-plate prism formed by combining two prisms (a first prism and a second prism) may be used as the color separation prism PR.

The camera head illustrated in Figs. 3 and 4 described above is a typical example of a two-plate type imaging module that performs imaging using two imaging elements, but the camera head 50 may perform imaging using three or more imaging elements.

Fig. 5 is a diagram illustrating a schematic configuration of another example of the imaging system of the camera head 50.

The camera head 50 illustrated in Fig. 5 has a configuration similar to the camera head 50 illustrated in Fig. 3, but the first branching optical system Bs1 and the second branching optical system Bs2 are provided as the optical element 15, and the observation light Lf is separated into the first light flux Lf1 to the third light flux Lf3.

The observation light Lf having passed through the excitation light cut filter FC is incident on the first branching optical system Bs1. The first branching optical system Bs1 transmits other light in the observation light Lf while reflecting the first light flux Lf1 toward the first imaging element 522a. The observation light Lf transmitted through the first branching optical system Bs1 is incident on the second branching optical system Bs2. The second branching optical system Bs2 reflects the second light flux Lf2 toward the second imaging element 522b and transmits the third light flux Lf3, which is another light in the observation light Lf, to guide the third light flux Lf3 to the third imaging element 522c.

The first branching optical system Bs1 and the second branching optical system Bs2 can be configured on the basis of, for example, a combination of a dichroic mirror and a wavelength selection filter, but specific configurations of the first branching optical system Bs1 and the second branching optical system Bs2 are not limited.

Fig. 6 is a diagram illustrating a schematic configuration of another example of the imaging system of the camera head 50.

The optical element 15 illustrated in Fig. 6 has a configuration similar to the optical element 15 illustrated in Fig. 4, but includes a color separation prism PR, a first wavelength selection filter FL1, and a second wavelength selection filter FL2 as the branching optical system Bs. The first wavelength selection filter FL1 is provided on the junction surface between the first prism located most upstream among the color separation prisms PR and the second prism adjacent to the first prism. The second wavelength selection filter FL2 is provided on the junction surface between the second prism and the third prism adjacent to the second prism.

A part of the observation light Lf incident on the optical element 15 of the present example is reflected by the junction surface between the first prism and the second prism of the color separation prism PR, then further reflected, and emitted from the optical element 15 toward the first imaging element 522a as the first light flux Lf1. On the other hand, the other light of the observation light Lf is transmitted through the junction surface between the first prism and the second prism. Then, a part of the observation light Lf is reflected by the junction surface between the second prism and the third prism, and then emits from the optical element 15 toward the second imaging element 522b as the second light flux Lf2. On the other hand, the other light of the observation light Lf transmits through the junction surface between the second prism and the third prism, and then emits from the optical element 15 toward the third imaging element 522c as the third light flux Lf.

As described above, by using a dichroic mirror or a plurality of prisms as the optical element 15, the observation light Lf can be separated into a plurality of light fluxes (including the first light flux Lf1 and the second light flux Lf2). Then, by appropriately selecting the optical characteristics such as the transmission wavelength band of the wavelength selection filter FL, light in a desired wavelength band can be included in each of the plurality of light fluxes separated from the observation light Lf.

In particular, the two-plate type imaging module (see Figs. 3 and 4) is advantageous for downsizing and cost reduction of the structure of the camera head 50. On the other hand, according to the three-plate type imaging module (see Figs. 5 and 6), three types of light can be simultaneously received by the three imaging elements (the first imaging element 522a to the third imaging element 522c).

### [Functional Configuration of Medical Observation System]

Next, a functional configuration example of the above-described medical observation system 100 will be described.

Fig. 7 is a block diagram illustrating a configuration example of the camera head 50 and the control apparatus 90.

The camera head 50 includes a lens unit 51, an imaging section 52, and a communication section 53.

The lens unit 51 includes one or a plurality of lenses, collects observation light transmitted through the insertion device 20 (see Fig. 1A), and guides the observation light to the imaging section 52.

The imaging section 52 receives the observation light transmitted via the lens unit 51 and outputs a corresponding image signal. The imaging section 52 illustrated in Fig. 7 includes a light incident section 521, an imaging element 522, and a signal processing section 523.

The imaging element 522 is a photoelectric conversion element that receives observation light transmitted via the light incident section 521 and generates an image signal under the control of the control apparatus 90 (particularly, a control section 94 to be described later). The imaging element 522 is a charge coupled device (CCD) image sensor or a complementary metal oxide semiconductor (CMOS) image sensor.

Exposure to the imaging element 522 can be controlled by an arbitrary shutter method, and either a mechanical shutter or an electronic shutter may be used. In each of the embodiments described later, a rolling shutter method is used, but another shutter method (for example, a global shutter method) may be used.

In the above-described examples illustrated in Figs. 3 to 6, a plurality of imaging elements 522 is provided, but the imaging section 52 may include a single imaging element 522. In a case where the imaging section 52 includes the single imaging element 522 and it is not necessary to separate the observation light into a plurality of light fluxes, an optical element (see reference sign "15" in Figs. 3 to 6) for separating the observation light into a plurality of light fluxes is unnecessary.

As described later, by irradiating the observation target with a plurality of types of light having different wavelength bands in a time division manner, a plurality of types of observation light (for example, reflected light and fluorescence of white light) caused by the plurality of types of light can be appropriately received by the single imaging element 522. Furthermore, the single imaging element 522 capable of receiving light of a plurality of types of wavelength bands may be used. For example, like an imaging element also called a direct image sensor, the imaging element 522 that can receive a plurality of types of observation light having different wavelength bands by each of a plurality of light receiving layers arranged in the vertical direction in the imaging element using the vertical color separation characteristic of the imaging element (for example, silicon) may be used. In this case, it is also possible to receive a plurality of light fluxes (in particular, a plurality of light fluxes including light in different wavelength bands) separated from the observation light by each of a plurality of light receiving layers of the single imaging element 522. As an example, the single imaging element 522 including a plurality of light receiving layers capable of receiving visible light (for example, red light, green light, and blue light) and one or a plurality of light receiving layers capable of receiving fluorescence in an invisible light wavelength band (for example, infrared light or ultraviolet light) can receive both a light flux containing the visible light as a main light component and a light flux containing the fluorescence as a main light component.

In a case where a plurality of imaging elements 522 is provided, two or more imaging elements 522 having different characteristics (for example, resolution, sensitivity, pixel size, number of pixels, overall size, color filter, and/or the like) may be provided. Alternatively, two or more imaging elements 522 having the same characteristics may be provided.

The light incident section 521 is an optical system device that guides observation light from the lens unit 51 to the light receiving surface of the imaging element 522. For example, in a case where a plurality of imaging elements 522 is provided, the light incident section 521 includes the above-described optical element 15 (see Figs. 3 to 6) that separates the observation light into a plurality of light fluxes and guides the plurality of light fluxes to the plurality of imaging elements 522.

Note that, in a case where two or more imaging elements 522 having different physical sizes are provided, a lens (angle of view adjustment lens) that optically enlarges or reduces an image so as to align angles of view with each other may be provided for both or one of the two or more imaging elements 522. Such an angle of view adjustment lens may be provided as the light incident section 521 or may be provided as a part of the imaging element 522. By installing the angle of view adjustment lens, images with uniform angles of view can be acquired by two or more imaging elements 522 having different sizes. Therefore, in the generation of the superimposed image to be described later, it is not necessary to display the boundary line between the superimposed region and the non-superimposed region between the images, mask the non-superimposed region, and the like, and the pixel data of the imaging element 522 can be effectively utilized.

The signal processing section 523 performs signal processing (for example, auto gain control (AGC) processing or analog-to-digital (AD) conversion processing) on the image signal generated by the imaging element 522 under the control of the control apparatus 90 (control section 94).

The communication section 53 communicates with the control apparatus 90 (in particular, the communication section 91) via the transmission cable 80 under the control of the control apparatus 90 (in particular, the control section 94). The image signal (digital signal) output from the imaging section 52 is transmitted from the communication section 53 to the control apparatus 90 (in particular, the communication section 91) via the transmission cable 80. The communication standard between the communication section 53 of the camera head 50 and the communication section 91 of the control apparatus 90 is not limited, and the communication section 53 and the communication section 91 may be configured as high-speed serial interfaces.

The control apparatus 90 includes a communication section 91, a memory 92, an image generation section 93, a control section 94, an input section 95, an output section 96, and a storage section 97.

The communication section 91 sends the image signal sent from the camera head 50 via the transmission cable 80 to the image generation section 93 under the control of the control section 94.

The image generation section 93 performs various types of image processing under the control of the control section 94, and generates an image on the basis of an image signal from the imaging section 52. The image generation section 93 illustrated in Fig. 7 includes a memory controller 930, an image processing section 931, a superimposed image generation section 934, and a display control section 935.

The memory controller 930 writes and reads data to and from the memory 92. For example, the memory controller 930 writes an image signal transmitted from the camera head 50 via the communication section 91 into the memory 92 as image data. Then, the memory controller 930 reads image data from the memory 92 as necessary, and provides the image data to the image processing section 931, the superimposed image generation section 934, and/or the display control section 935.

Note that the memory 92 can be configured by, for example, a volatile memory or a non-volatile memory, and can be used as a device capable of temporarily storing various data. Data that can be stored in the memory 92 is not limited. Therefore, the memory controller 930 may write the image data and other data transmitted from the image processing section 931, the superimposed image generation section 934, and/or the display control section 935 in the memory 92 or read them from the memory 92.

The image processing section 931 performs image processing on the image signal transmitted from the camera head 50 and generates a corresponding image (captured image). The image processing section 931 can perform arbitrary image processing. The image processing section 931 may perform, for example, one or more processes of AGC processing, optical black subtraction processing, white balance adjustment processing, demosaic processing, grayscale correction processing, image (video) signal level adjustment processing, color correction processing, gamma correction processing, and fluorescence image signal processing.

The specific configuration of the image processing section 931 is not limited, and the image processing section 931 may include a single processing section regardless of the number of imaging elements 522, or may include a plurality of processing sections corresponding to each of the plurality of imaging elements 522. In a case where a plurality of imaging elements 522 is assigned to one processing section of the image processing section 931, a plurality of corresponding images may be generated by sequentially processing a plurality of image signals from the plurality of imaging elements 522 by the one processing section.

Furthermore, for example, in a case where two imaging elements 522 are provided, the image processing section 931 may include a first processing section that generates an image on the basis of an image signal from one imaging element 522 and a second processing section that generates an image on the basis of an image signal from the other imaging element 522. In a case where the image processing section 931 includes a plurality of processing sections exclusively assigned to each of the plurality of imaging elements 522, it is possible to simultaneously perform image generation processing based on image signals from the plurality of imaging elements 522 in parallel.

The superimposed image generation section 934 combines a plurality of images to generate a superimposed image. The types of the plurality of images as the basis of the superimposed image generated by the superimposed image generation section 934 and the specific composition method are not limited.

For example, a superimposed image may be generated by combining a captured image (also referred to as a "normal light captured image") based on reflected light from the observation target irradiated with white light (broadband light) and a captured image (also referred to as a "fluorescence captured image") based on fluorescence from the observation target irradiated with excitation light (narrowband light). Alternatively, the superimposed image may be generated by combining captured images based on a plurality of fluorescences having different wavelength bands from the common observation target.

The superimposed image generation section 934 may perform the combining processing on the entire region of each of the plurality of images that are the basis of the superimposed image, or may perform the combining processing only on a partial region.

In a case where the total number of pixels (sizes) of the plurality of images to be the basis of the superimposed image is the same and the imaging ranges (including the observation target) included in the plurality of images completely coincide with each other, the superimposed image can be generated by performing the combining processing on the entire region of the plurality of images without performing relative size adjustment and position adjustment between the plurality of images. In addition, in a case where the total number of pixels of the plurality of images to be the basis of the superimposed image is the same as each other, but the imaging ranges (including the observation target) included in the plurality of images do not completely coincide with each other, either relative position adjustment or size adjustment between the plurality of images is performed, and combining processing is performed on images of an imaging range (for example, a portion of the common observation target) common among the plurality of images, whereby the superimposed image can be generated. Furthermore, in a case where the plurality of images to be the basis of the superimposed image includes images of two or more imaging elements having different view angles and total number of pixels from each other, the superimposed image generation section 934 may perform combining processing for generating the superimposed image after adjusting the view angles and the total number of pixels of the images of the two or more imaging elements using an arbitrary image processing technology such as digital zoom.

As an example, a case is assumed where a superimposed image of a first image (for example, a normal light captured image) and a second image (for example, a fluorescence captured image related to fluorescence emitted by a single type of fluorescent substance (for example, a fluorescent agent)) is generated. In this case, if each of the total number of pixels and the imaging range is the same between the first image and the second image, a superimposed image can be generated by superimposing and combining the first image and the second image on each other in the entire region without performing size adjustment and position adjustment. In a case where the total number of pixels is different between the first image and the second image, size adjustment (scaling) of one or both of the first image and the second image is performed, and the first image and the second image after the size adjustment are combined so as to overlap each other, whereby a superimposed image can be generated.

Note that superimposed images of a first image (for example, a normal light captured image), a second image (for example, a fluorescence captured image related to fluorescence emitted from a first fluorescent substance (for example, a first fluorescent agent)), and a third image (for example, a fluorescence captured image related to fluorescence emitted from a second fluorescent substance (for example, a second fluorescent agent)) can also be generated in a manner similar to the superimposed images of the first image and the second image described above. That is, when the total number of pixels and the imaging range are the same among the first to third images, the superimposed image can be generated by superimposing and combining the first to third images on each other in the entire region without performing size adjustment and position adjustment. In addition, in a case where the total number of pixels is different between the first to third images, size adjustment (scaling) of one or more of the first to third images is performed, and the first to third images after the size adjustment are combined so as to overlap each other, whereby a superimposed image can be generated.

The superimposed image generation section 934 may automatically generate a superimposed image of a plurality of mutually related images under the control of the control section 94, or may generate a superimposed image using a plurality of images designated by the user as an original image. The user may designate a plurality of original images via the input section 95, and the control section 94 may control the superimposed image generation section 934 to generate a superimposed image from the plurality of original images designated via the input section 95.

Furthermore, the superimposed image generation section 934 may change the color of the subject including the observation target in the superimposed image under the control of the control section 94. For example, the superimposed image generation section 934 may change the fluorescent portion in the superimposed image to a color with high visibility. The "color with high visibility" mentioned here is a color that is easy for the user viewing the superimposed image to identify, and may be, for example, a color that the observation target originally does not show or a color that is originally not shown so much (for example, yellowish green), or may be a color that is not used in other portions in the superimposed image.

For example, in a case where a superimposed image is generated from a fluorescence captured image based on luminance signal information having no color information and a normal light captured image, the fluorescence captured image may be combined with the normal light captured image as an image having no color information, or may be combined with the normal light captured image after being changed to a certain color (for example, a color with high visibility). Furthermore, in a case where the fluorescence captured image is a color image having color information, the fluorescence captured image may be combined with the normal light captured image by changing the color information to luminance signal information. Furthermore, in order to make the fluorescence captured image superimposed on the normal light captured image easily viewable, the normal light captured image may be changed to a monochrome image based on luminance signal information having no color information, and the fluorescence captured image may be changed to a certain color (for example, a color with high visibility) in the superimposed image.

The display control section 935 generates an output image under the control of the control section 94. The display control section 935 can generate an output image on the basis of the captured image generated by the image processing section 931 and/or the superimposed image generated by the superimposed image generation section 934. The output image generated by the display control section 935 may include only a single image of the captured image and the superimposed image, or may include a plurality of images.

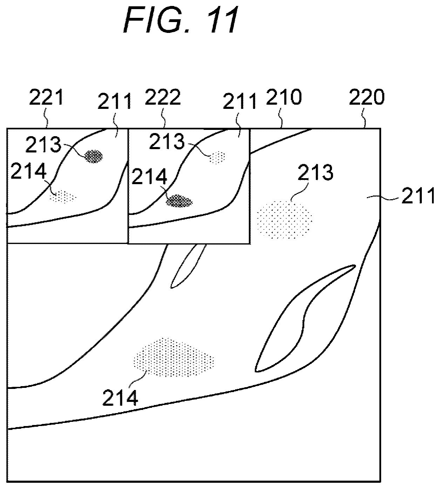

In a case where the output image includes a plurality of images, an arrangement mode of the plurality of images in the output image is not limited. Typically, a picture-in-picture (PiP) in which another image is arranged in a superimposed manner with respect to a certain image (see Fig. 11 described later) or a picture-by-picture (PbP) format in which a plurality of images is arranged can be adopted as the output image. Therefore, the display control section 935 may generate the output image in the PiP format in which one or a plurality of related captured images is reduced and arranged in a partial region of the superimposed image.

In addition, the output image generated by the display control section 935 may be a still image or a moving image (video).

The output image generated by the display control section 935 is transmitted to the display apparatus 70 and displayed on the display of the display apparatus 70. In a case where the plurality of display apparatuses 70 is connected to the control apparatus 90, the display control section 935 generates an output image corresponding to the characteristic of each of the plurality of display apparatuses 70 and outputs the output image to each of the plurality of display apparatuses 70. For example, in a case where two or more display apparatuses 70 having screen resolutions (that is, display total pixels) different from each other are connected to the control apparatus 90, the display control section 935 may generate a plurality of types of output images having the number of pixels corresponding to each of the screen resolutions of the two or more display apparatuses 70. As a result, an output image having an optimized size is displayed on each of the two or more display apparatuses 70.

The display control section 935 may automatically generate the output image under the control of the control section 94, or may generate the output image according to the designation of the user. The user may designate one or a plurality of images to be included in the output image via the input section 95, and the control section 94 may control the display control section 935 to generate the output image from the one or a plurality of images designated via the input section 95 and send the output image to the display apparatus 70. As a result, the user can determine an output image to be displayed on the display apparatus 70 on the basis of his/her own intention, and can switch the output image as necessary.

The control section 94 controls the light source apparatus 10, the camera head 50, and the display apparatus 70, and also controls each section (communication section 91, image generation section 93, input section 95, output section 96, storage section 97, and the like) of the control apparatus 90. The target of the control performed by the control section 94 is not limited. For example, in each embodiment described later, light emission in the light source apparatus 10, reading of image data from the imaging element 522, and image processing in the image generation section 93 are performed under the control of the control section 94.

For example, in a case where the sensitivity of the imaging element 522 is insufficient, the control section 94 may control the imaging element 522 to reduce the frame rate of the image signal output from the imaging element 522, thereby increasing the exposure time in the imaging element 522. Furthermore, the control section 94 may control the image generation section 93 (particularly, the image processing section 931) to add data (pixel values) of pixels of two or more imaging elements in the process of generating a captured image.

On the other hand, in a case of increasing the output frame rate of the imaging element 522, the control section 94 may control the imaging element 522 and the signal processing section 523 to perform thinning reading and region designation reading of pixel data. In this case, data (pixel value) of only a part of the plurality of pixels included in the imaging element 522 is used for image generation.

The input section 95 functions as an instruction receiving section that receives an instruction from the user, receives an instruction from the user under the control of the control section 94, and transmits the instruction to the control section 94. The input section 95 can take any form, and may be configured as a device (for example, a touch panel, an operation button, or the like) directly operated by the user.

Alternatively, the input section 95 may be configured as a connection portion to which a device (for example, a mouse, a keyboard or a portable device) operated by the user is connected in a wired or wireless manner. For example, an instruction from the user input via the operation section of the light source apparatus 10, the operation section of the camera head 50, the operation section of the insertion device 20, and the operation section of the display apparatus 70 may be transmitted to the control section 94 via the input section 95 of the control apparatus 90.

The output section 96 outputs various types of information under the control of the control section 94. The output section 96 can take any form, and may be provided as, for example, a speaker, a printer, a communication apparatus, and/or an application.

The storage section 97 writes and reads various data by the control section 94, and stores, for example, a program executed by the control section 94, information necessary for processing of the control section 94, and the like.

### [Image Display Example]

Next, an image display example in the display apparatus 70 will be described.

Hereinafter, a case where a biological tissue having a first identification target and a second identification target is captured and displayed as an image as an observation target will be exemplified. Here, the identification target is, for example, a lesion, a blood vessel, a nerve, or the like. In particular, the first identification target includes a first substance that is excited by the first narrowband light and emits fluorescence, but does not include a second substance that is excited by the second narrowband light and emits fluorescence. On the other hand, the second identification target does not include the first substance but includes the second substance.

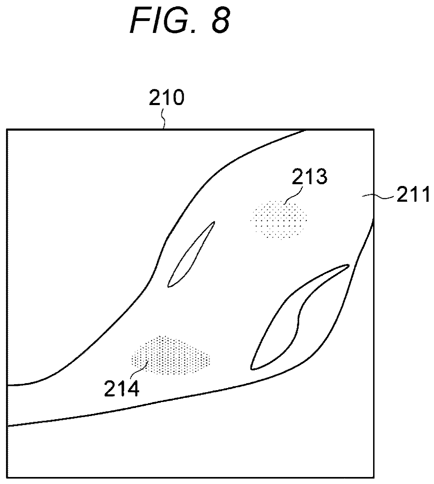

Fig. 8 illustrates an example of an output image 210 displayed on the display apparatus 70. The output image 210 illustrated in Fig. 8 is a captured image based on reflected light of broadband light (in particular, white light) emitted to the observation target.

The first substance in the first identification target and the second substance in the second identification target are not excited or have a weak degree of excitation even when irradiated with white light, and thus do not emit fluorescence or emit only weak fluorescence. Therefore, in the output image 210 illustrated in Fig. 8, the first identification target and the second identification target appearing as a first identification target image 213 and the second identification target image 214 are less likely to be identified than the observation target (for example, the contour) appearing as a clear observation target image 211.

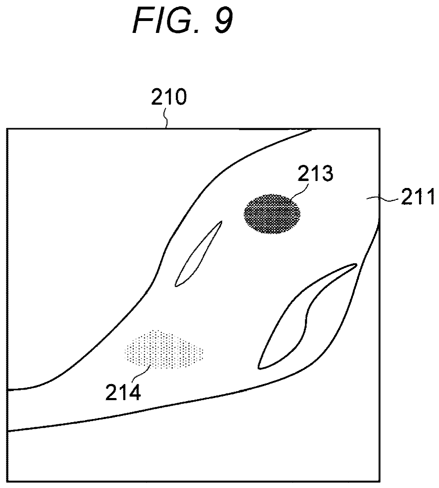

Fig. 9 illustrates another example of the output image 210 displayed on the display apparatus 70. The output image 210 illustrated in Fig. 9 is a superimposed image of a captured image based on reflected light of broadband light (particularly, white light) irradiated to the observation target and a captured image based on fluorescence from the observation target irradiated with the first narrowband light (excitation light).

The first substance in the first identification target is excited by being irradiated with the first narrowband light and emits strong fluorescence. On the other hand, even when the second substance in the second identification target is irradiated with the white light and the first narrowband light, since the second substance is not excited or the degree of excitation is weak, the second substance does not emit fluorescence or emits only weak fluorescence. Therefore, in the output image 210 illustrated in Fig. 9, the second identification target appearing as the second identification target image 214 is less likely to be identified than the observation target (for example, contour) appearing as the clear observation target image 211 and the first identification target image 213 and the first identification target.

Therefore, in the output image 210 of Fig. 9, the user can clearly view the observation target image 211 and the first identification target image 213 at the same time, but it is difficult or impossible to clearly view the second identification target image 214.

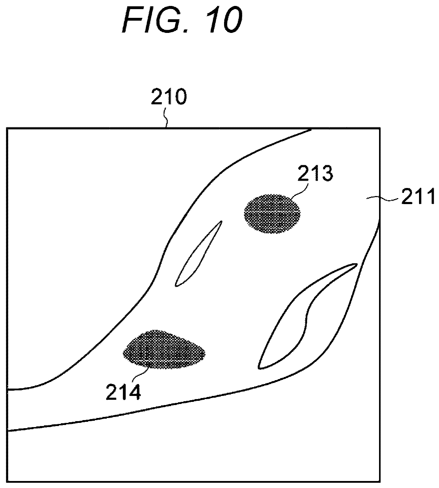

Fig. 10 illustrates another example of the output image 210 displayed on the display apparatus 70. The output image 210 illustrated in Fig. 10 is a superimposed image of a captured image based on reflected light of broadband light (particularly, white light) irradiated to the observation target, a captured image based on fluorescence from the observation target irradiated with the first narrowband light (excitation light), and a captured image based on fluorescence from the observation target irradiated with the second narrowband light (excitation light).

The first substance in the first identification target is excited by being irradiated with the first narrowband light and emits strong fluorescence, and the second substance in the second identification target is excited by being irradiated with the second narrowband light and emits strong fluorescence. Therefore, in the output image 210 illustrated in Fig. 10, the observation target, the first identification target, and the second identification target are illustrated as a clear observation target image 211, a clear first identification target image 213, and a clear second identification target image 214, respectively. Therefore, the user can clearly visually recognize the observation target image 211, the first identification target image 213, and the second identification target image 214 simultaneously in the output image 210 of Fig. 10.

Fig. 11 illustrates another example of the output image displayed on the display apparatus 70. The output image 210 illustrated in Fig. 11 is displayed in the PiP format, and includes a main image 220 and one or a plurality of reduced display images (in the present example, a first reduced display image 221 and a second reduced display image 222) occupying a partial region of the main image 220.

The main image 220 illustrated in Fig. 11 is a captured image (see Fig. 8) based on reflected light of broadband light (particularly, white light) emitted to an observation target. The first reduced display image 221 is a reduced image (see Fig. 9) of a superimposed image of the captured image based on the reflected light of the broadband light (particularly, white light) with which the observation target is irradiated and the captured image based on the fluorescence from the observation target with which the first narrowband light (excitation light) is irradiated. The second reduced display image 222 is a reduced image of a superimposed image of the captured image based on the reflected light of the broadband light (particularly, white light) with which the observation target is irradiated and the captured image based on the fluorescence from the observation target with which the second narrowband light (excitation light) is irradiated.

Note that the image displayed as the output image 210 on the display apparatus 70 (see Figs. 8 to 11) may be changed on the basis of an instruction from the user input via the input section 95 under the control of the control apparatus 90 (particularly, the control section 94 (see Fig. 7)).

For example, the control section 94 may control the display control section 935 to change the images displayed as the main image 220 and the reduced display images 221 and 222 of the output image 210 in PiP format on the basis of an instruction from the user input via the input section 95. In addition, the control section 94 may control the display control section 935 to interchange the image displayed as the main image 220 with the images displayed as the reduced display images 221 and 222 and to interchange the display images between the reduced display images 221 and 222 on the basis of an instruction from the user input via the input section 95.

Furthermore, the control apparatus 90 (particularly, the control section 94) may control the display control section 935 to switch the display format of the output image 210 between the single image display format (see Figs. 8 to 10) and the multiple image display format (see Fig. 11) on the basis of an instruction from the user input via the input section 95.

The user can determine the state and characteristics of the observation target on the basis of the above-described output image 210 (see Figs. 8 to 11) that can be displayed on the display apparatus 70. In particular, the user can accurately and easily determine the state and characteristics of the observation target by simultaneously or switching and confirming a plurality of types of images having different characteristics of observation light related to the same observation target. By displaying the superimposed image obtained by superimposing the single or plurality of fluorescence images on the broadband light image on the display apparatus 70 in this manner, the user can confirm the position of the blood vessel, the lesion, or the like to be identified at the time of surgery on the basis of the fluorescence image while grasping the entire affected part to be observed on the basis of the broadband light image in the superimposed image, and it is possible to support smooth surgery.

### [Example of Observation Method]

Next, a typical embodiment of a method (medical observation method) of observing an observation target using the medical observation system 100 will be described.

The following first to fifth embodiments are typical examples in a case where a two-plate type imaging module (see Figs. 3 and 4) in which the imaging section 52 of the camera head 50 includes two imaging elements 522 is used. On the other hand, the fifth to 10th embodiments are typical examples in a case where a three-plate type imaging module (see Figs. 5 and 6) in which the imaging section 52 of the camera head 50 includes three imaging elements 522 is used.

Hereinafter, a representative observation mode performed in each embodiment will be described. In each embodiment, other observation modes not mentioned below can also be performed by the medical observation system 100.

The observation mode can be switched on the basis of an instruction from a user such as an operator of the medical observation system 100. When the user inputs an instruction indicating a desired observation mode via the input section 95 of the control apparatus 90, the instruction is transmitted from the input section 95 to the control section 94. The control section 94 controls each section of the light source apparatus 10, the camera head 50, and the control apparatus 90 on the basis of an instruction from the user input via the input section 95, whereby a desired observation mode is performed.

In the following embodiments, the broadband light emitted from the broadband light source 11 is white light, and the first wavelength band that is the wavelength band of the broadband light is included in the visible light wavelength band. In addition, the first fluorescence emitted from the first substance excited by the first narrowband light from the first narrowband light source 12 is visible light (in particular, visible light having a wavelength band overlapping that of broadband light (white light)). In addition, the second fluorescence and the third fluorescence emitted from the second substance and the third substance excited by the second narrowband light and the third narrowband light from the second narrowband light source 13 and the third narrowband light source 14 are invisible light (for example, infrared light) included in the invisible light wavelength band. The wavelength bands of the second fluorescence and the third fluorescence are included in the invisible light wavelength band and do not overlap with the wavelength bands of the broadband light (white light) and the first fluorescence. Note that the infrared light mentioned here includes near-infrared light, mid-infrared light, and far-infrared light, and is light (electromagnetic wave) in a wavelength band of approximately 700 nm to 1000 µm.

The broadband light (white light) emitted from the broadband light source 11 is used as illumination light for brightly illuminating the observation target. On the other hand, the first narrowband light to the third narrowband light are used as excitation light for fluorescent substances (first to third substances). When the observation target is irradiated with broadband light (white light), at least a part of the broadband light is reflected as broadband reflected light. In addition, when the observation target is irradiated with the excitation light (the first narrowband light to the third narrowband light), the observation target emits corresponding fluorescence at a portion containing the corresponding fluorescent substance, but does not emit corresponding fluorescence at a portion not containing the corresponding fluorescent substance.

Note that specific wavelength bands of the broadband light and the first fluorescence to the third fluorescence are not limited to the above-described examples, and even in a case where the broadband light and the first fluorescence to the third fluorescence are light of other wavelength bands, the embodiments described below can be appropriately applied.

### [First Embodiment]

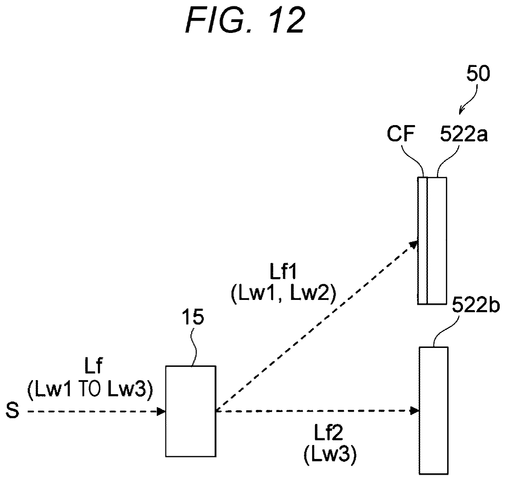

Fig. 12 is a diagram for explaining types of light incident on the imaging elements (the first imaging element 522a and the second imaging element 522b) according to the first embodiment.

The camera head 50 (in particular, the imaging section 52) of the present embodiment includes a two-plate type imaging module (see Figs. 3 and 4), and includes a first imaging element 522a having a color filter CF and a second imaging element 522b having no color filter CF.

For example, the resolution of the first imaging element 522a may be higher than the resolution of the second imaging element 522b, and the sensitivity of the second imaging element 522b may be higher than the sensitivity of the first imaging element 522a. However, the resolution and sensitivity of the first imaging element 522a and the second imaging element 522b are not limited thereto, and the relative relationship between the resolutions and sensitivities between the first imaging element 522a and the second imaging element 522b is not limited thereto.

The color filter CF can have an arbitrary color filter and an arbitrary filter array as long as light in a desired wavelength band can be incident on the corresponding imaging element, and may include a primary color filter (RGB filter) or a complementary color filter (CMYG filter). The color filter CF provided in the first imaging element 522a of the present embodiment transmits the broadband reflected light Lw1 and the first fluorescence Lw2 received by the first imaging element 522a as described later, but may or may not transmit light (for example, the second fluorescence Lw3) in a wavelength band different from the broadband reflected light Lw1 and the first fluorescence Lw2. The color filter CF may be provided for all the pixels of the first imaging element 522a, or the color filter CF may not be provided for some pixels of the first imaging element 522a.

Note that the second imaging element 522b of the present example does not include the color filter CF, but may include a color filter CF capable of transmitting the second fluorescence Lw3 as described later. For example, in a case where the second fluorescence Lw3 is infrared light, an R filter capable of transmitting not only red light in the visible light region but also infrared light (particularly, the second fluorescence Lw3) may be provided in the second imaging element 522b. As described above, the second imaging element 522b may or may not include the color filter CF, but in a case where the color filter CF is not included, a captured image can be acquired with higher light receiving sensitivity. For example, in a case where the first imaging element 522a includes an RGB filter as the color filter CF, the second imaging element 522b may also include an RGB filter similar to that of the first imaging element 522a.

The light source apparatus 10 (see Fig. 1A) emits light from at least one of the broadband light source 11, the first narrowband light source 12, or the second narrowband light source 13, and can irradiate the observation target S with at least one of broadband light, first narrowband light, or second narrowband light.

Therefore, the observation light Lf from the observation target S may include broadband reflected light Lw1 which is reflected light of broadband light, first fluorescence Lw2 emitted from the first substance excited by the first narrowband light, and second fluorescence Lw3 emitted from the second substance excited by the second narrowband light. Note that the broadband reflected light Lw1 and the first fluorescence Lw2 are light included in the first wavelength band, and the second fluorescence Lw3 is light included in the second wavelength band outside the first wavelength band.

The observation light Lf incident on the optical element 15 is separated into a first light flux Lf1 and a second light flux Lf2 by the optical element 15. The optical element 15 of the present embodiment guides light included in a first wavelength band to the first imaging element 522a as a first light flux Lf1, and guides light included in a second wavelength band to the second imaging element 522b as a second light flux Lf2.

As described above, in the present embodiment, the first light flux Lf1 in which the light in the wavelength band of the second fluorescence Lw3 is partially, substantially, or completely suppressed in the observation light Lf is guided to the first imaging element 522a. That is, light including at least the broadband reflected light Lw1 and the first fluorescence Lw2 is guided to the first imaging element 522a as the first light flux Lf1. In addition, the second light flux Lf2 in which the light in the first wavelength band is partially, substantially, or completely suppressed by the optical element 15 in the observation light Lf is guided to the second imaging element 522b. That is, light including at least the second fluorescence Lw3 included in the second wavelength band is guided to the second imaging element 522b as the second light flux Lf2.

The medical observation system 100 of the present embodiment having the above-described configuration can acquire various captured images of the observation target S according to the following observation modes (first to third modes).

### <First Mode>

The first mode of the present embodiment is an observation mode of irradiating the observation target S with the broadband light and the first narrowband light to acquire a captured image based on the broadband reflected light Lw1 and the first fluorescence Lw2 from the observation target S.

The control apparatus 90 (control section 94 (see Fig. 7)) controls the light source apparatus 10 (the broadband light source 11 and the first narrowband light source 12 (see Fig. 1A)), the broadband light and the first narrowband light are emitted from the light source apparatus 10 in a time division manner, and the broadband light and the first narrowband light are emitted to the observation target S in a time division manner. That is, the light source apparatus 10 sequentially emits the broadband light and the first narrowband light, so that the observation target S is sequentially irradiated with the broadband light and the first narrowband light.

The light emission duration per pulse in time-division light emission (pulse light emission) is not limited. The light emission durations may be the same or different between the pulses emitted from one light source, and the light emission durations may be the same or different between the pulses emitted from the plurality of light sources. In addition, an irradiation interval which is a time interval between a certain pulse and a next pulse is not limited. The irradiation intervals may be the same or different between the pulses emitted from one light source, and the irradiation intervals may be the same or different between the pulses emitted from the plurality of light sources.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. As a result, the first imaging element 522a sequentially receives the first light flux Lf1 including the broadband reflected light Lw1 and the first light flux Lf1 including the first fluorescence Lw2.

Then, the first imaging element 522a sequentially and repeatedly outputs the image signal based on the broadband reflected light Lw1 and the image signal based on the first fluorescence Lw2 under the control of the control apparatus 90 (control section 94).

Then, the image generation section 93 (particularly, the image processing section 931 (see Fig. 7)) generates a captured image (high-resolution color image) of the observation target S based on the broadband reflected light Lw1 from the image signal based on the broadband reflected light Lw1 output from the first imaging element 522a. In addition, the image generation section 93 (image processing section 931) generates a captured image (high-resolution color image) of the observation target S based on the first fluorescence Lw2 from the image signal based on the first fluorescence Lw2 output from the first imaging element 522a. The "high-resolution color image" mentioned here is a color image acquired by a high-resolution imaging element, and it is not necessarily required that an output image sent from the high-resolution imaging element to a subsequent stage has the same high resolution as the "color image acquired by the high-resolution imaging element".

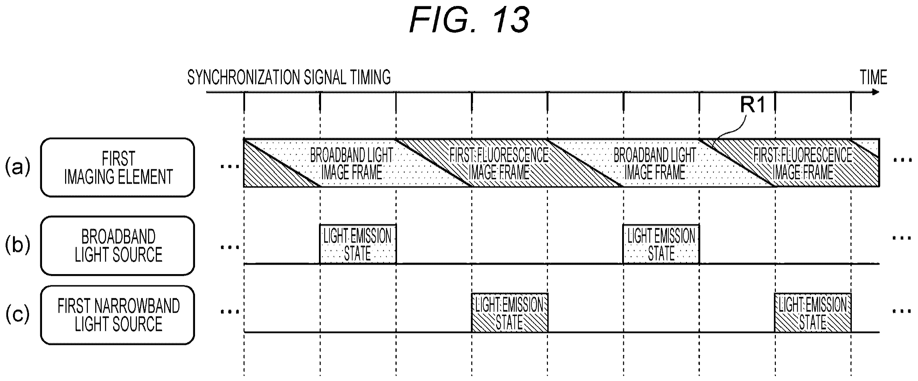

Fig. 13 illustrates an example of a timing chart of light source light emission and imaging element exposure in the first mode of the first embodiment.

(a) of Fig. 13 illustrates an exposure state of the first imaging element 522a, a vertical axis indicates a horizontal line of the first imaging element 522a, and a horizontal axis indicates time. In (a) of Fig. 13, the uppermost line represents the uppermost horizontal line (that is, the first line), and the lowermost line represents the lowermost horizontal line (that is, the last line).

A line (oblique line) indicated by a reference sign "R1" indicates a pixel data read start timing of each horizontal line regarding each image frame. The "broadband light image frame" between the lines R1 is an image frame for receiving (exposing) the broadband reflected light Lw1 from the observation target S. The "first fluorescence image frame" between the lines R1 is an image frame for receiving (exposing) the first fluorescence Lw2 from the observation target S.

(b) of Fig. 13 illustrates the emission timing of the broadband light in the broadband light source 11, and (c) illustrates the emission timing of the first narrowband light in the first narrowband light source 12. Note that the light emitted from the light source apparatus 10 instantaneously reaches the observation target S after light emission. Therefore, the timing at which the broadband light from the broadband light source 11 is emitted to the observation target S and the timing at which the broadband reflected light Lw1 from the observation target S is received by the first imaging element 522a are substantially the same as the timing at which the broadband light is emitted from the broadband light source 11.

In this mode, as described above, the light emission in the light source apparatus 10 and the irradiation of the observation target S with respect to the broadband light and the first narrowband light are performed in a time division manner. In addition, exposure (light reception) in the first imaging element 522a and reading of image data (pixel values) from the first imaging element 522a regarding the broadband reflected light Lw1 and the first fluorescence Lw2 from the observation target S are sequentially performed. Therefore, control is performed by the control apparatus 90 (control section 94) so that the timing of time-division light emission by the light source apparatus 10 and the timing of reading image data from the first imaging element 522a are associated with each other.

Specifically, on the basis of the timing determined by the common synchronization signal from the control section 94, the time-division light emission of the broadband light and the first narrowband light in the broadband light source 11 and the first narrowband light source 12 is performed, and the exposure and the reading of the image data in the first imaging element 522a are performed. More specifically, the broadband light and the first narrowband light are sequentially emitted so as not to overlap each other such that the first imaging element 522a is not simultaneously exposed by both the broadband reflected light Lw1 and the first fluorescence Lw2.

Then, image data is read from the first imaging element 522a such that a broadband light image frame to be exposed by the broadband reflected light Lw1 and a first fluorescence image frame to be exposed by the first fluorescence Lw2 are alternately output from the first imaging element 522a.

In the present example, exposure and reading of image data in the first imaging element 522a are performed on the basis of the global shutter method. That is, in imaging of a certain image frame, exposure is sequentially started row by row from the first row to the last row of the plurality of pixels of the first imaging element 522a. Then, after the exposure time has elapsed, pixel data is sequentially output row by row from the first row to the last row of the plurality of pixels. The exposure time of each image frame meaning "a period from the start to the end of charge accumulation in each pixel" is not limited, but is generally set to 1/60 seconds or 1/50 seconds in many cases.

In the example illustrated in Fig. 13, the emission of the broadband light and the first narrowband light is started and terminated while the image data is not read from the first imaging element 522a. Therefore, while the image data is not read from the first imaging element 522a, substantial incidence of the broadband reflected light Lw1 and the first fluorescence Lw2 on the first imaging element 522a starts and ends. In this case, in the broadband light image frame of the first imaging element 522a, the first fluorescence Lw2 from the observation target S irradiated with the first narrowband light can be suppressed from entering the first imaging element 522a. Furthermore, in the first fluorescence image frame of the first imaging element 522a, the broadband reflected light Lw1 from the observation target S irradiated with the broadband light can be suppressed from entering the first imaging element 522a.

Note that the start timing and the end timing of the light emission of the broadband light and the first narrowband light are not limited to the example illustrated in Fig. 13, and can be set to arbitrary timing. For example, while image data is being read from the first imaging element 522a (see "R1" in Fig. 13), light emission of one or both of the broadband light and the first narrowband light may be started or ended. For example, in a case where the intensity of fluorescence that is a light receiving target in the first fluorescence image frame is weak (that is, in a case where the amount of fluorescence emission is small), the first narrowband light may be emitted from the first narrowband light source 12 while image data of the broadband light image frame is being read. In this case, the exposure time of fluorescence in the first fluorescence image frame can be lengthened, which is advantageous for obtaining image data of a bright first fluorescence image frame. In particular, in a case where the first fluorescence Lw2 has a sufficiently smaller light amount than the broadband reflected light Lw1 and the influence of the first fluorescence Lw2 on the broadband light image frame is sufficiently smaller than the influence of the broadband reflected light Lw1 on the broadband light image frame, the first narrowband light source 12 may constantly emit the first narrowband light. The "constant light emission" mentioned here means continuous light emission.

In this manner, the first imaging element 522a alternately and repeatedly outputs the image signal of the broadband light image frame (that is, the image signal based on the broadband reflected light Lw1) and the image signal of the first fluorescence image frame (that is, the image signal based on the first fluorescence Lw2). Then, on the basis of the image signal of the broadband light image frame, a normal light captured image of the observation target S, which is a reflected image of the broadband light (white light), is generated. In addition, a first fluorescence captured image which is a captured image based on the first fluorescence Lw2 of the observation target S is generated on the basis of the image signal of the first fluorescence image frame.

As described above, in this mode, in the common time frame, the normal light captured image which is the reflected image of the visible light (white light) and the first fluorescence captured image in which the first substance in the observation target S is emphasized, that is, the fluorescence is emitted from the first substance in the observation target S excited by the narrowband light are acquired. Therefore, the user can compare and observe the normal light captured image and the first fluorescence captured image of the observation target S and observe a composite image (superimposed image) formed from these images via the display apparatus 70 (see Fig. 1A).

### <Second Mode>

The second mode of the present embodiment is an observation mode of irradiating the observation target S with the broadband light and the second narrowband light to acquire a captured image based on the broadband reflected light Lw1 and the second fluorescence Lw3 from the observation target S.

The control apparatus 90 (control section 94) controls the light source apparatus 10 (the broadband light source 11 and the second narrowband light source 13), the broadband light and the second narrowband light are continuously emitted from the light source apparatus 10, and the broadband light and the second narrowband light are continuously emitted to the observation target S.

Then, the optical element 15 continuously guides the first light flux Lf1 including the broadband reflected light Lw1 from the observation target S irradiated with the broadband light to the first imaging element 522a. In addition, the optical element 15 continuously guides the second light flux Lf2 including the second fluorescence Lw3 from the observation target S irradiated with the second narrowband light to the second imaging element 522b.

As a result, the first imaging element 522a continuously receives the first light flux Lf1 including the broadband reflected light Lw1, and the second imaging element 522b continuously receives the second light flux Lf2 including the second fluorescence Lw3. Then, under the control of the control apparatus 90 (control section 94), the first imaging element 522a continuously and repeatedly outputs an image signal based on the broadband reflected light Lw1, and the second imaging element 522b continuously and repeatedly outputs an image signal based on the second fluorescence Lw3.

Then, the image generation section 93 (image processing section 931) generates a captured image (high-resolution color image) of the observation target S based on the broadband reflected light Lw1 from the image signal based on the broadband reflected light Lw1 output from the first imaging element 522a. In addition, the image generation section 93 (image processing section 931) generates a captured image (high-sensitivity monochrome image) of the observation target S based on the second fluorescence Lw3 from the image signal based on the second fluorescence Lw3 output from the second imaging element 522b. The "high-resolution color image" mentioned here is a color image acquired by the high-resolution imaging element, and the "high-sensitivity monochrome image" is a monochrome image acquired by the high-sensitivity imaging element.

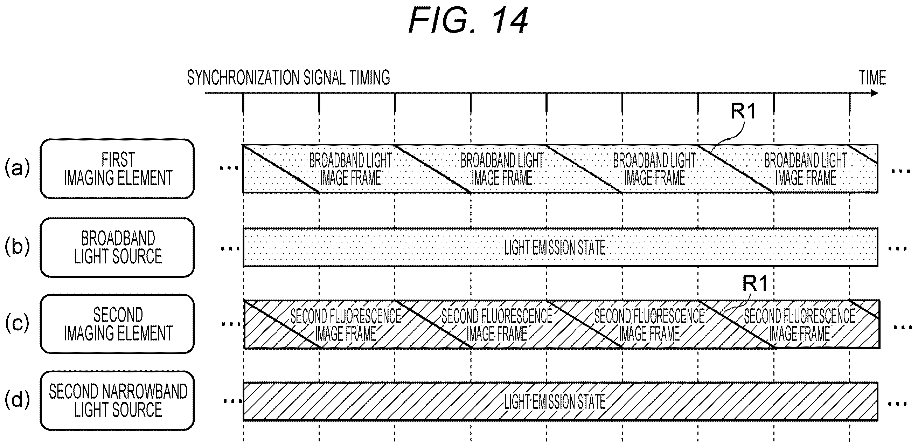

Fig. 14 illustrates an example of a timing chart of light source light emission and imaging element exposure in the second mode of the first embodiment.

(a) of Fig. 14 illustrates an exposure state of the first imaging element 522a, and (c) illustrates an exposure state of the second imaging element 522b. In (a) and (c) of Fig. 14, the vertical axis represents a horizontal line of the first imaging element 522a and the second imaging element 522b, the horizontal axis represents time, and line R1 represents pixel data read start timing of each horizontal line regarding each image frame. The "broadband light image frame" between the lines R1 is an image frame for receiving (exposing) the broadband reflected light Lw1 from the observation target S, and the "second fluorescence image frame" between the lines R1 is an image frame for receiving (exposing) the second fluorescence Lw3 from the observation target S. (b) of Fig. 14 illustrates the light emission timing of the broadband light in the broadband light source 11, and (d) illustrates the light emission timing of the second narrowband light in the second narrowband light source 13.

In this mode, as described above, the light emission in the light source apparatus 10 and the irradiation of the observation target S with respect to the broadband light and the second narrowband light are continuously performed. Then, exposure (light reception) in the first imaging element 522a regarding the broadband reflected light Lw1 from the observation target S is continuously performed. Furthermore, exposure (light reception) in the second imaging element 522b regarding the second fluorescence Lw3 from the observation target S is continuously performed.

Therefore, reading of image data from the first imaging element 522a and the second imaging element 522b can be executed at any timing while the broadband light and the second narrowband light are emitted by the light source apparatus 10. In the present example, exposure and reading of image data in the first imaging element 522a and the second imaging element 522b are performed on the basis of a timing determined by a common synchronization signal.

In the example illustrated in Fig. 14, the pixel data read start timing R1 of the first imaging element 522a coincides with the pixel data read start timing R1 of the second imaging element 522b. However, the pixel data read start timing R1 of the first imaging element 522a may not coincide with the pixel data read start timing R1 of the second imaging element 522b.

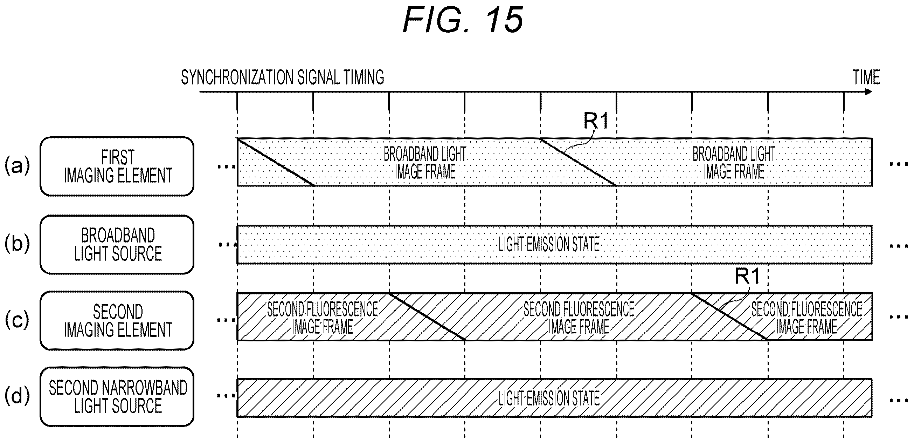

Fig. 15 illustrates another example of a timing chart of light source light emission and imaging element exposure in the second mode of the first embodiment. In the example illustrated in Fig. 15, the pixel data read start timing R1 of the first imaging element 522a and the pixel data read start timing R1 of the second imaging element 522b are shifted from each other by 1/2 of the exposure time of each image frame, and do not overlap each other in time. Furthermore, the exposure time of each image frame of the first imaging element 522a and the second imaging element 522b in the example illustrated in Fig. 15 is twice the exposure time of each image frame in the example illustrated in Fig. 14.

As described above, the first imaging element 522a continuously and repeatedly outputs the image signal of the broadband light image frame exposed by the broadband reflected light Lw1 (that is, the image signal based on the broadband reflected light Lw1). In addition, the second imaging element 522b continuously repeatedly outputs the image signal of the second fluorescence image frame exposed with the second fluorescence Lw3 (that is, the image signal based on the second fluorescence Lw3).

Then, on the basis of the image signal of the broadband light image frame, a normal light captured image of the observation target S, which is a reflected image of the broadband light (white light), is generated. In addition, the second fluorescence captured image that is the captured image of the observation target S based on the second fluorescence Lw3 is generated on the basis of the image signal of the second fluorescence image frame.

As described above, in this mode, in the common time frame, the normal light captured image which is the reflected image of the visible light (white light) and the second fluorescence captured image in which the second substance in the observation target S is emphasized, that is, the fluorescence is emitted from the second substance in the observation target S excited by the narrowband light are acquired. Therefore, the user can compare and observe the normal light captured image and the second fluorescence captured image of the observation target S and observe a composite image (superimposed image) formed from these images via the display apparatus 70.

Note that, in the above-described example, the broadband light and the second narrowband light are constantly emitted from the light source apparatus 10 (that is, continuous emission), but the light source apparatus 10 may turn off the emission of each of the broadband light and the second narrowband light in the middle under the control of the control apparatus 90 (control section 94). For example, the light source apparatus 10 may repeatedly turn on and off the emission of the broadband light and the second narrowband light, and may emit the broadband light and the second narrowband light in a time division manner.

However, by constantly emitting the broadband light and the second narrowband light by the light source apparatus 10, the first imaging element 522a can continuously receive the broadband reflected light Lw1, and the second imaging element 522b can continuously receive the first fluorescence. As a result, in the first imaging element 522a and the second imaging element 522b, the charge accumulation amount increases with long-time exposure, and a bright captured image can be acquired, and an increase in noise due to gain adjustment can be suppressed. In addition, all the frames of the first imaging element 522a and the second imaging element 522b can be used for generating a captured image. By preventing the occurrence of a frame in which substantial imaging is not performed in this manner, it is possible to avoid a substantial decrease in the frame rate and to provide a smooth video.

### <Third Mode>

The third mode of the present embodiment is an observation mode of irradiating the observation target S with the broadband light, the first narrowband light, and the second narrowband light to acquire a captured image based on the broadband reflected light Lw1, the first fluorescence Lw2, and the second fluorescence Lw3 from the observation target S.

That is, the control apparatus 90 (control section 94) controls the light source apparatus 10 (broadband light source 11, first narrowband light source 12, and second narrowband light source 13), the broadband light and the first narrowband light are emitted from the light source apparatus 10 in a time division manner, and the second narrowband light is continuously emitted. As a result, the broadband light and the first narrowband light are emitted to the observation target S in a time division manner, and the second narrowband light is continuously emitted to the observation target S.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 continuously guides the second light flux Lf2 including the second fluorescence Lw3 from the observation target S irradiated with the second narrowband light to the second imaging element 522b.

As a result, the first imaging element 522a sequentially receives the first light flux Lf1 including the broadband reflected light Lw1 and the first light flux Lf1 including the first fluorescence Lw2. Then, the first imaging element 522a sequentially and repeatedly outputs the image signal based on the broadband reflected light Lw1 and the image signal based on the first fluorescence Lw2 under the control of the control apparatus 90 (control section 94).

On the other hand, the second imaging element 522b continuously receives the second light flux Lf2 including the second fluorescence Lw3, and continuously and repeatedly outputs an image signal based on the second fluorescence Lw3 under the control of the control apparatus 90 (control section 94).

Then, the image generation section 93 (image processing section 931) generates a captured image (high-resolution color image) of the observation target S based on the broadband reflected light Lw1 from the image signal based on the broadband reflected light Lw1 output from the first imaging element 522a. In addition, the image generation section 93 (image processing section 931) generates a captured image (high-resolution color image) of the observation target S based on the first fluorescence Lw2 from the image signal based on the first fluorescence Lw2 output from the first imaging element 522a. In addition, the image generation section 93 (image processing section 931) generates a captured image (high-sensitivity monochrome image) of the observation target S based on the second fluorescence Lw3 from the image signal based on the second fluorescence Lw3 output from the second imaging element 522b. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light), the first fluorescence captured image which is the image in which the first substance in the observation target S is emphasized, that is, the fluorescence is emitted from the first substance in the observation target S excited by the narrowband light, and the second fluorescence captured image which is the image in which the second substance in the observation target S is emphasized, that is, the fluorescence is emitted from the second substance in the observation target S excited by the narrowband light are acquired. Therefore, the user can compare and observe the normal light captured image, the first fluorescence captured image, and the second fluorescence captured image of the observation target S or observe a composite image (superimposed image) formed from these images via the display apparatus 70.

### [Second Embodiment]

In the present embodiment, the same or corresponding elements as those in the above-described first embodiment are denoted by the same reference signs, and a detailed description thereof will be omitted.

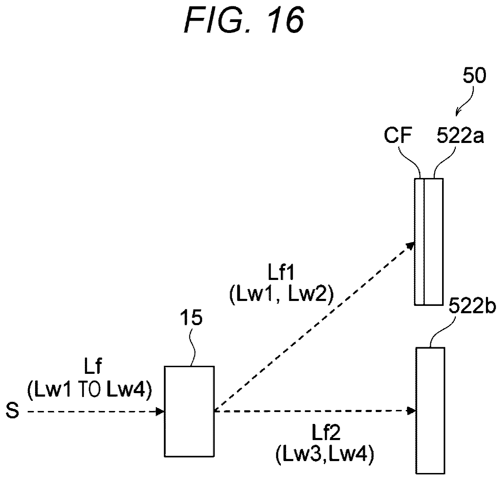

Fig. 16 is a diagram for explaining types of light incident on the imaging elements (the first imaging element 522a and the second imaging element 522b) according to the second embodiment.

The camera head 50 (in particular, the imaging section 52) of the present embodiment includes a two-plate type imaging module (see Figs. 3 and 4), and includes a first imaging element 522a having a color filter CF and a second imaging element 522b having no color filter CF.

The color filter CF provided in the first imaging element 522a of the present embodiment transmits the broadband reflected light Lw1 and the first fluorescence Lw2 received by the first imaging element 522a as described later, but may or may not transmit light (for example, the second fluorescence Lw3 and the third fluorescence Lw4) in a wavelength band different from the broadband reflected light Lw1 and the first fluorescence Lw2. Note that the second imaging element 522b does not include the color filter CF in the present example, but may include a color filter CF capable of transmitting the second fluorescence Lw3 and the third fluorescence Lw4 as described later.

For example, the resolution of the first imaging element 522a may be higher than the resolution of the second imaging element 522b, and the sensitivity of the second imaging element 522b may be higher than the sensitivity of the first imaging element 522a. However, the resolution and sensitivity of the first imaging element 522a and the second imaging element 522b are not limited thereto, and the relative relationship between the resolutions and sensitivities between the first imaging element 522a and the second imaging element 522b is not limited thereto.

The light source apparatus 10 (see Fig. 1B) emits light from at least one of the broadband light source 11, the first narrowband light source 12, the second narrowband light source 13, or the third narrowband light source 14, and can irradiate the observation target S with at least one of broadband light, first narrowband light, second narrowband light, or third narrowband light.

Therefore, the observation light Lf from the observation target S may include broadband reflected light Lw1 which is reflected light of broadband light, first fluorescence Lw2 emitted from the first substance excited by the first narrowband light, second fluorescence Lw3 emitted from the second substance excited by the second narrowband light, and third fluorescence Lw4 emitted from the third substance excited by the third narrowband light. Note that the broadband reflected light Lw1 and the first fluorescence Lw2 are light included in the first wavelength band, and the second fluorescence Lw3 and the third fluorescence Lw4 are light included in the second wavelength band outside the first wavelength band.

The observation light Lf incident on the optical element 15 is separated into a first light flux Lf1 and a second light flux Lf2 by the optical element 15. The optical element 15 of the present embodiment guides light included in a first wavelength band to the first imaging element 522a as a first light flux Lf1, and guides light included in a second wavelength band to the second imaging element 522b as a second light flux Lf2.

As described above, in the present embodiment, the first light flux Lf1 in which the light in the wavelength bands of the second fluorescence Lw3 and the third fluorescence Lw4 in the observation light Lf is partially, substantially, or completely suppressed is guided to the first imaging element 522a. That is, light including at least the broadband reflected light Lw1 and the first fluorescence Lw2 is guided to the first imaging element 522a as the first light flux Lf1. In addition, the second light flux Lf2 in which the light in the first wavelength band is partially, substantially, or completely suppressed by the optical element 15 in the observation light Lf is guided to the second imaging element 522b. That is, light including at least the second fluorescence Lw3 and the third fluorescence Lw4 included in the second wavelength band is guided to the second imaging element 522b as the second light flux Lf2.

The medical observation system 100 of the present embodiment having the above-described configuration can acquire various captured images of the observation target S according to the following observation modes (first to fifth modes).

### <First Mode to Third Mode>

The first mode to the third mode of the present embodiment are performed similarly to the first mode to the third mode of the first embodiment described above.

Therefore, in the first mode, the broadband light and the first narrowband light are emitted in a time division manner by the light source apparatus 10, and the broadband reflected light Lw1 and the first fluorescence Lw2 are received by the first imaging element 522a. Furthermore, in the second mode, the broadband light and the second narrowband light are continuously emitted by the light source apparatus 10, the broadband reflected light Lw1 is received by the first imaging element 522a, and the second fluorescence Lw3 is received by the second imaging element 522b. In addition, in the third mode, the light source apparatus 10 emits the broadband light and the first narrowband light in a time division manner and the second narrowband light is continuously emitted, the broadband reflected light Lw1 and the first fluorescence Lw2 are received by the first imaging element 522a, and the second fluorescence Lw3 is received by the second imaging element 522b.

### <Fourth Mode>

The fourth mode of the present embodiment is an observation mode of irradiating the observation target S with the broadband light and the third narrowband light to acquire a captured image based on the broadband reflected light Lw1 and the third fluorescence Lw4 from the observation target S.

That is, the control apparatus 90 (control section 94) controls the light source apparatus 10 (the broadband light source 11 and the third narrowband light source 14), the broadband light and the third narrowband light are continuously emitted from the light source apparatus 10, and the broadband light and the third narrowband light are continuously emitted to the observation target S. Note that, in this mode, the first narrowband light source 12 and the second narrowband light source 13 are placed in the OFF state, and the first narrowband light and the second narrowband light are not emitted from the light source apparatus 10.

Then, the optical element 15 continuously guides the first light flux Lf1 including the broadband reflected light Lw1 from the observation target S irradiated with the broadband light to the first imaging element 522a. In addition, the optical element 15 continuously guides the second light flux Lf2 including the third fluorescence Lw4 from the observation target S irradiated with the third narrowband light to the second imaging element 522b.

As a result, the first imaging element 522a continuously receives the first light flux Lf1 including the broadband reflected light Lw1, and continuously and repeatedly outputs an image signal based on the broadband reflected light Lw1 under the control of the control apparatus 90 (control section 94). Furthermore, the second imaging element 522b continuously receives the second light flux Lf2 including the third fluorescence Lw4, and continuously and repeatedly outputs an image signal based on the third fluorescence Lw4 under the control of the control apparatus 90 (control section 94).

Then, the image generation section 93 (image processing section 931) generates a captured image (high-resolution color image) of the observation target S based on the broadband reflected light Lw1 from the image signal based on the broadband reflected light Lw1 output from the first imaging element 522a. In addition, the image generation section 93 (image processing section 931) generates a captured image (high-sensitivity monochrome image) of the observation target S based on the third fluorescence Lw4 from the image signal based on the third fluorescence Lw4 output from the second imaging element 522b. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the third fluorescence captured image in which the third substance in the observation target S is emphasized, that is, the third substance in the observation target S excited by the narrowband light emits fluorescence are acquired. Therefore, the user can compare and observe the normal light captured image and the third fluorescence captured image of the observation target S and observe a composite image (superimposed image) formed from these images via the display apparatus 70.

### <Fifth Mode>

The fifth mode of the present embodiment is an observation mode of irradiating the observation target S with the broadband light and the first to third narrowband light to acquire a captured image based on the broadband reflected light Lw1 and the first fluorescence Lw2 to the third fluorescence Lw4 from the observation target S.

That is, the control apparatus 90 (control section 94) controls the light source apparatus 10 (the broadband light source 11 and the first to third narrowband light sources 12 to 14), and the broadband light and the first narrowband light are emitted from the light source apparatus 10 in a time division manner, and the second narrowband light and the third narrowband light are emitted in a time division manner. As a result, the broadband light and the first narrowband light are emitted to the observation target S in a time division manner, and the second narrowband light and the third narrowband light are emitted to the observation target S in a time division manner. Note that each of the broadband light and the first narrowband light may be simultaneously emitted with one of the second narrowband light and the third narrowband light, and simultaneously emitted to the observation target S.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the second fluorescence Lw3 from the observation target S irradiated with the second narrowband light and the second light flux Lf2 including the third fluorescence Lw4 from the observation target S irradiated with the third narrowband light to the second imaging element 522b.

As a result, the first imaging element 522a sequentially receives the first light flux Lf1 including the broadband reflected light Lw1 and the first light flux Lf1 including the first fluorescence Lw2. Then, the first imaging element 522a sequentially and repeatedly outputs the image signal based on the broadband reflected light Lw1 and the image signal based on the first fluorescence Lw2 under the control of the control apparatus 90 (control section 94).

On the other hand, the second imaging element 522b sequentially receives the second light flux Lf2 including the second fluorescence Lw3 and the second light flux Lf2 including the third fluorescence Lw4. Then, the second imaging element 522b sequentially and repeatedly outputs the image signal based on the second fluorescence Lw3 and the image signal based on the third fluorescence Lw4 under the control of the control apparatus 90 (control section 94).

Then, the image generation section 93 (image processing section 931) generates a captured image (high-resolution color image) of the observation target S based on the broadband reflected light Lw1 from the image signal based on the broadband reflected light Lw1 output from the first imaging element 522a. In addition, the image generation section 93 (image processing section 931) generates a captured image (high-resolution color image) of the observation target S based on the first fluorescence Lw2 from the image signal based on the first fluorescence Lw2 output from the first imaging element 522a. The "high-resolution color image" mentioned here is a color image acquired by the high-resolution imaging element.

In addition, the image generation section 93 (image processing section 931) generates a captured image (high-sensitivity monochrome image) of the observation target S based on the second fluorescence Lw3 from the image signal based on the second fluorescence Lw3 output from the second imaging element 522b. In addition, the image generation section 93 (image processing section 931) generates a captured image (high-sensitivity monochrome image) of the observation target S based on the third fluorescence Lw4 from the image signal based on the third fluorescence Lw4 output from the second imaging element 522b. The "high-sensitivity monochrome image" mentioned here is a monochrome image acquired by the high-sensitivity imaging element.

As described above, in this mode, in the common time frame, the normal light captured image which is the reflected image of the visible light (white light) and the first to third fluorescence captured images in which the first to third substances in the observation target S are emphasized, that is, the fluorescence is emitted from the first to third substances in the observation target S excited by the narrowband light are acquired. Therefore, the user can compare and observe the normal light captured image, the first fluorescence captured image, the second fluorescence captured image, and the third fluorescence captured image of the observation target S or observe a composite image (superimposed image) formed from these images via the display apparatus 70.

As described above, according to the present embodiment, the broadband reflected light Lw1 and the first fluorescence Lw2 of the observation light Lf are imaged by the first imaging element 522a, while the second fluorescence Lw3 and the third fluorescence Lw4 are imaged by the second imaging element 522b.

Therefore, a high-resolution color image is obtained as a captured image from the broadband reflected light Lw1 and the first fluorescence Lw2 in the visible light wavelength band having color information.

On the other hand, a high-sensitivity monochrome image is obtained as a captured image from the second fluorescence Lw3 and the third fluorescence Lw4 in the invisible light wavelength band having no color information. Therefore, even in a case where it is difficult for the first imaging element 522a to perform appropriate imaging due to the small light amounts of the second fluorescence Lw3 and the third fluorescence Lw4, such imaging of the second fluorescence Lw3 and the third fluorescence Lw4 is appropriately performed by the second imaging element 522b having excellent sensitivity.

### [Third Embodiment]

In the present embodiment, the same or corresponding elements as those in the first embodiment and the second embodiment described above are denoted by the same reference signs, and a detailed description thereof will be omitted.

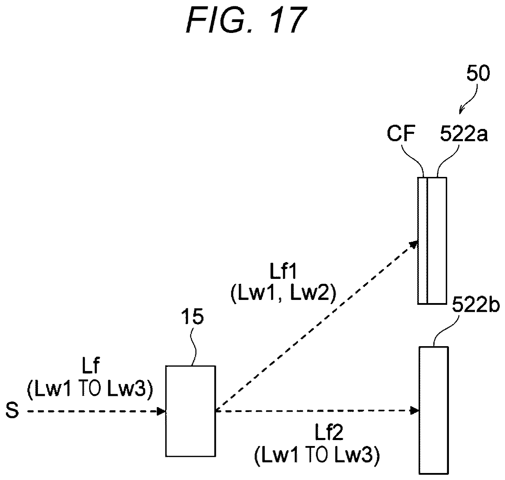

Fig. 17 is a diagram for explaining types of light incident on the imaging elements (the first imaging element 522a and the second imaging element 522b) according to the third embodiment.

The camera head 50 (in particular, the imaging section 52) of the present embodiment includes a two-plate type imaging module (see Figs. 3 and 4), and includes a first imaging element 522a having a color filter CF and a second imaging element 522b having no color filter CF. For example, the resolution of the first imaging element 522a may be higher than the resolution of the second imaging element 522b, and the sensitivity of the second imaging element 522b may be higher than the sensitivity of the first imaging element 522a. However, the resolution and sensitivity of the first imaging element 522a and the second imaging element 522b are not limited thereto, and the relative relationship between the resolutions and sensitivities between the first imaging element 522a and the second imaging element 522b is not limited thereto.

The color filter CF provided in the first imaging element 522a of the present embodiment transmits the broadband reflected light Lw1 and the first fluorescence Lw2 received by the first imaging element 522a as described later, but may or may not transmit light (for example, the second fluorescence Lw3) in a wavelength band different from the broadband reflected light Lw1 and the first fluorescence Lw2. Note that the second imaging element 522b does not include the color filter CF in the present example, but may include a color filter CF capable of transmitting the broadband reflected light Lw1, the first fluorescence Lw2, and the second fluorescence Lw3 as described later.

The light source apparatus 10 (see Fig. 1A) emits light from at least one of the broadband light source 11, the first narrowband light source 12, or the second narrowband light source 13, and can irradiate the observation target S with at least one of broadband light, first narrowband light, or second narrowband light.

Therefore, the observation light Lf from the observation target S may include broadband reflected light Lw1 which is reflected light of broadband light, first fluorescence Lw2 emitted from the first substance excited by the first narrowband light, and second fluorescence Lw3 emitted from the second substance excited by the second narrowband light. Note that the broadband reflected light Lw1 and the first fluorescence Lw2 are light included in the first wavelength band, and the second fluorescence Lw3 is light included in the second wavelength band outside the first wavelength band.

The observation light Lf incident on the optical element 15 is separated into a first light flux Lf1 and a second light flux Lf2 by the optical element 15. The optical element 15 of the present embodiment guides a part of light included in the first wavelength band to the first imaging element 522a as a first light flux Lf1, and guides a part of light included in the first wavelength band and light included in the second wavelength band to the second imaging element 522b as a second light flux Lf2.

As described above, in the present embodiment, the first light flux Lf1 in which the light in the first wavelength band is partially suppressed and the light in the wavelength band of the second fluorescence Lw3 is partially, substantially, or completely suppressed in the observation light Lf is guided to the first imaging element 522a. That is, light including at least a part of the broadband reflected light Lw1 is guided to the first imaging element 522a as the first light flux Lf1. In addition, the second light flux Lf2 in which the light of the first wavelength band is partially suppressed in the observation light Lf is guided to the second imaging element 522b. That is, light including at least a part of the broadband reflected light Lw1 and the first fluorescence Lw2 and the second fluorescence Lw3 is guided to the second imaging element 522b as the second light flux Lf2.

The medical observation system 100 of the present embodiment having the above-described configuration can acquire various captured images of the observation target S according to the following observation modes (first to third modes).

### <First Mode>

The first mode of the present embodiment is an observation mode of irradiating the observation target S with the broadband light and the first narrowband light to acquire a captured image based on the broadband reflected light Lw1 and the first fluorescence Lw2 from the observation target S.

The control apparatus 90 (control section 94 (see Fig. 7)) controls the light source apparatus 10 (the broadband light source 11 and the first narrowband light source 12 (see Fig. 1A)), emits the broadband light and the first narrowband light from the light source apparatus 10 in a time division manner, and irradiates the observation target S with the broadband light and the first narrowband light in a time division manner. Note that, in this mode, the second narrowband light source 13 is placed in the OFF state, and the second narrowband light is not emitted from the light source apparatus 10.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the broadband reflected light Lw1 from the observation target S irradiated with the broadband light and the second light flux Lf2 including the first fluorescence Lw2 from the observation target S irradiated with the first narrowband light to the second imaging element 522b.

As a result, the first imaging element 522a sequentially receives the first light flux Lf1 including the broadband reflected light Lw1 and the first light flux Lf1 including the first fluorescence Lw2. In addition, the second imaging element 522b sequentially receives the second light flux Lf2 including the broadband reflected light Lw1 and the second light flux Lf2 including the first fluorescence Lw2.

Then, each of the first imaging element 522a and the second imaging element 522b sequentially and repeatedly outputs the image signal based on the broadband reflected light Lw1 and the image signal based on the first fluorescence Lw2 under the control of the control apparatus 90 (control section 94 (see Fig. 7)).

Then, under the control of the control section 94, the image generation section 93 (particularly, the image processing section 931 (see Fig. 7)) generates a captured image (high-resolution color image) of the observation target S based on the broadband reflected light Lw1 on the basis of the image signal output from the first imaging element 522a that has received the first light flux Lf1 including the broadband reflected light Lw1. In addition, under the control of the control section 94, the image generation section 93 (image processing section 931) generates a captured image (high-sensitivity monochrome image) of the observation target S based on the first fluorescence Lw2 on the basis of the image signal output from the second imaging element 522b that has received the second light flux Lf2 including the first fluorescence Lw2. The "high-resolution color image" mentioned here is a color image acquired by the high-resolution imaging element, and the "high-sensitivity monochrome image" is a monochrome image acquired by the high-sensitivity imaging element.

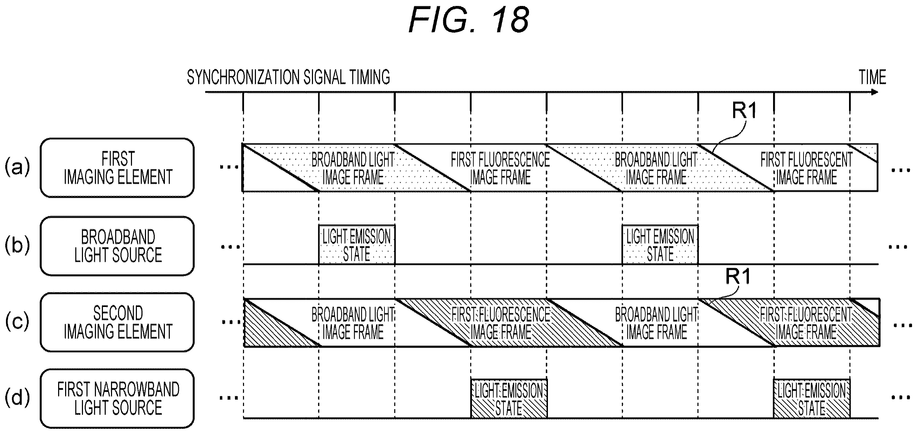

Fig. 18 illustrates an example of a timing chart of light source light emission and imaging element exposure in the first mode of the third embodiment.

(a) of Fig. 18 illustrates an exposure state of the first imaging element 522a, and (c) illustrates an exposure state of the second imaging element 522b. In (a) and (c) of Fig. 18, the vertical axis represents a horizontal line of the first imaging element 522a and the second imaging element 522b, the horizontal axis represents time, and line R1 represents pixel data read start timing of each horizontal line regarding each image frame. The "broadband light image frame" between the lines R1 is an image frame for receiving (exposing) the broadband reflected light Lw1 from the observation target S, and the "first fluorescence image frame" between the lines R1 is an image frame for receiving (exposing) the first fluorescence Lw2 from the observation target S. (b) of Fig. 18 illustrates the emission timing of the broadband light in the broadband light source 11, and (d) illustrates the emission timing of the first narrowband light in the first narrowband light source 12.

In this mode, as described above, the light emission in the light source apparatus 10 and the irradiation of the observation target S with respect to the broadband light and the first narrowband light are performed in a time division manner. Then, both the broadband reflected light Lw1 and the first fluorescence Lw2 from the observation target S are guided to both the first imaging element 522a and the second imaging element 522b, and are sequentially received by each of the first imaging element 522a and the second imaging element 522b. Then, in the above-described example, the captured image based on the broadband reflected light Lw1 is generated on the basis of the image signal from the first imaging element 522a, and the captured image based on the first fluorescence Lw2 is generated on the basis of the image signal from the second imaging element 522b.

Therefore, control by the control apparatus 90 (control section 94) is performed such that the timing of time-division light emission by the light source apparatus 10 and the timing of reading image data from the first imaging element 522a and the second imaging element 522b are associated with each other. Specifically, on the basis of a common synchronization signal, time-division light emission of broadband light and first narrowband light in the broadband light source 11 and the first narrowband light source 12 is performed, and exposure and image data reading in the first imaging element 522a and the second imaging element 522b are performed. More specifically, the broadband light and the first narrowband light are alternately emitted separately in time so that the first imaging element 522a and the second imaging element 522b are not simultaneously exposed by both the broadband reflected light Lw1 and the first fluorescence Lw2.

Then, image data is read such that an image signal of a broadband light image frame exposed by the broadband reflected light Lw1 and an image signal of a first fluorescence image frame exposed by the first fluorescence Lw2 are output from the first imaging element 522a and the second imaging element 522b. As a result, each of the first imaging element 522a and the second imaging element 522b alternately repeatedly outputs the image signal of the broadband light image frame and the image signal of the first fluorescence image frame.

Then, the image generation section 93 (image processing section 931) generates a normal light captured image of the observation target S, which is a reflected image of broadband light (white light), from the image signal of the broadband light image frame output from the first imaging element 522a. In addition, the image generation section 93 (image processing section 931) generates a first fluorescence captured image, which is a captured image based on the first fluorescence Lw2 of the observation target S, from the image signal of the first fluorescence image frame output from the second imaging element 522b.

As described above, in this mode, in the common time frame, the normal light captured image which is the reflected image of the visible light (white light) and the first fluorescence captured image in which the first substance in the observation target S is emphasized, that is, the fluorescence is emitted from the first substance in the observation target S excited by the narrowband light are acquired. Therefore, the user can compare and observe the normal light captured image and the first fluorescence captured image of the observation target S and observe a composite image (superimposed image) formed from these images via the display apparatus 70 (see Fig. 1A).

Note that, in the example illustrated in Fig. 18, while the image data is not read from the first imaging element 522a and the second imaging element 522b, the emission of the broadband light and the first narrowband light is started and terminated. Therefore, in the broadband light image frames of the first imaging element 522a and the second imaging element 522b, the first fluorescence Lw2 from the observation target S irradiated with the first narrowband light can be suppressed from entering the first imaging element 522a. Furthermore, in the first fluorescence image frames of the first imaging element 522a and the second imaging element 522b, the broadband reflected light Lw1 from the observation target S irradiated with the broadband light can be suppressed from entering the first imaging element 522a and the second imaging element 522b.

Note that the start timing and the end timing of the light emission of the broadband light and the first narrowband light are not limited to the example illustrated in Fig. 18, and can be set to arbitrary timing. For example, while image data is being read from the first imaging element 522a and the second imaging element 522b (see "R1" in Fig. 18), emission of one or both of the broadband light and the first narrowband light may be started or ended. For example, in a case where the intensity of fluorescence that is a light receiving target in the first fluorescence image frame is weak (that is, in a case where the amount of fluorescence emission is small), the first narrowband light may be emitted from the first narrowband light source 12 while image data of the broadband light image frame is being read. In this case, the exposure time of fluorescence in the first fluorescence image frame can be lengthened, which is advantageous for obtaining image data of a bright first fluorescence image frame. In particular, in a case where the first fluorescence Lw2 has a sufficiently smaller light amount than the broadband reflected light Lw1 and the influence of the first fluorescence Lw2 on the broadband light image frame is sufficiently smaller than the influence of the broadband reflected light Lw1 on the broadband light image frame, the first narrowband light source 12 may constantly emit the first narrowband light.

Note that, in the this mode, the image signal of the first fluorescence image frame is also output from the first imaging element 522a, and the image signal of the broadband light image frame is also output from the second imaging element 522b. However, in the above-described example, these image frames are not used for generating the captured image.

In the above example, the image data of these image frames not used to generate the captured image is also repeatedly output as the image signal from the imaging element (the first imaging element 522a and the second imaging element 522b), but the image data of the image frames not used to generate the captured image may not be output as the image signal from the imaging element. That is, while the image data of the image frame used to generate the captured image is output as the image signal from the imaging element, the accumulated charge of the imaging element is reset for the image frame not used to generate the captured image, and may not be output as the image signal from the imaging element.

In addition, in the above example, these image frames (that is, the first fluorescence image frame from the first imaging element 522a and the broadband light image frame from the second imaging element 522b) that are not used to generate the captured image may be used to generate the captured image. In this case, the captured image generated from the first fluorescence image frame from the first imaging element 522a and the broadband light image frame from the second imaging element 522b may be used for generating the output image, or may not be used for generating the output image.

The captured image that is not used to generate the output image can be used for any purpose. For example, the correction processing regarding the brightness of the output image may be performed by the image generation section 93 (see Fig. 7) on the basis of the image analysis result of the captured image that is not used for generating the output image. In addition, processing related to adjustment of a focal position such as contrast autofocus (AF) may be performed on the basis of an image analysis result of a captured image that is not used to generate an output image, and the focal position may be adjusted on the basis of contrast or a spatial frequency of such a captured image.

That is, the captured image based on the first fluorescence Lw2 may be generated on the basis of the image signal of the first fluorescence image frame from the first imaging element 522a. For example, the captured image based on the first fluorescence Lw2 may be generated on the basis of the image signal of the first fluorescence image frame from both the first imaging element 522a and the second imaging element 522b.

In addition, the captured image based on the broadband reflected light Lw1 may be generated on the basis of the image signal of the broadband light image frame from the second imaging element 522b. That is, the captured image based on the broadband reflected light Lw1 may be generated on the basis of the image signal of the broadband light image frame from both the first imaging element 522a and the second imaging element 522b.

### <Second Mode>

The second mode of the present embodiment is an observation mode of irradiating the observation target S with the broadband light and the second narrowband light to acquire a captured image based on the broadband reflected light Lw1 and the second fluorescence Lw3 from the observation target S.

That is, the control apparatus 90 (control section 94) controls the light source apparatus 10 (the broadband light source 11 and the second narrowband light source 13), the broadband light and the second narrowband light are emitted from the light source apparatus 10 in a time division manner, and the broadband light and the second narrowband light are emitted to the observation target S in a time division manner. Note that, in this mode, the first narrowband light source 12 is placed in the OFF state, and the first narrowband light is not emitted from the light source apparatus 10.

Then, the optical element 15 guides the first light flux Lf1 including the broadband reflected light Lw1 from the observation target S irradiated with the broadband light to the first imaging element 522a. In addition, the optical element 15 sequentially guides the second light flux Lf2 including the broadband reflected light Lw1 from the observation target S irradiated with the broadband light and the second light flux Lf2 including the second fluorescence Lw3 from the observation target S irradiated with the second narrowband light to the second imaging element 522b.

As a result, the first imaging element 522a receives the first light flux Lf1 including the broadband reflected light Lw1. In addition, the second imaging element 522b sequentially receives the second light flux Lf2 including the broadband reflected light Lw1 and the second light flux Lf2 including the second fluorescence Lw3.

Then, the first imaging element 522a repeatedly outputs an image signal based on the broadband reflected light Lw1 under the control of the control apparatus 90 (control section 94). Furthermore, the second imaging element 522b sequentially and repeatedly outputs the image signal based on the broadband reflected light Lw1 and the image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 on the basis of the image signal output from the first imaging element 522a that has received the first light flux Lf1 including the broadband reflected light Lw1. In addition, under the control of the control section 94, the image generation section 93 (image processing section 931) generates a captured image (high-sensitivity monochrome image) of the observation target S based on the second fluorescence Lw3 on the basis of the image signal output from the second imaging element 522b that has received the second light flux Lf2 including the second fluorescence Lw3. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the second fluorescence captured image in which the second substance in the observation target S is emphasized, that is, the fluorescence is emitted from the second substance in the observation target S excited by the narrowband light are acquired. Therefore, the user can compare and observe the normal light captured image and the second fluorescence captured image of the observation target S and observe a composite image (superimposed image) formed from these images via the display apparatus 70.

Note that, in the above-described example, the broadband light and the second narrowband light are emitted in a time division manner, but the light source apparatus 10 may constantly emit the second narrowband light while repeatedly turning on and off the emission of the broadband light under the control of the control apparatus 90 (control section 94).

In this case, the light source apparatus 10 (broadband light source 11) emits broadband light so that the broadband reflected light Lw1 is exposed to the first imaging element 522a in the broadband light image frame of the first imaging element 522a. On the other hand, the light source apparatus 10 (broadband light source 11) stops emission of broadband light so that the broadband reflected light Lw1 is not exposed to the second imaging element 522b in the second fluorescence image frame of the second imaging element 522b. As a result, the image signal of the broadband light image frame can be appropriately output from the first imaging element 522a, and the image signal of the second fluorescence image frame can be appropriately output from the second imaging element 522b.

Note that the start timing and the end timing of the light emission of the broadband light and the second narrowband light are not limited, and can be set to any timing. For example, while image data is being read from the first imaging element 522a and the second imaging element 522b (see "R1" in Fig. 18), light emission of one or both of the broadband light and the second narrowband light may be started or ended. For example, while image data of the second fluorescence image frame is being read, emission of broadband light from the broadband light source 11 may be performed.

Note that, in this mode, the image signal of the broadband light image frame is also output from the second imaging element 522b, but in the above-described example, the broadband light image frame from the second imaging element 522b is not used for generating the captured image. In the above example, the image data of the broadband light image frame not used to generate the captured image is repeatedly output from the second imaging element 522b as the image signal, but the image data of the broadband light image frame not used to generate the captured image may not be output from the second imaging element 522b as the image signal.

Furthermore, in the above-described example, the captured image based on the broadband reflected light Lw1 may be generated on the basis of the image signal of the broadband light image frame from the second imaging element 522b that is not used for generating the captured image. That is, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Third Mode>

The third mode of the present embodiment is an observation mode of irradiating the observation target S with the broadband light, the first narrowband light, and the second narrowband light to acquire a captured image based on the broadband reflected light Lw1, the first fluorescence Lw2, and the second fluorescence Lw3 from the observation target S.

That is, the control apparatus 90 (control section 94) controls the light source apparatus 10 (broadband light source 11, first narrowband light source 12, and second narrowband light source 13), and the broadband light, the first narrowband light, and the second narrowband light are emitted from the light source apparatus 10 in a time division manner. As a result, the broadband light, the first narrowband light, and the second narrowband light are emitted to the observation target S in a time division manner.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the broadband reflected light Lw1 from the observation target S irradiated with the broadband light, the second light flux Lf2 including the first fluorescence Lw2 from the observation target S irradiated with the first narrowband light, and the second light flux Lf2 including the second fluorescence Lw3 from the observation target S irradiated with the second narrowband light to the second imaging element 522b.

As a result, the first imaging element 522a sequentially receives the first light flux Lf1 including the broadband reflected light Lw1 and the first light flux Lf1 including the first fluorescence Lw2. Then, the first imaging element 522a sequentially and repeatedly outputs the image signal based on the broadband reflected light Lw1 and the image signal based on the first fluorescence Lw2 under the control of the control apparatus 90 (control section 94).

On the other hand, the second imaging element 522b sequentially receives the second light flux Lf2 including the broadband reflected light Lw1, the second light flux Lf2 including the first fluorescence Lw2, and the second light flux Lf2 including the second fluorescence Lw3. Then, the second imaging element 522b sequentially and repeatedly outputs an image signal based on the broadband reflected light Lw1, an image signal based on the first fluorescence Lw2, and an image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 on the basis of the image signal output from the first imaging element 522a that has received the first light flux Lf1 including the broadband reflected light Lw1. In addition, under the control of the control section 94, the image generation section 93 (image processing section 931) generates a captured image (high-sensitivity monochrome image) of the observation target S based on the first fluorescence Lw2 on the basis of the image signal output from the second imaging element 522b that has received the second light flux Lf2 including the first fluorescence Lw2. In addition, under the control of the control section 94, the image generation section 93 (image processing section 931) generates a captured image (high-sensitivity monochrome image) of the observation target S based on the second fluorescence Lw3 on the basis of the image signal output from the second imaging element 522b that has received the second light flux Lf2 including the second fluorescence Lw3. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light), and the first fluorescence captured image and the second fluorescence captured image which are images in which the first substance and the second substance in the observation target S are emphasized, that is, fluorescence is emitted from the first substance and the second substance in the observation target S excited by the narrowband light are acquired. Therefore, the user can compare and observe the normal light captured image, the first fluorescence captured image, and the second fluorescence captured image of the observation target S, and can observe a composite image (superimposed image) formed from these images.

Note that, in the this mode, the image signal of the first fluorescence image frame is also output from the first imaging element 522a, and the image signal of the broadband light image frame is also output from the second imaging element 522b. However, in the above-described example, these image frames are not used for generating the captured image. In the above example, the image data of these image frames not used to generate the captured image is also repeatedly output as the image signal from the imaging element (the first imaging element 522a and the second imaging element 522b), but the image data of the image frames not used to generate the captured image may not be output as the image signal from the imaging element.

Furthermore, in the above-described example, the captured image based on the first fluorescence Lw2 may be generated on the basis of the image signal of the first fluorescence image frame from the first imaging element 522a that is not used for generating the captured image. For example, the captured image based on the first fluorescence Lw2 may be generated on the basis of the image signal of the first fluorescence image frame from both the first imaging element 522a and the second imaging element 522b. In addition, the captured image based on the broadband reflected light Lw1 may be generated on the basis of the image signal of the broadband light image frame from the second imaging element 522b. That is, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### [Fourth Embodiment]

In the present embodiment, the same or corresponding elements as those in the first to third embodiments described above are denoted by the same reference signs, and a detailed description thereof will be omitted.

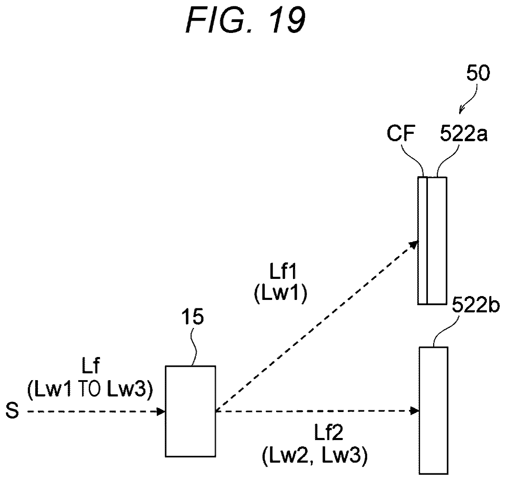

Fig. 19 is a diagram for explaining types of light incident on the imaging elements (the first imaging element 522a and the second imaging element 522b) according to the fourth embodiment.

The camera head 50 (in particular, the imaging section 52) of the present embodiment includes a two-plate type imaging module (see Figs. 3 and 4), and includes a first imaging element 522a having a color filter CF and a second imaging element 522b having no color filter CF. The first imaging element 522a has relatively lower sensitivity and higher resolution (for example, 4K resolution) than the second imaging element 522b, while the second imaging element 522b has relatively higher sensitivity and lower resolution (for example, HD resolution) than the first imaging element 522a.

The color filter CF provided in the first imaging element 522a of the present embodiment transmits the broadband reflected light Lw1 received by the first imaging element 522a as described later, but may or may not transmit light of a wavelength band different from the broadband reflected light Lw1 (for example, the second fluorescence Lw3). Note that the second imaging element 522b of the present example does not include the color filter CF, but may include a color filter CF capable of transmitting the first fluorescence Lw2 and the second fluorescence Lw3 as described later.

The light source apparatus 10 (see Fig. 1A) emits light from at least one of the broadband light source 11, the first narrowband light source 12, or the second narrowband light source 13, and can irradiate the observation target S with at least one of broadband light, first narrowband light, or second narrowband light.

Therefore, the observation light Lf from the observation target S may include broadband reflected light Lw1 which is reflected light of broadband light, first fluorescence Lw2 emitted from the first substance excited by the first narrowband light, and second fluorescence Lw3 emitted from the second substance excited by the second narrowband light. Note that the broadband reflected light Lw1 and the first fluorescence Lw2 are light included in the first wavelength band, and the second fluorescence Lw3 is light included in the second wavelength band outside the first wavelength band.

The observation light Lf incident on the optical element 15 is separated into a first light flux Lf1 and a second light flux Lf2 by the optical element 15. The optical element 15 of the present embodiment guides a part of the light included in the first wavelength band to the first imaging element 522a as a first light flux Lf1, and guides at least the first fluorescence Lw2 and the light included in the second wavelength band to the second imaging element 522b as a second light flux Lf2.

As described above, in the present embodiment, the first light flux Lf1 in which the light in the wavelength bands of the first fluorescence Lw2 and the second fluorescence Lw3 in the observation light Lf is partially, substantially, or completely suppressed is guided to the first imaging element 522a. That is, light including at least a part of the broadband reflected light Lw1 is guided to the first imaging element 522a as the first light flux Lf1. In addition, the second light flux Lf2 in which the light of the wavelength band other than the wavelength band of the first fluorescence Lw2 in the first wavelength band is partially, substantially, or completely suppressed in the observation light Lf is guided to the second imaging element 522b. That is, light including at least the first fluorescence Lw2 and the second fluorescence Lw3 is guided to the second imaging element 522b as the second light flux Lf2.

The medical observation system 100 of the present embodiment having the above-described configuration can acquire various captured images of the observation target S according to the following observation modes (first to third modes).

### <First Mode>

The first mode of the present embodiment is an observation mode of irradiating the observation target S with the broadband light and the first narrowband light to acquire a captured image based on the broadband reflected light Lw1 and the first fluorescence Lw2 from the observation target S.

That is, the control apparatus 90 (the control section 94 (see Fig. 7)) controls the light source apparatus 10 (the broadband light source 11 and the first narrowband light source 12 (see Fig. 1A)), the broadband light and the first narrowband light are emitted from the light source apparatus 10 in a time division manner, and the broadband light and the first narrowband light are emitted to the observation target S in a time division manner. Note that, in this mode, the second narrowband light source 13 is placed in the OFF state, and the second narrowband light is not emitted from the light source apparatus 10.

Then, the optical element 15 guides the first light flux Lf1 including the broadband reflected light Lw1 (however, the light in the wavelength band of the first fluorescence Lw2 is partially, substantially, or completely suppressed) from the observation target S irradiated with the broadband light to the first imaging element 522a. In addition, the optical element 15 guides the second light flux Lf2 including the first fluorescence Lw2 from the observation target S irradiated with the first narrowband light to the second imaging element 522b.

As a result, the first imaging element 522a receives the first light flux Lf1 including the broadband reflected light Lw1 (however, the light in the wavelength band of the first fluorescence Lw2 is partially, substantially, or completely suppressed), and the second imaging element 522b receives the second light flux Lf2 including the first fluorescence Lw2. Then, under the control of the control apparatus 90 (control section 94), the first imaging element 522a repeatedly outputs an image signal based on the broadband reflected light Lw1 (however, the light in the wavelength band of the first fluorescence Lw2 is partially, substantially, or completely suppressed), and the second imaging element 522b repeatedly outputs an image signal based on the first fluorescence Lw2.

Then, under the control of the control section 94, the image generation section 93 (in particular, the image processing section 931 (see Fig. 7)) generates a captured image (high-resolution color image) of the observation target S based on the broadband reflected light Lw1 on the basis of the image signal output from the first imaging element 522a that has received the first light flux Lf1 including the broadband reflected light Lw1 (however, the light in the wavelength band of the first fluorescence Lw2 is partially, substantially, or completely suppressed). In addition, under the control of the control section 94, the image generation section 93 (image processing section 931) generates a captured image (high-sensitivity monochrome image) of the observation target S based on the first fluorescence Lw2 on the basis of the image signal output from the second imaging element 522b that has received the second light flux Lf2 including the first fluorescence Lw2. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the first fluorescence captured image in which the first substance in the observation target S is emphasized, that is, the fluorescence is emitted from the first substance in the observation target S excited by the narrowband light are acquired. Therefore, the user can compare and observe the normal light captured image and the first fluorescence captured image of the observation target S and observe a composite image (superimposed image) formed from these images via the display apparatus 70 (see Fig. 1A).

Note that, in the above-described example, the broadband light and the first narrowband light are emitted in a time division manner, but the light source apparatus 10 may constantly emit the broadband light and/or the first narrowband light under the control of the control apparatus 90 (control section 94).

In a case where constant emission of broadband light is performed, the first imaging element 522a is continuously exposed by the broadband reflected light Lw1 (however, the light in the wavelength band of the first fluorescence Lw2 is partially, substantially, or completely suppressed). Furthermore, in a case where the constant emission of the first narrowband light is performed, the second imaging element 522b is continuously exposed by the first fluorescence Lw2. Therefore, in these cases, the charge accumulation amount in the imaging element increases, a bright captured image can be acquired, an increase in noise due to gain adjustment can be suppressed, and a substantial decrease in the frame rate can be prevented.

### <Second Mode>

The second mode of the present embodiment is an observation mode of irradiating the observation target S with the broadband light and the second narrowband light to acquire a captured image based on the broadband reflected light Lw1 and the second fluorescence Lw3 from the observation target S.

That is, the control apparatus 90 (control section 94) controls the light source apparatus 10 (the broadband light source 11 and the second narrowband light source 13), the broadband light and the second narrowband light are emitted from the light source apparatus 10 in a time division manner, and the broadband light and the second narrowband light are emitted to the observation target S in a time division manner. Note that, in this mode, the first narrowband light source 12 is placed in the OFF state, and the first narrowband light is not emitted from the light source apparatus 10.

Then, the optical element 15 guides the first light flux Lf1 including the broadband reflected light Lw1 (however, the light in the wavelength band of the first fluorescence Lw2 is partially, substantially, or completely suppressed) from the observation target S irradiated with the broadband light to the first imaging element 522a. In addition, the optical element 15 guides the second light flux Lf2 including the second fluorescence Lw3 from the observation target S irradiated with the second narrowband light to the second imaging element 522b.

As a result, the first imaging element 522a receives the first light flux Lf1 including the broadband reflected light Lw1 (however, the light in the wavelength band of the first fluorescence Lw2 is partially, substantially, or completely suppressed), and the second imaging element 522b receives the second light flux Lf2 including the second fluorescence Lw3.

Then, under the control of the control apparatus 90 (control section 94), the first imaging element 522a repeatedly outputs an image signal based on the broadband reflected light Lw1 (however, the light in the wavelength band of the first fluorescence Lw2 is partially, substantially, or completely suppressed), and the second imaging element 522b repeatedly outputs an image signal based on the second fluorescence Lw3.

Then, under the control of the control section 94, the image generation section 93 (image processing section 931) generates a captured image (high-resolution color image) of the observation target S based on the broadband reflected light Lw1 on the basis of the image signal output from the first imaging element 522a that has received the first light flux Lf1 including the broadband reflected light Lw1 (however, the light in the wavelength band of the first fluorescence Lw2 is partially, substantially, or completely suppressed). In addition, under the control of the control section 94, the image generation section 93 (image processing section 931) generates a captured image (high-sensitivity monochrome image) of the observation target S based on the second fluorescence Lw3 on the basis of the image signal output from the second imaging element 522b that has received the second light flux Lf2 including the second fluorescence Lw3. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the second fluorescence captured image in which the second substance in the observation target S is emphasized, that is, the fluorescence is emitted from the second substance in the observation target S excited by the narrowband light are acquired. Therefore, the user can compare and observe the normal light captured image and the second fluorescence captured image of the observation target S and observe a composite image (superimposed image) formed from these images via the display apparatus 70.

Note that, in the above-described example, the broadband light and the second narrowband light are emitted in a time division manner, but the light source apparatus 10 may constantly emit the broadband light and/or the second narrowband light under the control of the control apparatus 90 (control section 94).

In a case where constant emission of broadband light is performed, the first imaging element 522a is continuously exposed by the broadband reflected light Lw1 (however, the light in the wavelength band of the first fluorescence Lw2 is partially, substantially, or completely suppressed). Furthermore, in a case where the constant emission of the second narrowband light is performed, the second imaging element 522b can be continuously exposed by the second fluorescence Lw3. Therefore, in these cases, the charge accumulation amount in the imaging element increases, a bright captured image can be acquired, an increase in noise due to gain adjustment can be suppressed, and a substantial decrease in the frame rate can be prevented.

### <Third Mode>

The third mode of the present embodiment is an observation mode of irradiating the observation target S with the broadband light, the first narrowband light, and the second narrowband light to acquire a captured image based on the broadband reflected light Lw1, the first fluorescence Lw2, and the second fluorescence Lw3 from the observation target S.

That is, the control apparatus 90 (control section 94) controls the light source apparatus 10 (broadband light source 11, first narrowband light source 12, and second narrowband light source 13), and while the broadband light is continuously emitted from the light source apparatus 10, the first narrowband light and the second narrowband light are emitted from the light source apparatus 10 in a time division manner. As a result, the broadband light is continuously emitted to the observation target S, and the first narrowband light and the second narrowband light are emitted to the observation target S in a time division manner.

Then, the optical element 15 continuously guides the first light flux Lf1 including the broadband reflected light Lw1 (however, the light in the wavelength band of the first fluorescence Lw2 is partially, substantially, or completely suppressed) from the observation target S irradiated with the broadband light to the first imaging element 522a. In addition, the optical element 15 sequentially guides the second light flux Lf2 including the first fluorescence Lw2 from the observation target S irradiated with the first narrowband light and the second light flux Lf2 including the second fluorescence Lw3 from the observation target S irradiated with the second narrowband light to the second imaging element 522b.

As a result, the first imaging element 522a continuously receives the first light flux Lf1 including the broadband reflected light Lw1 (however, the light in the wavelength band of the first fluorescence Lw2 is partially, substantially, or completely suppressed), and repeatedly outputs an image signal based on the broadband reflected light Lw1 under the control of the control apparatus 90 (control section 94).

On the other hand, the second imaging element 522b sequentially receives the second light flux Lf2 including the first fluorescence Lw2 and the second light flux Lf2 including the second fluorescence Lw3. Then, the second imaging element 522b sequentially and repeatedly outputs an image signal based on the first fluorescence Lw2 and an image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 on the basis of the image signal output from the first imaging element 522a that has received the first light flux Lf1 including the broadband reflected light Lw1 (however, the light in the wavelength band of the first fluorescence Lw2 is partially, substantially, or completely suppressed). In addition, under the control of the control section 94, the image generation section 93 (image processing section 931) generates a captured image (high-sensitivity monochrome image) of the observation target S based on the first fluorescence Lw2 on the basis of the image signal output from the second imaging element 522b that has received the second light flux Lf2 including the first fluorescence Lw2. In addition, under the control of the control section 94, the image generation section 93 (image processing section 931) generates a captured image (high-sensitivity monochrome image) of the observation target S based on the second fluorescence Lw3 on the basis of the image signal output from the second imaging element 522b that has received the second light flux Lf2 including the second fluorescence Lw3. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light), and the first fluorescence captured image and the second fluorescence captured image which are images in which the first substance and the second substance in the observation target S are emphasized, that is, fluorescence is emitted from the first substance and the second substance in the observation target S excited by the narrowband light are acquired. Therefore, the user can compare and observe the normal light captured image, the first fluorescence captured image, and the second fluorescence captured image of the observation target S, and can observe a composite image (superimposed image) formed from these images.

Note that, in the above-described example, the broadband light is constantly emitted, but the light source apparatus 10 may repeatedly turn on and off the emission of the broadband light under the control of the control apparatus 90 (control section 94).

### [Fifth Embodiment]

In the present embodiment, the same or corresponding elements as those in the first to fourth embodiments described above are denoted by the same reference signs, and a detailed description thereof will be omitted.

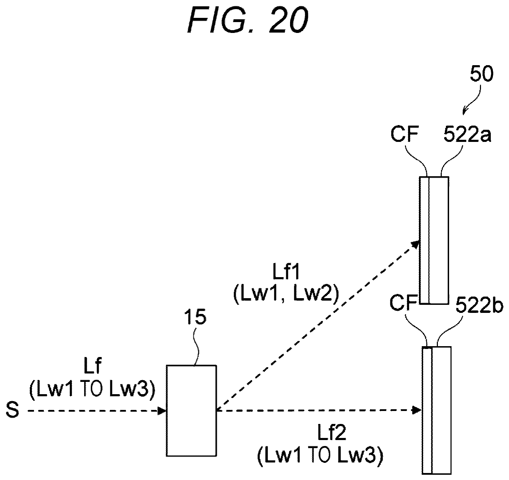

Fig. 20 is a diagram for explaining types of light incident on the imaging elements (the first imaging element 522a and the second imaging element 522b) according to the fifth embodiment.

The camera head 50 (in particular, the imaging section 52) of the present embodiment includes a two-plate type imaging module (see Figs. 3 and 4), and includes a first imaging element 522a having a color filter CF and a second imaging element 522b having a color filter CF. In particular, the color filter CF provided in the second imaging element 522b includes a filter through which the second fluorescence Lw3 can transmit in addition to the broadband reflected light Lw1 and the first fluorescence Lw2. On the other hand, the color filter CF provided in the first imaging element 522a transmits the broadband reflected light Lw1 and the first fluorescence Lw2 received by the first imaging element 522a as described later, but may or may not transmit light (for example, the second fluorescence Lw3) in a wavelength band different from the broadband reflected light Lw1 and the first fluorescence Lw2. The first imaging element 522a has relatively lower sensitivity and higher resolution (for example, 4K resolution) than the second imaging element 522b, while the second imaging element 522b has relatively higher sensitivity and lower resolution (for example, HD resolution) than the first imaging element 522a.

The light source apparatus 10 (see Fig. 1A) emits light from at least one of the broadband light source 11, the first narrowband light source 12, or the second narrowband light source 13, and can irradiate the observation target S with at least one of broadband light, first narrowband light, or second narrowband light.

Therefore, the observation light Lf from the observation target S may include broadband reflected light Lw1 which is reflected light of broadband light, first fluorescence Lw2 emitted from the first substance excited by the first narrowband light, and second fluorescence Lw3 emitted from the second substance excited by the second narrowband light. Note that the broadband reflected light Lw1 and the first fluorescence Lw2 are light included in the first wavelength band, and the second fluorescence Lw3 is light included in the second wavelength band outside the first wavelength band.

The observation light Lf incident on the optical element 15 is separated into a first light flux Lf1 and a second light flux Lf2 by the optical element 15. The optical element 15 of the present embodiment guides a part of light included in the first wavelength band to the first imaging element 522a as a first light flux Lf1, and guides a part of light included in the first wavelength band and light included in the second wavelength band to the second imaging element 522b as a second light flux Lf2.

As described above, in the present embodiment, the first light flux Lf1 in which the light in the first wavelength band is partially suppressed and the light in the wavelength band of the second fluorescence Lw3 is partially, substantially, or completely suppressed in the observation light Lf is guided to the first imaging element 522a. That is, light including at least a part of the broadband reflected light Lw1 is guided to the first imaging element 522a as the first light flux Lf1. In addition, the second light flux Lf2 in which the light of the first wavelength band is partially suppressed in the observation light Lf is guided to the second imaging element 522b. That is, light including at least a part of the broadband reflected light Lw1 and the first fluorescence Lw2 and the second fluorescence Lw3 is guided to the second imaging element 522b as the second light flux Lf2.

The medical observation system 100 of the present embodiment having the above-described configuration can acquire various captured images of the observation target S according to the following observation modes (first to third modes).

### <First Mode>

The first mode of the present embodiment is an observation mode of irradiating the observation target S with the broadband light and the first narrowband light to acquire a captured image based on the broadband reflected light Lw1 and the first fluorescence Lw2 from the observation target S.

That is, the control apparatus 90 (the control section 94 (see Fig. 7)) controls the light source apparatus 10 (the broadband light source 11 and the first narrowband light source 12 (see Fig. 1A)), the broadband light and the first narrowband light are emitted from the light source apparatus 10 in a time division manner, and the broadband light and the first narrowband light are emitted to the observation target S in a time division manner. Note that, in this mode, the second narrowband light source 13 is placed in the OFF state, and the second narrowband light is not emitted from the light source apparatus 10.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the broadband reflected light Lw1 from the observation target S irradiated with the broadband light and the second light flux Lf2 including the first fluorescence Lw2 from the observation target S irradiated with the first narrowband light to the second imaging element 522b.

As a result, the first imaging element 522a sequentially receives the first light flux Lf1 including the broadband reflected light Lw1 and the first light flux Lf1 including the first fluorescence Lw2. In addition, the second imaging element 522b sequentially receives the second light flux Lf2 including the broadband reflected light Lw1 and the second light flux Lf2 including the first fluorescence Lw2.

Then, the first imaging element 522a sequentially and repeatedly outputs the image signal based on the broadband reflected light Lw1 and the image signal based on the first fluorescence Lw2 under the control of the control apparatus 90 (control section 94 (see Fig. 7)). However, in the present example, these image signals output from the first imaging element 522a are not used for generating the captured image.

Furthermore, the second imaging element 522b sequentially and repeatedly outputs the image signal based on the broadband reflected light Lw1 and the image signal based on the first fluorescence Lw2 under the control of the control apparatus 90 (control section 94).

Then, under the control of the control section 94, the image generation section 93 (particularly, the image processing section 931 (see Fig. 7)) generates a captured image (high-sensitivity color image) of the observation target S based on the broadband reflected light Lw1 on the basis of the image signal output from the second imaging element 522b that has received the second light flux Lf2 including the broadband reflected light Lw1. In addition, under the control of the control section 94, the image generation section 93 (image processing section 931) generates a captured image (high-sensitivity color image) of the observation target S based on the first fluorescence Lw2 on the basis of the image signal output from the second imaging element 522b that has received the second light flux Lf2 including the first fluorescence Lw2. The "high-sensitivity color image" mentioned here is a color image acquired by the high-sensitivity imaging element.

As described above, in this mode, in the common time frame, the normal light captured image which is the reflected image of the visible light (white light) and the first fluorescence captured image in which the first substance in the observation target S is emphasized, that is, the fluorescence is emitted from the first substance in the observation target S excited by the narrowband light are acquired. Therefore, the user can compare and observe the normal light captured image and the first fluorescence captured image of the observation target S and observe a composite image (superimposed image) formed from these images via the display apparatus 70 (see Fig. 1A).

Note that, in the above example, the image signal output from the second imaging element 522b is used to generate the captured image based on the broadband reflected light Lw1 and the first fluorescence Lw2, but the image signal output from the first imaging element 522a may be used.

That is, the control section 94 may control the imaging section 52 (imaging element 522) and the image generation section 93 to generate an image based on the broadband reflected light Lw1 on the basis of one or both of the image signal output from the first imaging element 522a that has received the first light flux Lf1 including the broadband reflected light Lw1 and the image signal output from the second imaging element 522b that has received the second light flux Lf2 including the broadband reflected light Lw1.

For example, in a case where priority is given to resolution over sensitivity, a captured image (high-resolution color image) based on the broadband reflected light Lw1 may be generated on the basis of an image signal output from the first imaging element 522a. On the other hand, in a case where sensitivity is prioritized over resolution, a captured image (high-sensitivity color image) based on the broadband reflected light Lw1 may be generated on the basis of an image signal output from the second imaging element 522b. The "high-resolution color image" mentioned here is a color image acquired by a high-resolution imaging element, and the "high-sensitivity color image" is a color image acquired by a high-sensitivity imaging element.

As an example, in a case where the amount of broadband reflected light Lw1 is sufficient for the first imaging element 522a to perform imaging (light reception), a captured image based on the broadband reflected light Lw1 may be generated on the basis of an image signal output from the first imaging element 522a. On the other hand, in a case where the amount of broadband reflected light Lw1 is insufficient for the first imaging element 522a to perform imaging (light reception), a captured image based on the broadband reflected light Lw1 may be generated on the basis of an image signal output from the second imaging element 522b. For example, in a case where it is not desirable to irradiate the observation target S with broadband light of a large amount of light, a captured image based on the broadband reflected light Lw1 may be generated on the basis of an image signal output from the second imaging element 522b while suppressing the light emission amount of the broadband light in the light source apparatus 10.

Furthermore, the captured image based on the broadband reflected light Lw1 may be generated on the basis of the image signal output from the first imaging element 522a and the image signal output from the second imaging element 522b. For example, the image generation section 93 (image processing section 931) may generate a high-quality "captured image based on the broadband reflected light Lw1" on the basis of the image signals output from the first imaging element 522a and the second imaging element 522b using an arbitrary image composition technology.

The control section 94 may determine which one or both of the image signal output from the first imaging element 522a and the image signal output from the second imaging element 522b is used to generate the image based on the broadband reflected light Lw1 on the basis of the instruction from the user received via the input section 95.

### <Second Mode>

The second mode of the present embodiment is an observation mode of irradiating the observation target S with the broadband light and the second narrowband light to acquire a captured image based on the broadband reflected light Lw1 and the second fluorescence Lw3 from the observation target S.

That is, the control apparatus 90 (control section 94) controls the light source apparatus 10 (the broadband light source 11 and the second narrowband light source 13), the broadband light and the second narrowband light are emitted from the light source apparatus 10 in a time division manner, and the broadband light and the second narrowband light are emitted to the observation target S in a time division manner. Note that, in this mode, the first narrowband light source 12 is placed in the OFF state, and the first narrowband light is not emitted from the light source apparatus 10.

Then, the optical element 15 guides the first light flux Lf1 including the broadband reflected light Lw1 from the observation target S irradiated with the broadband light to the first imaging element 522a. In addition, the optical element 15 sequentially guides the second light flux Lf2 including the broadband reflected light Lw1 from the observation target S irradiated with the broadband light and the second light flux Lf2 including the second fluorescence Lw3 from the observation target S irradiated with the second narrowband light to the second imaging element 522b.

As a result, the first imaging element 522a receives the first light flux Lf1 including the broadband reflected light Lw1, and repeatedly outputs an image signal based on the broadband reflected light Lw1 under the control of the control apparatus 90 (control section 94). In addition, the second imaging element 522b sequentially receives the second light flux Lf2 including the broadband reflected light Lw1 and the second light flux Lf2 including the second fluorescence Lw3. Then, the second imaging element 522b sequentially and repeatedly outputs the image signal based on the broadband reflected light Lw1 and the image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 on the basis of the image signal output from the first imaging element 522a that has received the first light flux Lf1 including the broadband reflected light Lw1. In addition, under the control of the control section 94, the image generation section 93 (image processing section 931) generates a captured image (high-sensitivity image) of the observation target S based on the second fluorescence Lw3 on the basis of the image signal output from the second imaging element 522b that has received the second light flux Lf2 including the second fluorescence Lw3. The "high-resolution color image" mentioned here is a color image acquired by a high-resolution imaging element, and the "high-sensitivity image" is an image acquired by a high-sensitivity imaging element. The image based on the second fluorescence Lw3 generated in this manner is on the basis of the image signal output from the second imaging element 522b having the color filter CF and thus may be referred to as a color image, but may also be referred to as a monochrome image since it does not substantially include color information.

As described above, in this mode, in the common time frame, the normal light captured image which is the reflected image of the visible light (white light) and the second fluorescence captured image in which the second substance in the observation target S is emphasized, that is, the fluorescence is emitted from the second substance in the observation target S excited by the narrowband light are acquired. Therefore, the user can compare and observe the normal light captured image and the second fluorescence captured image of the observation target S and observe a composite image (superimposed image) formed from these images via the display apparatus 70.

Note that, in the above-described example, the broadband light and the second narrowband light are emitted in a time division manner, but the light source apparatus 10 may constantly emit the second narrowband light while repeatedly turning on and off the emission of the broadband light under the control of the control apparatus 90 (control section 94).

In this case, the light source apparatus 10 (broadband light source 11) emits broadband light so that the broadband reflected light Lw1 is exposed to the first imaging element 522a in the broadband light image frame of the first imaging element 522a. On the other hand, the light source apparatus 10 (broadband light source 11) stops emission of broadband light so that the broadband reflected light Lw1 is not exposed to the second imaging element 522b in the second fluorescence image frame of the second imaging element 522b. As a result, the image signal of the broadband light image frame can be appropriately output from the first imaging element 522a, and the image signal of the second fluorescence image frame can be appropriately output from the second imaging element 522b.

Note that, in this mode, the image signal of the broadband light image frame is also output from the second imaging element 522b, but in the above-described example, the broadband light image frame from the second imaging element 522b is not used for generating the captured image. In the above example, the image data of the broadband light image frame not used to generate the captured image is repeatedly output from the second imaging element 522b as the image signal, but the image data of the broadband light image frame not used to generate the captured image may not be output from the second imaging element 522b as the image signal.

Furthermore, in the above-described example, the captured image based on the broadband reflected light Lw1 may be generated on the basis of the image signal of the broadband light image frame from the second imaging element 522b that is not used for generating the captured image. For example, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Third Mode>

The third mode of the present embodiment is an observation mode of irradiating the observation target S with the broadband light, the first narrowband light, and the second narrowband light to acquire a captured image based on the broadband reflected light Lw1, the first fluorescence Lw2, and the second fluorescence Lw3 from the observation target S.

That is, the control apparatus 90 (control section 94) controls the light source apparatus 10 (broadband light source 11, first narrowband light source 12, and second narrowband light source 13), and the broadband light, the first narrowband light, and the second narrowband light are emitted from the light source apparatus 10 in a time division manner. As a result, the broadband light, the first narrowband light, and the second narrowband light are emitted to the observation target S in a time division manner.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the broadband reflected light Lw1 from the observation target S irradiated with the broadband light, the second light flux Lf2 including the first fluorescence Lw2 from the observation target S irradiated with the first narrowband light, and the second light flux Lf2 including the second fluorescence Lw3 from the observation target S irradiated with the second narrowband light to the second imaging element 522b.

As a result, the first imaging element 522a sequentially receives the first light flux Lf1 including the broadband reflected light Lw1 and the first light flux Lf1 including the first fluorescence Lw2. Then, the first imaging element 522a sequentially and repeatedly outputs the image signal based on the broadband reflected light Lw1 and the image signal based on the first fluorescence Lw2 under the control of the control apparatus 90 (control section 94).

On the other hand, the second imaging element 522b sequentially receives the second light flux Lf2 including the broadband reflected light Lw1, the second light flux Lf2 including the first fluorescence Lw2, and the second light flux Lf2 including the second fluorescence Lw3. Then, the second imaging element 522b sequentially and repeatedly outputs an image signal based on the broadband reflected light Lw1, an image signal based on the first fluorescence Lw2, and an image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 on the basis of the image signal output from the first imaging element 522a that has received the first light flux Lf1 including the broadband reflected light Lw1. In addition, under the control of the control section 94, the image generation section 93 (image processing section 931) generates a captured image (high-sensitivity color image) of the observation target S based on the first fluorescence Lw2 on the basis of the image signal output from the second imaging element 522b that has received the second light flux Lf2 including the first fluorescence Lw2. In addition, under the control of the control section 94, the image generation section 93 (image processing section 931) generates a captured image (high-sensitivity image) of the observation target S based on the second fluorescence Lw3 on the basis of the image signal output from the second imaging element 522b that has received the second light flux Lf2 including the second fluorescence Lw3. The "high-resolution color image" mentioned here is a color image acquired by a high-resolution imaging element, the "high-sensitivity color image" is a color image acquired by a high-sensitivity imaging element, and the "high-sensitivity image" is an image acquired by a high-sensitivity imaging element.

As described above, in this mode, in the common time frame, the normal light captured image which is the reflected image of the visible light (white light), and the first fluorescence captured image and the second fluorescence captured image which are images in which the first substance and the second substance in the observation target S are emphasized, that is, fluorescence is emitted from the first substance and the second substance in the observation target S excited by the narrowband light are acquired. Therefore, the user can compare and observe the normal light captured image, the first fluorescence captured image, and the second fluorescence captured image of the observation target S, and can observe a composite image (superimposed image) formed from these images.

Note that, in the this mode, the image signal of the first fluorescence image frame is also output from the first imaging element 522a, and the image signal of the broadband light image frame is also output from the second imaging element 522b. However, in the above-described example, these image frames are not used for generating the captured image. In the above example, the image data of these image frames not used to generate the captured image is also repeatedly output as the image signal from the imaging element (the first imaging element 522a and the second imaging element 522b), but the image data of the image frames not used to generate the captured image may not be output as the image signal from the imaging element.

In addition, in the above example, these image frames (that is, the first fluorescence image frame from the first imaging element 522a and the broadband light image frame from the second imaging element 522b) that are not used to generate the captured image may be used to generate the captured image.

That is, the captured image based on the first fluorescence Lw2 may be generated on the basis of the image signal of the first fluorescence image frame from the first imaging element 522a. For example, the captured image based on the first fluorescence Lw2 may be generated on the basis of the image signal of the first fluorescence image frame from both the first imaging element 522a and the second imaging element 522b.

In addition, the captured image based on the broadband reflected light Lw1 may be generated on the basis of the image signal of the broadband light image frame from the second imaging element 522b. For example, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### [Sixth Embodiment]

In the present embodiment, the same or corresponding elements as those in the first to fifth embodiments described above are denoted by the same reference signs, and a detailed description thereof will be omitted.

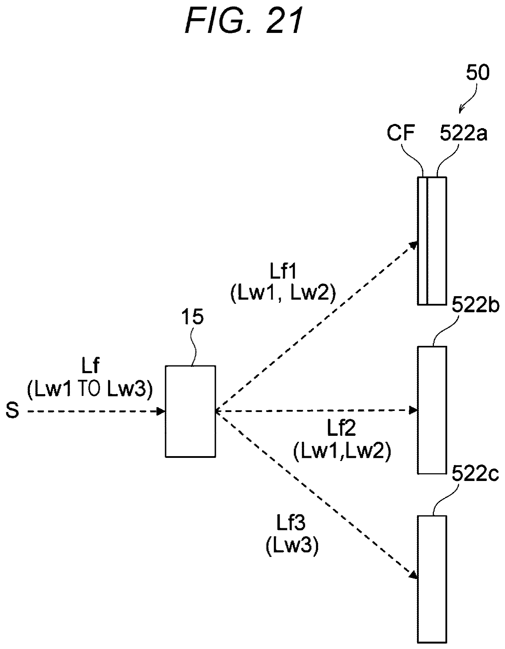

Fig. 21 is a diagram for explaining types of light incident on the imaging element (first imaging element 522a, second imaging element 522b, and third imaging element 522c) according to the sixth embodiment.

The camera head 50 (in particular, the imaging section 52) of the present embodiment includes a three-plate type imaging module (see Figs. 5 and 6), and includes a first imaging element 522a having a color filter CF, and a second imaging element 522b and a third imaging element 522c not having the color filter CF.

The color filter CF provided in the first imaging element 522a of the present embodiment transmits the broadband reflected light Lw1 and the first fluorescence Lw2 received by the first imaging element 522a as described later, but may or may not transmit light (for example, the second fluorescence Lw3) in a wavelength band different from the broadband reflected light Lw1 and the first fluorescence Lw2. Note that the second imaging element 522b and the third imaging element 522c of the present example do not include the color filter CF, but the second imaging element 522b may include the color filter CF capable of transmitting the broadband reflected light Lw1 and the first fluorescence Lw2, and the third imaging element 522c may include the color filter CF capable of transmitting the second fluorescence Lw3.

The first imaging element 522a has relatively lower sensitivity and higher resolution (for example, 4K resolution) than the second imaging element 522b and the third imaging element 522c, while the second imaging element 522b and the third imaging element 522c have relatively higher sensitivity and lower resolution (for example, HD resolution) than the first imaging element 522a. The second imaging element 522b and the third imaging element 522c may have the same characteristics or different characteristics from each other. As described above, the resolution of the first imaging element 522a is higher than the resolutions of the second imaging element 522b and the third imaging element 522c, but the sensitivity of the second imaging element 522b and the third imaging element 522c is higher than the sensitivity of the first imaging element 522a.

However, the resolution and sensitivity of the first imaging element 522a to the third imaging element 522c are not limited thereto, and the relationship between the resolution and sensitivity of the first imaging element 522a to the third imaging element 522c is not limited thereto.

The light source apparatus 10 (see Fig. 1A) emits light from at least one of the broadband light source 11, the first narrowband light source 12, or the second narrowband light source 13, and can irradiate the observation target S with at least one of broadband light, first narrowband light, or second narrowband light. Therefore, the observation light Lf from the observation target S may include broadband reflected light Lw1 which is reflected light of broadband light, first fluorescence Lw2 emitted from the first substance excited by the first narrowband light, and second fluorescence Lw3 emitted from the second substance excited by the second narrowband light. Note that the broadband reflected light Lw1 and the first fluorescence Lw2 are light included in the first wavelength band, and the second fluorescence Lw3 is light included in the second wavelength band outside the first wavelength band.

The observation light Lf incident on the optical element 15 is separated into the first light flux Lf1, the second light flux Lf2, and the third light flux Lf3 by the optical element 15. The optical element 15 of the present embodiment guides a part of light included in the first wavelength band to the first imaging element 522a as a first light flux Lf1, guides a part of light included in the first wavelength band to the second imaging element 522b as a second light flux Lf2, and guides light included in the second wavelength band to the third imaging element 522c as a third light flux Lf3.

As described above, in the present embodiment, the first light flux Lf1 in which the light in the first wavelength band is partially suppressed and the light in the wavelength band of the second fluorescence Lw3 is partially, substantially, or completely suppressed in the observation light Lf is guided to the first imaging element 522a by the optical element 15. That is, light including at least a part of the broadband reflected light Lw1 is guided to the first imaging element 522a as the first light flux Lf1. Furthermore, in the observation light Lf, the second light flux Lf2 in which the light in the first wavelength band is partially suppressed and the light in the wavelength band of the second fluorescence Lw3 is partially, substantially, or completely suppressed is guided to the second imaging element 522b. That is, light including at least a part of the broadband reflected light Lw1 and the first fluorescence Lw2 is guided to the second imaging element 522b as the second light flux Lf2. Furthermore, the third light flux Lf3 in which the light in the first wavelength band is partially, substantially, or completely suppressed in the observation light Lf is guided to the third imaging element 522c. That is, light including at least the second fluorescence Lw3 is guided to the third imaging element 522c as the third light flux Lf3.

The medical observation system 100 of the present embodiment having the above-described configuration can acquire various captured images of the observation target S according to the following observation modes (the first mode and the second mode).

### <First Mode>

The first mode of the present embodiment is an observation mode of irradiating the observation target S with the broadband light and the first narrowband light to acquire a captured image based on the broadband reflected light Lw1 and the first fluorescence Lw2 from the observation target S.

That is, the control apparatus 90 (the control section 94 (see Fig. 7)) controls the light source apparatus 10 (the broadband light source 11 and the first narrowband light source 12 (see Fig. 1A)), the broadband light and the first narrowband light are emitted from the light source apparatus 10 in a time division manner, and the broadband light and the first narrowband light are emitted to the observation target S in a time division manner. Note that, in this mode, the second narrowband light source 13 is placed in the OFF state, and the second narrowband light is not emitted from the light source apparatus 10.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. As a result, the first imaging element 522a sequentially receives the first light flux Lf1 including the broadband reflected light Lw1 and the first light flux Lf1 including the first fluorescence Lw2.

In addition, the optical element 15 sequentially guides the second light flux Lf2 including the broadband reflected light Lw1 from the observation target S irradiated with the broadband light and the second light flux Lf2 including the first fluorescence Lw2 from the observation target S irradiated with the first narrowband light to the second imaging element 522b. As a result, the second imaging element 522b sequentially receives the second light flux Lf2 including the broadband reflected light Lw1 and the second light flux Lf2 including the first fluorescence Lw2.

Then, each of the first imaging element 522a and the second imaging element 522b sequentially and repeatedly outputs the image signal based on the broadband reflected light Lw1 and the image signal based on the first fluorescence Lw2 under the control of the control apparatus 90 (control section 94 (see Fig. 7)).

Then, under the control of the control section 94, the image generation section 93 (particularly, the image processing section 931 (see Fig. 7)) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the first fluorescence Lw2 from the image signal based on the first fluorescence Lw2 output from the second imaging element 522b. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the first fluorescence captured image in which the first substance in the observation target S is emphasized, that is, the fluorescence is emitted from the first substance in the observation target S excited by the narrowband light are acquired. Therefore, the user can compare and observe the normal light captured image and the first fluorescence captured image of the observation target S and observe a composite image (superimposed image) formed from these images via the display apparatus 70 (see Fig. 1A).

Note that, in the this mode, the image signal of the first fluorescence image frame is also output from the first imaging element 522a, and the image signal of the broadband light image frame is also output from the second imaging element 522b. However, in the above-described example, these image frames are not used for generating the captured image. In the above example, the image data of these image frames not used to generate the captured image is also repeatedly output as the image signal from the imaging element (the first imaging element 522a and the second imaging element 522b), but the image data of the image frames not used to generate the captured image may not be output as the image signal from the imaging element.

In addition, in the above example, these image frames (that is, the first fluorescence image frame from the first imaging element 522a and the broadband light image frame from the second imaging element 522b) that are not used to generate the captured image may be used to generate the captured image.

That is, the captured image based on the first fluorescence Lw2 may be generated on the basis of the image signal of the first fluorescence image frame from the first imaging element 522a. For example, the captured image based on the first fluorescence Lw2 may be generated on the basis of the image signal of the first fluorescence image frame from both the first imaging element 522a and the second imaging element 522b.

In addition, the captured image based on the broadband reflected light Lw1 may be generated on the basis of the image signal of the broadband light image frame from the second imaging element 522b. That is, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Second Mode>

The second mode of the present embodiment is an observation mode of irradiating the observation target S with the broadband light and the second narrowband light to acquire a captured image based on the broadband reflected light Lw1 and the second fluorescence Lw3 from the observation target S.

That is, the control apparatus 90 (the control section 94) controls the light source apparatus 10 (the broadband light source 11 and the second narrowband light source 13), the broadband light and the second narrowband light are continuously emitted from the light source apparatus 10, and the observation target S is continuously irradiated with the broadband light and the second narrowband light. Note that, in this mode, the first narrowband light source 12 is placed in the OFF state, and the first narrowband light is not emitted from the light source apparatus 10.

Then, the optical element 15 continuously guides the first light flux Lf1 including the broadband reflected light Lw1 from the observation target S irradiated with the broadband light to the first imaging element 522a, and continuously guides the second light flux Lf2 including the broadband reflected light Lw1 to the second imaging element 522b. In addition, the optical element 15 continuously guides the third light flux Lf3 including the second fluorescence Lw3 from the observation target S irradiated with the second narrowband light to the third imaging element 522c.

As a result, the first imaging element 522a continuously receives the first light flux Lf1 including the broadband reflected light Lw1, and the second imaging element 522b continuously receives the second light flux Lf2 including the broadband reflected light Lw1. In addition, the third imaging element 522c continuously receives the third light flux Lf3 including the second fluorescence Lw3.

Then, under the control of the control apparatus 90 (control section 94), the first imaging element 522a and the second imaging element 522b continuously repeatedly output an image signal based on the broadband reflected light Lw1, and the third imaging element 522c continuously repeatedly outputs an image signal based on the second fluorescence Lw3.

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the third imaging element 522c. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the second fluorescence captured image in which the second substance in the observation target S is emphasized, that is, the fluorescence is emitted from the second substance in the observation target S excited by the narrowband light are acquired. Therefore, the user can compare and observe the normal light captured image and the second fluorescence captured image of the observation target S and observe a composite image (superimposed image) formed from these images via the display apparatus 70.

Note that, in the above example, the broadband light and the second narrowband light are constantly emitted from the light source apparatus 10, but the light source apparatus 10 may turn off the emission of each of the broadband light and the second narrowband light in the middle under the control of the control apparatus 90 (control section 94). For example, the light source apparatus 10 may repeatedly turn on and off the emission of the broadband light and the second narrowband light, and may emit the broadband light and the second narrowband light in a time division manner.

However, by constantly emitting the broadband light and the second narrowband light by the light source apparatus 10, the first imaging element 522a can continuously receive the broadband reflected light Lw1, and the third imaging element 522c can continuously receive the second fluorescence Lw3. As a result, the charge accumulation amount in the imaging element increases, a bright captured image can be acquired, an increase in noise due to gain adjustment can be suppressed, and a substantial decrease in the frame rate can be prevented.

Note that, in this mode, the image signal of the broadband light image frame is also output from the second imaging element 522b, but in the above-described example, the broadband light image frame from the second imaging element 522b is not used for generating the captured image. In the above example, the image data of the broadband light image frame not used to generate the captured image is repeatedly output from the second imaging element 522b as the image signal, but the image data of the broadband light image frame not used to generate the captured image may not be output from the second imaging element 522b as the image signal.

Furthermore, in the above-described example, the captured image based on the broadband reflected light Lw1 may be generated on the basis of the image signal of the broadband light image frame from the second imaging element 522b that is not used for generating the captured image. That is, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### [Seventh Embodiment]

In the present embodiment, the same or corresponding elements as those in the first to sixth embodiments described above are denoted by the same reference signs, and a detailed description thereof will be omitted.

The imaging element (first imaging element 522a, second imaging element 522b, and third imaging element 522c) according to the seventh embodiment has the same configuration as the imaging element (see Fig. 21) according to the sixth embodiment described above.

That is, the camera head 50 (in particular, the imaging section 52) of the present embodiment includes a three-plate type imaging module (see Figs. 5 and 6), and includes the first imaging element 522a having the color filter CF, and the second imaging element 522b and the third imaging element 522c not having the color filter CF.

The color filter CF provided in the first imaging element 522a of the present embodiment transmits the broadband reflected light Lw1 and the first fluorescence Lw2 received by the first imaging element 522a as described later, but may or may not transmit light (for example, the second fluorescence Lw3) in a wavelength band different from the broadband reflected light Lw1 and the first fluorescence Lw2. Note that the second imaging element 522b and the third imaging element 522c of the present example do not include the color filter CF, but the second imaging element 522b may include the color filter CF capable of transmitting the broadband reflected light Lw1 and the first fluorescence Lw2, and the third imaging element 522c may include the color filter CF capable of transmitting the second fluorescence Lw3.

The first imaging element 522a has relatively lower sensitivity and higher resolution (for example, 4K resolution) than the second imaging element 522b and the third imaging element 522c, while the second imaging element 522b and the third imaging element 522c have relatively higher sensitivity and lower resolution (for example, HD resolution) than the first imaging element 522a. The second imaging element 522b and the third imaging element 522c may have the same characteristics or different characteristics from each other. As described above, the resolution of the first imaging element 522a is higher than the resolutions of the second imaging element 522b and the third imaging element 522c, but the sensitivity of the second imaging element 522b and the third imaging element 522c is higher than the sensitivity of the first imaging element 522a.

However, the resolution and sensitivity of the first imaging element 522a to the third imaging element 522c are not limited thereto, and the relationship between the resolution and sensitivity of the first imaging element 522a to the third imaging element 522c is not limited thereto.

The light source apparatus 10 (see Fig. 1A) emits light from at least one of the broadband light source 11, the first narrowband light source 12, or the second narrowband light source 13, and can irradiate the observation target S with at least one of broadband light, first narrowband light, or second narrowband light. Therefore, the observation light Lf from the observation target S may include broadband reflected light Lw1 which is reflected light of broadband light, first fluorescence Lw2 emitted from the first substance excited by the first narrowband light, and second fluorescence Lw3 emitted from the second substance excited by the second narrowband light. Note that the broadband reflected light Lw1 and the first fluorescence Lw2 are light included in the first wavelength band, and the second fluorescence Lw3 is light included in the second wavelength band outside the first wavelength band.

The observation light Lf incident on the optical element 15 is separated into the first light flux Lf1, the second light flux Lf2, and the third light flux Lf3 by the optical element 15. The optical element 15 of the present embodiment guides a part of light included in the first wavelength band to the first imaging element 522a as a first light flux Lf1, guides a part of light included in the first wavelength band to the second imaging element 522b as a second light flux Lf2, and guides light included in the second wavelength band to the third imaging element 522c as a third light flux Lf3.

As described above, in the present embodiment, the first light flux Lf1 in which the light in the first wavelength band is partially suppressed and the light in the wavelength band of the second fluorescence Lw3 is partially, substantially, or completely suppressed in the observation light Lf is guided to the first imaging element 522a by the optical element 15. That is, light including at least a part of the broadband reflected light Lw1 is guided to the first imaging element 522a as the first light flux Lf1. Furthermore, in the observation light Lf, the second light flux Lf2 in which the light in the first wavelength band is partially suppressed and the light in the wavelength band of the second fluorescence Lw3 is partially, substantially, or completely suppressed is guided to the second imaging element 522b. That is, light including at least a part of the broadband reflected light Lw1 and the first fluorescence Lw2 is guided to the second imaging element 522b as the second light flux Lf2. Furthermore, the third light flux Lf3 in which the light in the first wavelength band is partially, substantially, or completely suppressed in the observation light Lf is guided to the third imaging element 522c. That is, light including at least the second fluorescence Lw3 is guided to the third imaging element 522c as the third light flux Lf3.

The medical observation system 100 of the present embodiment having the above-described configuration can acquire various captured images of the observation target S according to the following observation modes (first to third modes).

### <First Mode and Second Mode>

The first mode and the second mode of the present embodiment are performed similarly to the first mode and the second mode of the sixth embodiment described above.

Therefore, in the first mode, the broadband light and the first narrowband light are emitted in a time division manner by the light source apparatus 10, and the broadband reflected light Lw1 and the first fluorescence Lw2 are received in a time division manner by the first imaging element 522a and the second imaging element 522b. Furthermore, in the second mode, the broadband light and the second narrowband light are continuously emitted by the light source apparatus 10, the broadband reflected light Lw1 is continuously received by the first imaging element 522a and the second imaging element 522b, and the second fluorescence Lw3 is continuously received by the third imaging element 522c.

### <Third Mode>

The third mode of the present embodiment is an observation mode of irradiating the observation target S with the broadband light, the first narrowband light, and the second narrowband light to acquire a captured image based on the broadband reflected light Lw1, the first fluorescence Lw2, and the second fluorescence Lw3 from the observation target S.

That is, the control apparatus 90 (control section 94) controls the light source apparatus 10 (broadband light source 11, first narrowband light source 12, and second narrowband light source 13), the broadband light and the first narrowband light are emitted from the light source apparatus 10 in a time division manner, and the second narrowband light is continuously emitted. As a result, the broadband light and the first narrowband light are emitted to the observation target S in a time division manner, and the second narrowband light is continuously emitted to the observation target S.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the broadband reflected light Lw1 from the observation target S irradiated with the broadband light and the second light flux Lf2 including the first fluorescence Lw2 from the observation target S irradiated with the first narrowband light to the second imaging element 522b. In addition, the optical element 15 continuously guides the third light flux Lf3 including the second fluorescence Lw3 from the observation target S irradiated with the second narrowband light to the third imaging element 522c.

As a result, the first imaging element 522a sequentially receives the first light flux Lf1 including the broadband reflected light Lw1 and the first light flux Lf1 including the first fluorescence Lw2. In addition, the second imaging element 522b sequentially receives the second light flux Lf2 including the broadband reflected light Lw1 and the second light flux Lf2 including the first fluorescence Lw2. In addition, the third imaging element 522c continuously receives the third light flux Lf3 including the second fluorescence Lw3.

Then, each of the first imaging element 522a and the second imaging element 522b sequentially and repeatedly outputs the image signal based on the broadband reflected light Lw1 and the image signal based on the first fluorescence Lw2 under the control of the control apparatus 90 (control section 94). Furthermore, the second imaging element 522b continuously repeatedly outputs an image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the first fluorescence Lw2 from the image signal based on the first fluorescence Lw2 output from the second imaging element 522b. In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the third imaging element 522c. The "high-resolution color image" mentioned here is a color image acquired by the high-resolution imaging element, and the "high-sensitivity monochrome image" is a monochrome image acquired by the high-sensitivity imaging element.

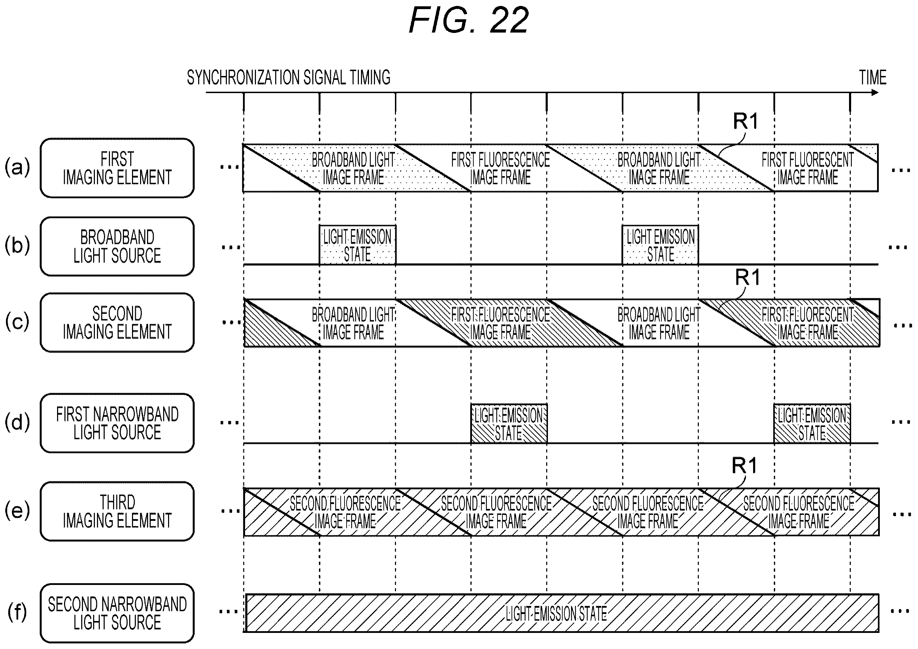

Fig. 22 illustrates an example of a timing chart of light source light emission and imaging element exposure in the third mode of the seventh embodiment.

(a) of Fig. 22 illustrates an exposure state of the first imaging element 522a, (c) illustrates an exposure state of the second imaging element 522b, and (e) illustrates an exposure state of the third imaging element 522c. In (a), (c), and (e) of Fig. 22, the vertical axis represents a horizontal line of the first imaging element 522a, the second imaging element 522b, and the third imaging element 522c, the horizontal axis represents time, and line R1 represents pixel data read start timing of each horizontal line regarding each image frame. The "broadband light image frame" between the lines R1 is an image frame for receiving (exposing) the broadband reflected light Lw1 from the observation target S. The "first fluorescence image frame" between the lines R1 is an image frame for receiving (exposing) the first fluorescence Lw2 from the observation target S. The "second fluorescence image frame" between the lines R1 is an image frame for receiving (exposing) the second fluorescence Lw3 from the observation target S. (b) of Fig. 22 illustrates the light emission timing of the broadband light in the broadband light source 11, (d) illustrates the light emission timing of the first narrowband light in the first narrowband light source 12, and (f) illustrates the light emission timing of the second narrowband light in the second narrowband light source 13.

In this mode, as described above, the light emission in the light source apparatus 10 and the irradiation of the observation target S with respect to the broadband light and the first narrowband light are performed in a time division manner. On the other hand, light emission in the light source apparatus 10 and irradiation of the observation target S with respect to the second narrowband light are continuously performed.

Therefore, the exposure (light reception) in the first imaging element 522a and the second imaging element 522b regarding the broadband reflected light Lw1 and the first fluorescence Lw2 from the observation target S is performed in a time division manner. On the other hand, exposure (light reception) of the second fluorescence Lw3 from the observation target S in the third imaging element 522c is continuously performed. Therefore, control is performed by the control apparatus 90 (the control section 94) so that timings of the time-division light emission of the broadband light and the first narrowband light from the light source apparatus 10 and timings of reading the image data from the first imaging element 522a and the second imaging element 522b are associated with each other.

Specifically, on the basis of a common synchronization signal, time-division light emission of broadband light and first narrowband light in the broadband light source 11 and the first narrowband light source 12 is performed, and exposure and image data reading in the first imaging element 522a and the second imaging element 522b are performed. More specifically, the broadband light and the first narrowband light are alternately emitted separately in time so that the first imaging element 522a and the second imaging element 522b are not simultaneously exposed by both the broadband reflected light Lw1 and the first fluorescence Lw2.

Then, image data is read such that an image signal of a broadband light image frame exposed by the broadband reflected light Lw1 and an image signal of a first fluorescence image frame exposed by the first fluorescence Lw2 are output from the first imaging element 522a and the second imaging element 522b. As a result, each of the first imaging element 522a and the second imaging element 522b alternately repeatedly outputs the image signal of the broadband light image frame and the image signal of the first fluorescence image frame.

On the other hand, reading of image data from the third imaging element 522c can be executed at any timing while the second narrowband light is emitted by the light source apparatus 10. As a result, the third imaging element 522c continuously repeatedly outputs the image signal of the second fluorescence image frame.

Then, the image generation section 93 (image processing section 931) generates a normal light captured image of the observation target S, which is a reflected image of broadband light (white light), from the image signal of the broadband light image frame output from the first imaging element 522a. In addition, the image generation section 93 (image processing section 931) generates a first fluorescence captured image, which is a captured image based on the first fluorescence Lw2 of the observation target S, from the image signal of the first fluorescence image frame output from the second imaging element 522b. In addition, the image generation section 93 (image processing section 931) generates a second fluorescence captured image, which is a captured image based on the second fluorescence Lw3 of the observation target S, from the image signal of the second fluorescence image frame from the third imaging element 522c.

As described above, in this mode, in the common time frame, the normal light captured image which is the reflected image of the visible light (white light), the first fluorescence captured image which is the image in which the first substance in the observation target S is emphasized, that is, the fluorescence is emitted from the first substance in the observation target S excited by the narrowband light, and the second fluorescence captured image which is the image in which the second substance in the observation target S is emphasized, that is, the fluorescence is emitted from the second substance in the observation target S excited by the narrowband light are acquired. Therefore, the user can compare and observe the normal light captured image, the first fluorescence captured image, and the second fluorescence captured image of the observation target S or observe a composite image (superimposed image) formed from these images via the display apparatus 70.

Note that, in the example illustrated in Fig. 22, while the image data is not read from the first imaging element 522a and the second imaging element 522b, the emission of the broadband light and the first narrowband light is started and terminated. Therefore, in the broadband light image frames of the first imaging element 522a and the second imaging element 522b, the first fluorescence Lw2 from the observation target S irradiated with the first narrowband light can be suppressed from entering the first imaging element 522a. Furthermore, in the first fluorescence image frames of the first imaging element 522a and the second imaging element 522b, the broadband reflected light Lw1 from the observation target S irradiated with the broadband light can be suppressed from entering the first imaging element 522a and the second imaging element 522b.

Note that the start timing and the end timing of the light emission of the broadband light and the first narrowband light are not limited to the example illustrated in Fig. 22, and can be set to arbitrary timing. For example, while image data is being read from the first imaging element 522a and the second imaging element 522b (see "R1" in Fig. 22), emission of one or both of the broadband light and the first narrowband light may be started or ended. For example, in a case where the intensity of fluorescence that is a light receiving target in the first fluorescence image frame is weak (that is, in a case where the amount of fluorescence emission is small), the first narrowband light may be emitted from the first narrowband light source 12 while image data of the broadband light image frame is being read. In this case, the exposure time of fluorescence in the first fluorescence image frame can be lengthened, which is advantageous for obtaining image data of a bright first fluorescence image frame. In particular, in a case where the first fluorescence Lw2 has a sufficiently smaller light amount than the broadband reflected light Lw1 and the influence of the first fluorescence Lw2 on the broadband light image frame is sufficiently smaller than the influence of the broadband reflected light Lw1 on the broadband light image frame, the first narrowband light source 12 may constantly emit the first narrowband light.

Note that, in the this mode, the image signal of the first fluorescence image frame is also output from the first imaging element 522a, and the image signal of the broadband light image frame is also output from the second imaging element 522b. However, in the above-described example, these image frames are not used for generating the captured image. In the above example, the image data of these image frames not used to generate the captured image is also repeatedly output as the image signal from the imaging element (the first imaging element 522a and the second imaging element 522b), but the image data of the image frames not used to generate the captured image may not be output as the image signal from the imaging element.

In addition, in the above example, these image frames (that is, the first fluorescence image frame from the first imaging element 522a and the broadband light image frame from the second imaging element 522b) that are not used to generate the captured image may be used to generate the captured image.

That is, the captured image based on the first fluorescence Lw2 may be generated on the basis of the image signal of the first fluorescence image frame from the first imaging element 522a. For example, the captured image based on the first fluorescence Lw2 may be generated on the basis of the image signal of the first fluorescence image frame from both the first imaging element 522a and the second imaging element 522b.

In addition, the captured image based on the broadband reflected light Lw1 may be generated on the basis of the image signal of the broadband light image frame from the second imaging element 522b. That is, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### [Eighth Embodiment]

In the present embodiment, the same or corresponding elements as those in the first to seventh embodiments described above are denoted by the same reference signs, and a detailed description thereof will be omitted.

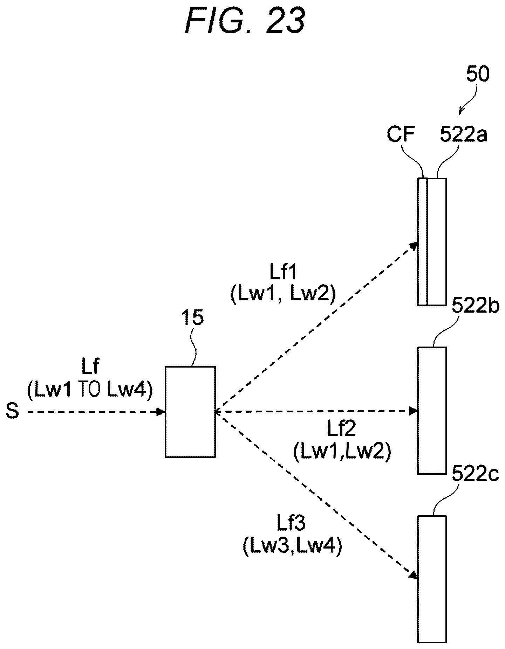

Fig. 23 is a diagram for explaining types of light incident on the imaging element (first imaging element 522a, second imaging element 522b, and third imaging element 522c) according to the eighth embodiment.

The camera head 50 (in particular, the imaging section 52) of the present embodiment includes a three-plate type imaging module (see Figs. 5 and 6), and includes a first imaging element 522a having a color filter CF, and a second imaging element 522b and a third imaging element 522c not having the color filter CF.

The color filter CF provided in the first imaging element 522a of the present embodiment transmits the broadband reflected light Lw1 and the first fluorescence Lw2 received by the first imaging element 522a as described later, but may or may not transmit light (for example, the second fluorescence Lw3 and the third fluorescence Lw4) in a wavelength band different from the broadband reflected light Lw1 and the first fluorescence Lw2. Note that the second imaging element 522b and the third imaging element 522c of the present example do not include the color filter CF, but the second imaging element 522b may include the color filter CF capable of transmitting the broadband reflected light Lw1 and the first fluorescence Lw2, and the third imaging element 522c may include the color filter CF capable of transmitting the second fluorescence Lw3 and the third fluorescence Lw4.

The first imaging element 522a has relatively lower sensitivity and higher resolution (for example, 4K resolution) than the second imaging element 522b and the third imaging element 522c, while the second imaging element 522b and the third imaging element 522c have relatively higher sensitivity and lower resolution (for example, HD resolution) than the first imaging element 522a. The second imaging element 522b and the third imaging element 522c may have the same characteristics or different characteristics from each other. As described above, the resolution of the first imaging element 522a is higher than the resolutions of the second imaging element 522b and the third imaging element 522c, but the sensitivity of the second imaging element 522b and the third imaging element 522c is higher than the sensitivity of the first imaging element 522a.

However, the resolution and sensitivity of the first imaging element 522a to the third imaging element 522c are not limited thereto, and the relationship between the resolution and sensitivity of the first imaging element 522a to the third imaging element 522c is not limited thereto.

The light source apparatus 10 (see Fig. 1B) emits light from at least one of the broadband light source 11, the first narrowband light source 12, the second narrowband light source 13, or the third narrowband light source 14, and can irradiate the observation target S with at least one of broadband light, first narrowband light, second narrowband light, or third narrowband light. Therefore, the observation light Lf from the observation target S may include broadband reflected light Lw1 which is reflected light of broadband light, first fluorescence Lw2 emitted from the first substance excited by the first narrowband light, second fluorescence Lw3 emitted from the second substance excited by the second narrowband light, and third fluorescence Lw4 emitted from the third substance excited by the third narrowband light. Note that the broadband reflected light Lw1 and the first fluorescence Lw2 are light included in the first wavelength band, and the second fluorescence Lw3 and the third fluorescence Lw4 are light included in the second wavelength band outside the first wavelength band.

The observation light Lf incident on the optical element 15 is separated into the first light flux Lf1, the second light flux Lf2, and the third light flux Lf3 by the optical element 15. The optical element 15 of the present embodiment guides a part of light included in the first wavelength band to the first imaging element 522a as a first light flux Lf1, guides a part of light included in the first wavelength band to the second imaging element 522b as a second light flux Lf2, and guides light included in the second wavelength band to the third imaging element 522c as a third light flux Lf3.

As described above, in the present embodiment, the first light flux Lf1 in which the light in the first wavelength band in the observation light Lf is partially suppressed and the light in the wavelength bands of the second fluorescence Lw3 and the third fluorescence Lw4 is partially, substantially, or completely suppressed is guided to the first imaging element 522a by the optical element 15. That is, light including at least a part of the broadband reflected light Lw1 is guided to the first imaging element 522a as the first light flux Lf1. Furthermore, in the observation light Lf, the second light flux Lf2 in which the light in the first wavelength band is partially suppressed and the light in the wavelength bands of the second fluorescence Lw3 and the third fluorescence Lw4 is partially, substantially, or completely suppressed is guided to the second imaging element 522b. That is, light including at least a part of the broadband reflected light Lw1 and the first fluorescence Lw2 is guided to the second imaging element 522b as the second light flux Lf2. Furthermore, the third light flux Lf3 in which the light in the first wavelength band is partially, substantially, or completely suppressed in the observation light Lf is guided to the third imaging element 522c. That is, light including at least the second fluorescence Lw3 and the third fluorescence Lw4 is guided to the third imaging element 522c as the third light flux Lf3.

The medical observation system 100 of the present embodiment having the above-described configuration can acquire various captured images of the observation target S according to the following observation modes (first to fifth modes).

### <First Mode to Third Mode>

The first mode to the third mode of the present embodiment are performed similarly to the first mode to the third mode of the seventh embodiment described above.

Therefore, in the first mode, the broadband light and the first narrowband light are emitted in a time division manner by the light source apparatus 10, and the broadband reflected light Lw1 and the first fluorescence Lw2 are received in a time division manner by the first imaging element 522a and the second imaging element 522b. Furthermore, in the second mode, the broadband light and the second narrowband light are continuously emitted by the light source apparatus 10, the broadband reflected light Lw1 is continuously received by the first imaging element 522a and the second imaging element 522b, and the second fluorescence Lw3 is continuously received by the third imaging element 522c. Furthermore, in the third mode, the light source apparatus 10 emits broadband light and first narrowband light in a time division manner and continuously emits second narrowband light, and the broadband reflected light Lw1 and the first fluorescence Lw2 are received by the first imaging element 522a and the second imaging element 522b in a time division manner and the second fluorescence Lw3 is continuously received by the third imaging element 522c.

In the first mode to the third mode of the present embodiment, the third narrowband light source 14 is placed in the OFF state, and the third narrowband light is not emitted from the light source apparatus 10.

### <Fourth Mode>

The fourth mode of the present embodiment is an observation mode of irradiating the observation target S with the broadband light and the third narrowband light to acquire a captured image based on the broadband reflected light Lw1 and the third fluorescence Lw4 from the observation target S.

That is, the control apparatus 90 (control section 94) controls the light source apparatus 10 (the broadband light source 11 and the third narrowband light source 14), the broadband light and the third narrowband light are continuously emitted from the light source apparatus 10, and the broadband light and the third narrowband light are continuously emitted to the observation target S. Note that, in this mode, the first narrowband light source 12 and the second narrowband light source 13 are placed in the OFF state, and the first narrowband light and the second narrowband light are not emitted from the light source apparatus 10.

Then, the optical element 15 continuously guides the first light flux Lf1 including the broadband reflected light Lw1 from the observation target S irradiated with the broadband light to the first imaging element 522a. In addition, the optical element 15 continuously guides the second light flux Lf2 including the broadband reflected light Lw1 from the observation target S irradiated with the broadband light to the second imaging element 522b. In addition, the optical element 15 continuously guides the third light flux Lf3 including the third fluorescence Lw4 from the observation target S irradiated with the third narrowband light to the third imaging element 522c.

As a result, the first imaging element 522a continuously receives the first light flux Lf1 including the broadband reflected light Lw1, and continuously and repeatedly outputs an image signal based on the broadband reflected light Lw1 under the control of the control apparatus 90 (control section 94). Furthermore, the second imaging element 522b continuously receives the second light flux Lf2 including the broadband reflected light Lw1, and continuously and repeatedly outputs an image signal based on the broadband reflected light Lw1 under the control of the control apparatus 90 (control section 94). Furthermore, the third imaging element 522c continuously receives the third light flux Lf3 including the third fluorescence Lw4, and continuously and repeatedly outputs an image signal based on the third fluorescence Lw4 under the control of the control apparatus 90 (control section 94).

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the third fluorescence Lw4 from the image signal based on the third fluorescence Lw4 output from the third imaging element 522c. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the third fluorescence captured image in which the third substance in the observation target S is emphasized, that is, the third substance in the observation target S excited by the narrowband light emits fluorescence are acquired. Therefore, the user can compare and observe the normal light captured image and the third fluorescence captured image of the observation target S and observe a composite image (superimposed image) formed from these images via the display apparatus 70.

Note that, in this mode, the image signal of the broadband light image frame is also output from the second imaging element 522b, but in the above-described example, the broadband light image frame from the second imaging element 522b is not used for generating the captured image. In the above example, the image data of the broadband light image frame not used to generate the captured image is repeatedly output from the second imaging element 522b as the image signal, but the image data of the broadband light image frame not used to generate the captured image may not be output from the second imaging element 522b as the image signal.

Furthermore, in the above-described example, the captured image based on the broadband reflected light Lw1 may be generated on the basis of the image signal of the broadband light image frame from the second imaging element 522b that is not used for generating the captured image. That is, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Fifth Mode>

The fifth mode of the present embodiment is an observation mode of irradiating the observation target S with the broadband light and the first to third narrowband light to acquire a captured image based on the broadband reflected light Lw1 and the first fluorescence Lw2 to the third fluorescence Lw4 from the observation target S.

That is, the control apparatus 90 (control section 94) controls the light source apparatus 10 (the broadband light source 11 and the first to third narrowband light sources 12 to 14), and the broadband light and the first narrowband light are emitted from the light source apparatus 10 in a time division manner, and the second narrowband light and the third narrowband light are emitted in a time division manner. As a result, the broadband light and the first narrowband light are emitted to the observation target S in a time division manner, and the second narrowband light and the third narrowband light are emitted to the observation target S in a time division manner. Note that each of the broadband light and the first narrowband light may be simultaneously emitted with one of the second narrowband light and the third narrowband light, and simultaneously emitted to the observation target S.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the broadband reflected light Lw1 from the observation target S irradiated with the broadband light and the second light flux Lf2 including the first fluorescence Lw2 from the observation target S irradiated with the first narrowband light to the second imaging element 522b. In addition, the optical element 15 sequentially guides the third light flux Lf3 including the second fluorescence Lw3 from the observation target S irradiated with the second narrowband light and the third light flux Lf3 including the third fluorescence Lw4 from the observation target S irradiated with the third narrowband light to the third imaging element 522c.

As a result, the first imaging element 522a sequentially receives the first light flux Lf1 including the broadband reflected light Lw1 and the first light flux Lf1 including the first fluorescence Lw2. Then, the first imaging element 522a sequentially and repeatedly outputs the image signal based on the broadband reflected light Lw1 and the image signal based on the first fluorescence Lw2 under the control of the control apparatus 90 (control section 94).

In addition, the second imaging element 522b sequentially receives the second light flux Lf2 including the broadband reflected light Lw1 and the second light flux Lf2 including the first fluorescence Lw2. Then, the second imaging element 522b sequentially and repeatedly outputs the image signal based on the broadband reflected light Lw1 and the image signal based on the first fluorescence Lw2 under the control of the control apparatus 90 (control section 94).

In addition, the third imaging element 522c sequentially receives the third light flux Lf3 including the second fluorescence Lw3 and the third light flux Lf3 including the third fluorescence Lw4. Then, the third imaging element 522c sequentially and repeatedly outputs the image signal based on the second fluorescence Lw3 and the image signal based on the third fluorescence Lw4 under the control of the control apparatus 90 (control section 94).

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the first fluorescence Lw2 from the image signal based on the first fluorescence Lw2 output from the second imaging element 522b. The "high-resolution color image" mentioned here is a color image acquired by the high-resolution imaging element, and the "high-sensitivity monochrome image" is a monochrome image acquired by the high-sensitivity imaging element.

In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the third imaging element 522c. In addition, under the control of the control section 94, the image generation section 93 (image processing section 931) generates a captured image (high-sensitivity monochrome image) of the observation target S based on the third fluorescence Lw4 from the image signal based on the third fluorescence Lw4 output from the third imaging element 522c. The "high-sensitivity monochrome image" mentioned here is a monochrome image acquired by the high-sensitivity imaging element.

As described above, in this mode, in the common time frame, the normal light captured image which is the reflected image of the visible light (white light) and the first to third fluorescence captured images in which the first to third substances in the observation target S are emphasized, that is, the fluorescence is emitted from the first to third substances in the observation target S excited by the narrowband light are acquired. Therefore, the user can compare and observe the normal light captured image, the first fluorescence captured image, the second fluorescence captured image, and the third fluorescence captured image of the observation target S or observe a composite image (superimposed image) formed from these images via the display apparatus 70.

Note that, in the this mode, the image signal of the first fluorescence image frame is also output from the first imaging element 522a, and the image signal of the broadband light image frame is also output from the second imaging element 522b. However, in the above-described example, these image frames are not used for generating the captured image. In the above example, the image data of these image frames not used to generate the captured image is also repeatedly output as the image signal from the imaging element (the first imaging element 522a and the second imaging element 522b), but the image data of the image frames not used to generate the captured image may not be output as the image signal from the imaging element.

In addition, in the above example, these image frames (that is, the first fluorescence image frame from the first imaging element 522a and the broadband light image frame from the second imaging element 522b) that are not used to generate the captured image may be used to generate the captured image.

That is, the captured image based on the first fluorescence Lw2 may be generated on the basis of the image signal of the first fluorescence image frame from the first imaging element 522a. For example, the captured image based on the first fluorescence Lw2 may be generated on the basis of the image signal of the first fluorescence image frame from both the first imaging element 522a and the second imaging element 522b.

In addition, the captured image based on the broadband reflected light Lw1 may be generated on the basis of the image signal of the broadband light image frame from the second imaging element 522b. That is, the captured image based on the broadband reflected light Lw1 may be generated on the basis of the image signal of the broadband light image frame from both the first imaging element 522a and the second imaging element 522b.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### [Ninth Embodiment]

In the present embodiment, the same or corresponding elements as those in the first to eighth embodiments described above are denoted by the same reference signs, and a detailed description thereof will be omitted.

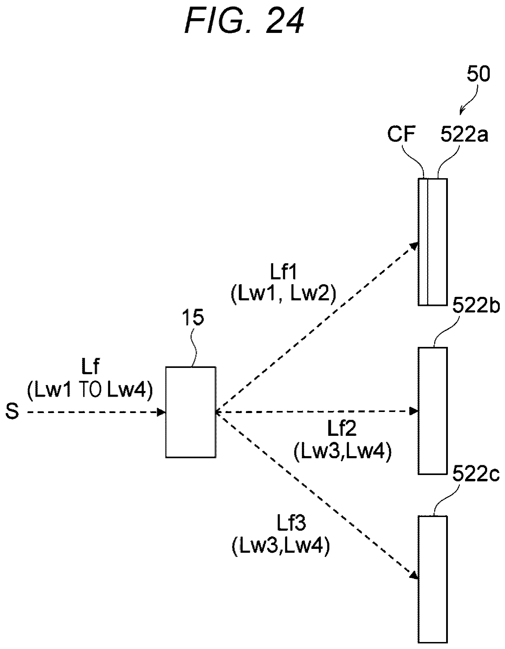

Fig. 24 is a diagram for explaining types of light incident on the imaging element (first imaging element 522a, second imaging element 522b, and third imaging element 522c) according to the ninth embodiment.

The camera head 50 (in particular, the imaging section 52) of the present embodiment includes a three-plate type imaging module (see Figs. 5 and 6), and includes a first imaging element 522a having a color filter CF, and a second imaging element 522b and a third imaging element 522c not having the color filter CF.

The color filter CF provided in the first imaging element 522a of the present embodiment transmits the broadband reflected light Lw1 and the first fluorescence Lw2 received by the first imaging element 522a as described later, but may or may not transmit light (for example, the second fluorescence Lw3 and the third fluorescence Lw4) in a wavelength band different from the broadband reflected light Lw1 and the first fluorescence Lw2. Note that the second imaging element 522b and the third imaging element 522c of the present example do not include the color filter CF, but each of the second imaging element 522b and the third imaging element 522c may include the color filter CF capable of transmitting the second fluorescence Lw3 and the third fluorescence Lw4.

The first imaging element 522a has relatively lower sensitivity and higher resolution (for example, 4K resolution) than the second imaging element 522b and the third imaging element 522c, while the second imaging element 522b and the third imaging element 522c have relatively higher sensitivity and lower resolution (for example, HD resolution) than the first imaging element 522a. The second imaging element 522b and the third imaging element 522c may have the same characteristics or different characteristics from each other. However, the resolution and sensitivity of the first imaging element 522a to the third imaging element 522c are not limited thereto, and the relationship between the resolution and sensitivity of the first imaging element 522a to the third imaging element 522c is not limited thereto.

The light source apparatus 10 (see Fig. 1B) emits light from at least one of the broadband light source 11, the first narrowband light source 12, the second narrowband light source 13, or the third narrowband light source 14, and can irradiate the observation target S with at least one of broadband light, first narrowband light, second narrowband light, or third narrowband light. Therefore, the observation light Lf from the observation target S may include broadband reflected light Lw1 which is reflected light of broadband light, first fluorescence Lw2 emitted from the first substance excited by the first narrowband light, second fluorescence Lw3 emitted from the second substance excited by the second narrowband light, and third fluorescence Lw4 emitted from the third substance excited by the third narrowband light. Note that the broadband reflected light Lw1 and the first fluorescence Lw2 are light included in the first wavelength band, and the second fluorescence Lw3 and the third fluorescence Lw4 are light included in the second wavelength band outside the first wavelength band.

The observation light Lf incident on the optical element 15 is separated into the first light flux Lf1, the second light flux Lf2, and the third light flux Lf3 by the optical element 15. The optical element 15 of the present embodiment guides light included in the first wavelength band to the first imaging element 522a as a first light flux Lf1, guides light including second fluorescence Lw3 to the second imaging element 522b as a second light flux Lf2, and guides light including third fluorescence Lw4 to the third imaging element 522c as a third light flux Lf3.

As described above, in the present embodiment, the first light flux Lf1 in which the light in the wavelength bands of the second fluorescence Lw3 and the third fluorescence Lw4 in the observation light Lf is partially, substantially, or completely suppressed is guided to the first imaging element 522a. That is, light including the broadband reflected light Lw1 and the first fluorescence Lw2 is guided to the first imaging element 522a as the first light flux Lf1. Furthermore, in the observation light Lf, the second light flux Lf2 in which the light in the first wavelength band is partially, substantially, or completely suppressed and the light in the wavelength band of the third fluorescence Lw4 is partially, substantially, or completely suppressed is guided to the second imaging element 522b. That is, light including at least the second fluorescence Lw3 is guided to the second imaging element 522b as the second light flux Lf2. Furthermore, in the observation light Lf, the third light flux Lf3 in which the light in the first wavelength band is partially, substantially, or completely suppressed and the light in the wavelength band of the second fluorescence Lw3 is partially, substantially, or completely suppressed is guided to the third imaging element 522c. That is, light including at least the third fluorescence Lw4 is guided to the third imaging element 522c as the third light flux Lf3.

The medical observation system 100 of the present embodiment having the above-described configuration can acquire various captured images of the observation target S according to the following observation modes (first to sixth modes).

### <First Mode>

The first mode of the present embodiment is an observation mode of irradiating the observation target S with the broadband light and the first narrowband light to acquire a captured image based on the broadband reflected light Lw1 and the first fluorescence Lw2 from the observation target S.

That is, the control apparatus 90 (the control section 94 (see Fig. 7)) controls the light source apparatus 10 (the broadband light source 11 and the first narrowband light source 12 (see Fig. 1A)), the broadband light and the first narrowband light are emitted from the light source apparatus 10 in a time division manner, and the broadband light and the first narrowband light are emitted to the observation target S in a time division manner. Note that, in this mode, the second narrowband light source 13 and the third narrowband light source 14 are placed in the OFF state, and the second narrowband light and the third narrowband light are not emitted from the light source apparatus 10.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. As a result, the first imaging element 522a sequentially receives the first light flux Lf1 including the broadband reflected light Lw1 and the first light flux Lf1 including the first fluorescence Lw2.

Then, the first imaging element 522a sequentially and repeatedly outputs the image signal based on the broadband reflected light Lw1 and the image signal based on the first fluorescence Lw2 under the control of the control apparatus 90 (control section 94 (see Fig. 7)).

Then, under the control of the control section 94, the image generation section 93 (particularly, the image processing section 931 (see Fig. 7)) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-resolution color image) of the observation target S based on the first fluorescence Lw2 from the image signal based on the first fluorescence Lw2 output from the first imaging element 522a. The "high-resolution color image" mentioned here is a color image acquired by the high-resolution imaging element.

As described above, in this mode, in the common time frame, the normal light captured image which is the reflected image of the visible light (white light) and the first fluorescence captured image in which the first substance in the observation target S is emphasized, that is, the fluorescence is emitted from the first substance in the observation target S excited by the narrowband light are acquired. Therefore, the user can compare and observe the normal light captured image and the first fluorescence captured image of the observation target S and observe a composite image (superimposed image) formed from these images via the display apparatus 70 (see Fig. 1A).

### <Second Mode>

The second mode of the present embodiment is an observation mode of irradiating the observation target S with the broadband light and the second narrowband light to acquire a captured image based on the broadband reflected light Lw1 and the second fluorescence Lw3 from the observation target S.

That is, the control apparatus 90 (the control section 94) controls the light source apparatus 10 (the broadband light source 11 and the second narrowband light source 13), the broadband light and the second narrowband light are continuously emitted from the light source apparatus 10, and the observation target S is continuously irradiated with the broadband light and the second narrowband light. Note that, in this mode, the first narrowband light source 12 and the third narrowband light source 14 are placed in the OFF state, and the first narrowband light and the third narrowband light are not emitted from the light source apparatus 10.

Then, the optical element 15 continuously guides the first light flux Lf1 including the broadband reflected light Lw1 from the observation target S irradiated with the broadband light to the first imaging element 522a. In addition, the optical element 15 continuously guides the second light flux Lf2 including the second fluorescence Lw3 from the observation target S irradiated with the second narrowband light to the second imaging element 522b. In addition, the optical element 15 continuously guides the third light flux Lf3 including the second fluorescence Lw3 from the observation target S irradiated with the second narrowband light to the third imaging element 522c.

As a result, the first imaging element 522a continuously receives the first light flux Lf1 including the broadband reflected light Lw1. In addition, the second imaging element 522b continuously receives the second light flux Lf2 including the second fluorescence Lw3, and the third imaging element 522c continuously receives the third light flux Lf3 including the second fluorescence Lw3.

Then, under the control of the control apparatus 90 (control section 94), the first imaging element 522a continuously and repeatedly outputs an image signal based on the broadband reflected light Lw1, and the second imaging element 522b and the third imaging element 522c continuously and repeatedly output an image signal based on the second fluorescence Lw3.

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the second imaging element 522b. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the second fluorescence captured image in which the second substance in the observation target S is emphasized, that is, the fluorescence is emitted from the second substance in the observation target S excited by the narrowband light are acquired. Therefore, the user can compare and observe the normal light captured image and the second fluorescence captured image of the observation target S and observe a composite image (superimposed image) formed from these images via the display apparatus 70.

Note that, in the above example, the broadband light and the second narrowband light are constantly emitted from the light source apparatus 10, but the light source apparatus 10 may turn off the emission of each of the broadband light and the second narrowband light in the middle under the control of the control apparatus 90 (control section 94). However, by constantly emitting the broadband light and the second narrowband light by the light source apparatus 10, a bright captured image can be acquired, an increase in noise due to gain adjustment can be suppressed, and a substantial decrease in frame rate can be prevented.

Note that, in this mode, the image signal of the second fluorescence image frame is also output from the third imaging element 522c, but in the above-described example, the second fluorescence image frame from the third imaging element 522c is not used for generating the captured image. In the above example, the image data of the second fluorescence image frame not used to generate the captured image is repeatedly output as the image signal from the third imaging element 522c, but the image data of the second fluorescence image frame not used to generate the captured image may not be output as the image signal from the third imaging element 522c.

Furthermore, in the above-described example, the captured image based on the second fluorescence Lw3 may be generated on the basis of the image signal of the second fluorescence image frame from the third imaging element 522c that is not used for generating the captured image. For example, the captured image based on the second fluorescence Lw3 may be generated on the basis of the image signal of the second fluorescence image frame from both the second imaging element 522b and the third imaging element 522c.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Third Mode>

The third mode of the present embodiment is an observation mode of irradiating the observation target S with the broadband light, the first narrowband light, and the second narrowband light to acquire a captured image based on the broadband reflected light Lw1, the first fluorescence Lw2, and the second fluorescence Lw3 from the observation target S.

That is, the control apparatus 90 (control section 94) controls the light source apparatus 10 (broadband light source 11, first narrowband light source 12, and second narrowband light source 13), the broadband light and the first narrowband light are emitted from the light source apparatus 10 in a time division manner, and the second narrowband light is continuously emitted. As a result, the broadband light and the first narrowband light are emitted to the observation target S in a time division manner, and the second narrowband light is continuously emitted to the observation target S. Note that, in this mode, the third narrowband light source 14 is placed in the OFF state, and the third narrowband light is not emitted from the light source apparatus 10.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 continuously guides the second light flux Lf2 including the second fluorescence Lw3 from the observation target S irradiated with the second narrowband light to the second imaging element 522b. In addition, the optical element 15 continuously guides the third light flux Lf3 including the second fluorescence Lw3 from the observation target S irradiated with the second narrowband light to the third imaging element 522c.

As a result, the first imaging element 522a sequentially receives the first light flux Lf1 including the broadband reflected light Lw1 and the first light flux Lf1 including the first fluorescence Lw2. In addition, the second imaging element 522b continuously receives the second light flux Lf2 including the second fluorescence Lw3. In addition, the third imaging element 522c continuously receives the third light flux Lf3 including the second fluorescence Lw3.

Then, the first imaging element 522a sequentially and repeatedly outputs the image signal based on the broadband reflected light Lw1 and the image signal based on the first fluorescence Lw2 under the control of the control apparatus 90 (control section 94). In addition, each of the second imaging element 522b and the third imaging element 522c continuously and repeatedly outputs an image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-resolution color image) of the observation target S based on the first fluorescence Lw2 from the image signal based on the first fluorescence Lw2 output from the first imaging element 522a. In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the second imaging element 522b. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light), the first fluorescence captured image which is the image in which the first substance in the observation target S is emphasized, that is, the fluorescence is emitted from the first substance in the observation target S excited by the narrowband light, and the second fluorescence captured image which is the image in which the second substance in the observation target S is emphasized, that is, the fluorescence is emitted from the second substance in the observation target S excited by the narrowband light are acquired. Therefore, the user can compare and observe the normal light captured image, the first fluorescence captured image, and the second fluorescence captured image of the observation target S or observe a composite image (superimposed image) formed from these images via the display apparatus 70.

Note that, in this mode, the image signal of the second fluorescence image frame is also output from the third imaging element 522c, but in the above-described example, the second fluorescence image frame from the third imaging element 522c is not used for generating the captured image. In the above example, the image data of the second fluorescence image frame not used to generate the captured image is repeatedly output as the image signal from the third imaging element 522c, but the image data of the second fluorescence image frame not used to generate the captured image may not be output as the image signal from the third imaging element 522c.

Furthermore, in the above-described example, the captured image based on the second fluorescence Lw3 may be generated on the basis of the image signal of the second fluorescence image frame from the third imaging element 522c that is not used for generating the captured image. For example, the captured image based on the second fluorescence Lw3 may be generated on the basis of the image signal of the second fluorescence image frame from both the second imaging element 522b and the third imaging element 522c.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Fourth Mode>

The fourth mode of the present embodiment is an observation mode of irradiating the observation target S with the broadband light and the third narrowband light to acquire a captured image based on the broadband reflected light Lw1 and the third fluorescence Lw4 from the observation target S.

That is, the control apparatus 90 (control section 94) controls the light source apparatus 10 (the broadband light source 11 and the third narrowband light source 14), the broadband light and the third narrowband light are continuously emitted from the light source apparatus 10, and the broadband light and the third narrowband light are continuously emitted to the observation target S. Note that, in this mode, the first narrowband light source 12 and the second narrowband light source 13 are placed in the OFF state, and the first narrowband light and the second narrowband light are not emitted from the light source apparatus 10.

Then, the optical element 15 continuously guides the first light flux Lf1 including the broadband reflected light Lw1 from the observation target S irradiated with the broadband light to the first imaging element 522a. In addition, the optical element 15 continuously guides the second light flux Lf2 including the third fluorescence Lw4 from the observation target S irradiated with the third narrowband light to the second imaging element 522b. In addition, the optical element 15 continuously guides the third light flux Lf3 including the third fluorescence Lw4 from the observation target S irradiated with the third narrowband light to the third imaging element 522c.

As a result, the first imaging element 522a continuously receives the first light flux Lf1 including the broadband reflected light Lw1, and continuously and repeatedly outputs an image signal based on the broadband reflected light Lw1 under the control of the control apparatus 90 (control section 94). Furthermore, the second imaging element 522b continuously receives the second light flux Lf2 including the third fluorescence Lw4, and continuously and repeatedly outputs an image signal based on the third fluorescence Lw4 under the control of the control apparatus 90 (control section 94). Furthermore, the third imaging element 522c continuously receives the third light flux Lf3 including the third fluorescence Lw4, and continuously and repeatedly outputs an image signal based on the third fluorescence Lw4 under the control of the control apparatus 90 (control section 94).

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the third fluorescence Lw4 from the image signal based on the third fluorescence Lw4 output from the third imaging element 522c. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the third fluorescence captured image in which the third substance in the observation target S is emphasized, that is, the third substance in the observation target S excited by the narrowband light emits fluorescence are acquired. Therefore, the user can compare and observe the normal light captured image and the third fluorescence captured image of the observation target S and observe a composite image (superimposed image) formed from these images via the display apparatus 70.

Note that, in this mode, the image signal of the third fluorescence image frame is also output from the second imaging element 522b, but in the above-described example, the third fluorescence image frame from the second imaging element 522b is not used for generating the captured image. In the above example, the image data of the third fluorescence image frame not used to generate the captured image is repeatedly output as the image signal from the second imaging element 522b, but the image data of the third fluorescence image frame not used to generate the captured image may not be output as the image signal from the second imaging element 522b.

Furthermore, in the above-described example, the captured image based on the third fluorescence Lw4 may be generated on the basis of the image signal of the third fluorescence image frame from the second imaging element 522b that is not used for generating the captured image. For example, the captured image based on the third fluorescence Lw4 may be generated on the basis of the image signal of the third fluorescence image frame from both the second imaging element 522b and the third imaging element 522c.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Fifth Mode>

The fifth mode of the present embodiment is an observation mode of irradiating the observation target S with the broadband light, the second narrowband light, and the third narrowband light to acquire a captured image based on the broadband reflected light Lw1, the second fluorescence Lw3, and the third fluorescence Lw4 from the observation target S.

That is, the control apparatus 90 (control section 94) controls the light source apparatus 10 (broadband light source 11, second narrowband light source 13, and third narrowband light source 14), the broadband light is continuously emitted from the light source apparatus 10, and the second narrowband light and the third narrowband light are emitted in a time division manner. As a result, the broadband light is continuously emitted to the observation target S, and the second narrowband light and the third narrowband light are emitted to the observation target S in a time division manner. Note that, in this mode, the first narrowband light source 12 is placed in the OFF state, and the first narrowband light is not emitted from the light source apparatus 10.

Then, the optical element 15 continuously guides the first light flux Lf1 including the broadband reflected light Lw1 from the observation target S irradiated with the broadband light to the first imaging element 522a. In addition, the optical element 15 sequentially guides the second light flux Lf2 including the second fluorescence Lw3 from the observation target S irradiated with the second narrowband light and the second light flux Lf2 including the third fluorescence Lw4 from the observation target S irradiated with the third narrowband light to the second imaging element 522b. In addition, the optical element 15 guides the third light flux Lf3 including the second fluorescence Lw3 from the observation target S irradiated with the second narrowband light and the third light flux Lf3 including the third fluorescence Lw4 from the observation target S irradiated with the third narrowband light to the third imaging element 522c.

As a result, the first imaging element 522a continuously receives the first light flux Lf1 including the broadband reflected light Lw1. In addition, the second imaging element 522b sequentially receives the second light flux Lf2 including the second fluorescence Lw3 and the second light flux Lf2 including the third fluorescence Lw4. In addition, the third imaging element 522c sequentially receives the third light flux Lf3 including the second fluorescence Lw3 and the third light flux Lf3 including the third fluorescence Lw4.

Then, the first imaging element 522a repeatedly outputs an image signal based on the broadband reflected light Lw1 under the control of the control apparatus 90 (control section 94). Furthermore, each of the second imaging element 522b and the third imaging element 522c alternately and repeatedly outputs an image signal based on the second fluorescence Lw3 and an image signal based on the third fluorescence Lw4 under the control of the control apparatus 90 (control section 94).

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the second imaging element 522b. In addition, under the control of the control section 94, the image generation section 93 (image processing section 931) generates a captured image (high-sensitivity monochrome image) of the observation target S based on the third fluorescence Lw4 from the image signal based on the third fluorescence Lw4 output from the third imaging element 522c. The "high-resolution color image" mentioned here is a color image acquired by the high-resolution imaging element, and the "high-sensitivity monochrome image" is a monochrome image acquired by the high-sensitivity imaging element.

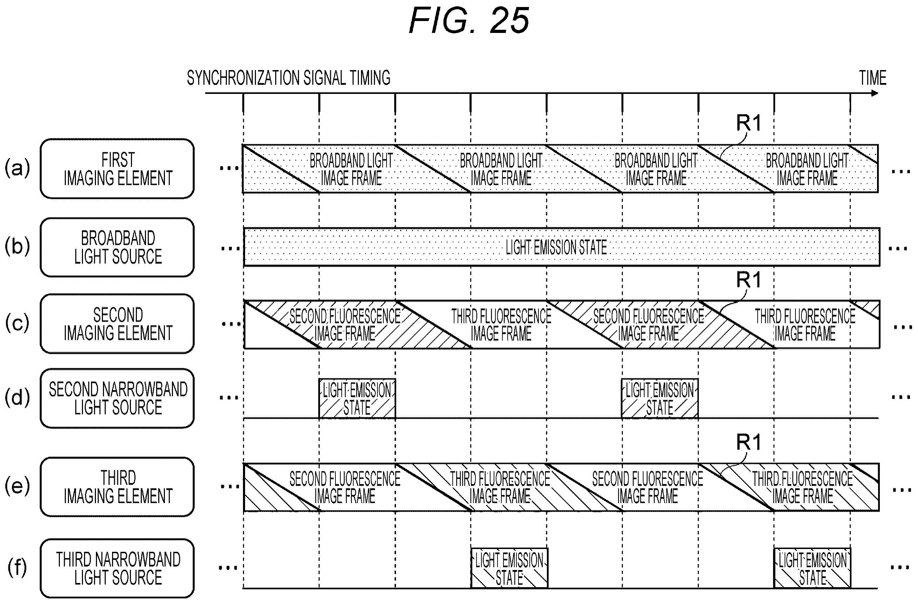

Fig. 25 illustrates an example of a timing chart of light source light emission and imaging element exposure in the fifth mode of the ninth embodiment.

(a) of Fig. 25 illustrates an exposure state of the first imaging element 522a, (c) illustrates an exposure state of the second imaging element 522b, and (e) illustrates an exposure state of the third imaging element 522c. In (a), (c), and (e) of Fig. 25, the vertical axis represents a horizontal line of the first imaging element 522a, the second imaging element 522b, and the third imaging element 522c, the horizontal axis represents time, and line R1 represents pixel data read start timing of each horizontal line regarding each image frame. The "broadband light image frame" between the lines R1 is an image frame for receiving (exposing) the broadband reflected light Lw1 from the observation target S. The "second fluorescence image frame" between the lines R1 is an image frame for receiving (exposing) the second fluorescence Lw3 from the observation target S. The "third fluorescence image frame" between the lines R1 is an image frame for receiving (exposing) the third fluorescence Lw4 from the observation target S. (b) of Fig. 25 illustrates the light emission timing of the broadband light in the broadband light source 11, (d) illustrates the light emission timing of the second narrowband light in the second narrowband light source 13, and (f) illustrates the light emission timing of the third narrowband light in the third narrowband light source 14.

In this mode, as described above, the light emission of the light source apparatus 10 related to the broadband light and the irradiation of the observation target S are continuously performed. On the other hand, light emission from the light source apparatus 10 and irradiation of the observation target S with respect to the second narrowband light and the third narrowband light are performed in a time division manner.

Therefore, exposure (light reception) in the first imaging element 522a regarding the broadband reflected light Lw1 from the observation target S is continuously performed.

On the other hand, the second imaging element 522b and the third imaging element 522c are sequentially exposed (received) to the second fluorescence Lw3 and the third fluorescence Lw4 from the observation target S. Therefore, control is performed by the control apparatus 90 (the control section 94) so that timings of time-division light emission of the second narrowband light and the third narrowband light from the light source apparatus 10 and timings of reading image data from the second imaging element 522b and the third imaging element 522c are associated with each other.

Specifically, on the basis of a common synchronization signal, the second narrowband light source 13 and the third narrowband light source 14 perform time-division light emission of the second narrowband light and the third narrowband light, and the second imaging element 522b and the third imaging element 522c perform exposure and reading of image data. More specifically, the second narrowband light and the third narrowband light are alternately emitted separately in time so that the second imaging element 522b and the third imaging element 522c are not simultaneously exposed by the second fluorescence Lw3 and the third fluorescence Lw4.

Then, image data is read such that an image signal of a second fluorescence image frame exposed with the second fluorescence Lw3 and an image signal of a third fluorescence image frame exposed with the third fluorescence Lw4 are output from the second imaging element 522b and the third imaging element 522c. As a result, each of the second imaging element 522b and the third imaging element 522c alternately and repeatedly outputs the image signal of the second fluorescence image frame and the image signal of the third fluorescence image frame.

On the other hand, reading of the image data (that is, the broadband light image frame) from the first imaging element 522a can be executed at an arbitrary timing while the broadband light is emitted by the light source apparatus 10. As a result, the first imaging element 522a continuously and repeatedly outputs the image signal of the broadband light image frame.

Then, the image generation section 93 (image processing section 931) generates a normal light captured image of the observation target S, which is a reflected image of broadband light (white light), from the image signal of the broadband light image frame output from the first imaging element 522a. In addition, the image generation section 93 (image processing section 931) generates a second fluorescence captured image, which is a captured image based on the second fluorescence Lw3 of the observation target S, from the image signal of the second fluorescence image frame from the second imaging element 522b. In addition, the image generation section 93 (image processing section 931) generates a third fluorescence captured image which is an image of the third fluorescence Lw4 of the observation target S from the image signal of the third fluorescence image frame from the third imaging element 522c.

As described above, in this mode, in the common time frame, the normal light captured image of the observation target S, the second fluorescence captured image in which the second substance in the observation target S is emphasized, that is, the second substance in the observation target S excited by the narrowband light emits fluorescence, and the third fluorescence captured image in which the third substance in the observation target S is emphasized, that is, the third substance in the observation target S excited by the narrowband light emits fluorescence are acquired. Therefore, the user can compare and observe the normal light captured image, the second fluorescence captured image, and the third fluorescence captured image of the observation target S or observe a composite image (superimposed image) formed from these images via the display apparatus 70.

Note that, in the example illustrated in Fig. 25, while image data is not read from the second imaging element 522b and the third imaging element 522c, light emission of the second narrowband light and the third narrowband light is started and terminated. Therefore, in the second fluorescence image frames of the second imaging element 522b and the third imaging element 522c, the third fluorescence Lw4 from the observation target S irradiated with the third narrowband light can be suppressed from entering the second imaging element 522b. Furthermore, in the third fluorescence image frames of the second imaging element 522b and the third imaging element 522c, the second fluorescence Lw3 from the observation target S irradiated with the second narrowband light can be suppressed from entering the second imaging element 522b and the third imaging element 522c.

Note that the start timing and the end timing of the light emission of the second narrowband light and the third narrowband light are not limited to the example illustrated in Fig. 25, and can be set to arbitrary timing. For example, while image data is being read from the second imaging element 522b and the third imaging element 522c (see "R1" in Fig. 25), light emission of one or both of the second narrowband light and the third narrowband light may be started or ended.

Note that, in the this mode, the image signal of the third fluorescence image frame is also output from the second imaging element 522b, and the image signal of the second fluorescence image frame is also output from the third imaging element 522c, but these image frames are not used to generate the captured image in the above-described example. In the above example, the image data of these image frames not used to generate the captured image is also repeatedly output as the image signal from the imaging element (the second imaging element 522b and the third imaging element 522c), but the image data of the image frames not used to generate the captured image may not be output as the image signal from the imaging element.

In addition, in the above example, these image frames (that is, the third fluorescence image frame from the second imaging element 522b and the second fluorescence image frame from the third imaging element 522c) that are not used to generate the captured image may be used to generate the captured image.

That is, the captured image based on the third fluorescence Lw4 may be generated on the basis of the image signal of the third fluorescence image frame from the second imaging element 522b. For example, the captured image based on the third fluorescence Lw4 may be generated on the basis of the image signal of the third fluorescence image frame from both the second imaging element 522b and the third imaging element 522c.

In addition, the captured image based on the second fluorescence Lw3 may be generated on the basis of the image signal of the second fluorescence image frame from the third imaging element 522c. For example, the captured image based on the second fluorescence Lw3 may be generated on the basis of the image signal of the second fluorescence image frame from both the second imaging element 522b and the third imaging element 522c.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### <Sixth Mode>

The sixth mode of the present embodiment is an observation mode of irradiating the observation target S with the broadband light and the first to third narrowband light to acquire a captured image based on the broadband reflected light Lw1 and the first fluorescence Lw2 to the third fluorescence Lw4 from the observation target S.

That is, the control apparatus 90 (control section 94) controls the light source apparatus 10 (the broadband light source 11 and the first to third narrowband light sources 12 to 14), and the broadband light and the first narrowband light are emitted from the light source apparatus 10 in a time division manner, and the second narrowband light and the third narrowband light are emitted in a time division manner. As a result, the broadband light and the first narrowband light are emitted to the observation target S in a time division manner, and the second narrowband light and the third narrowband light are emitted to the observation target S in a time division manner. Note that each of the broadband light and the first narrowband light may be simultaneously emitted with one of the second narrowband light and the third narrowband light, and simultaneously emitted to the observation target S.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the second fluorescence Lw3 from the observation target S irradiated with the second narrowband light and the second light flux Lf2 including the third fluorescence Lw4 from the observation target S irradiated with the third narrowband light to the second imaging element 522b. In addition, the optical element 15 sequentially guides the third light flux Lf3 including the second fluorescence Lw3 from the observation target S irradiated with the second narrowband light and the third light flux Lf3 including the third fluorescence Lw4 from the observation target S irradiated with the third narrowband light to the third imaging element 522c.

As a result, the first imaging element 522a sequentially receives the first light flux Lf1 including the broadband reflected light Lw1 and the first light flux Lf1 including the first fluorescence Lw2. Then, the first imaging element 522a sequentially and repeatedly outputs the image signal based on the broadband reflected light Lw1 and the image signal based on the first fluorescence Lw2 under the control of the control apparatus 90 (control section 94).

In addition, the second imaging element 522b sequentially receives the second light flux Lf2 including the second fluorescence Lw3 and the second light flux Lf2 including the third fluorescence Lw4. In addition, the third imaging element 522c sequentially receives the third light flux Lf3 including the second fluorescence Lw3 and the third light flux Lf3 including the third fluorescence Lw4. Then, each of the second imaging element 522b and the third imaging element 522c sequentially and repeatedly outputs an image signal based on the second fluorescence Lw3 and an image signal based on the third fluorescence Lw4 under the control of the control apparatus 90 (control section 94).

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-resolution color image) of the observation target S based on the first fluorescence Lw2 from the image signal based on the first fluorescence Lw2 output from the first imaging element 522a. In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the second imaging element 522b. In addition, under the control of the control section 94, the image generation section 93 (image processing section 931) generates a captured image (high-sensitivity monochrome image) of the observation target S based on the third fluorescence Lw4 from the image signal based on the third fluorescence Lw4 output from the third imaging element 522c. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the first to third fluorescence captured images in which the first to third substances in the observation target S are emphasized, that is, the fluorescence is emitted from the first to third substances in the observation target S excited by the narrowband light are acquired. Therefore, the user can compare and observe the normal light captured image, the first fluorescence captured image, the second fluorescence captured image, and the third fluorescence captured image of the observation target S or observe a composite image (superimposed image) formed from these images via the display apparatus 70.

Note that, in the this mode, the image signal of the third fluorescence image frame is also output from the second imaging element 522b, and the image signal of the second fluorescence image frame is also output from the third imaging element 522c, but these image frames are not used to generate the captured image in the above-described example. In the above example, the image data of these image frames not used to generate the captured image is also repeatedly output as the image signal from the imaging element (the second imaging element 522b and the third imaging element 522c), but the image data of the image frames not used to generate the captured image may not be output as the image signal from the imaging element.

In addition, in the above example, these image frames (that is, the third fluorescence image frame from the second imaging element 522b and the second fluorescence image frame from the third imaging element 522c) that are not used to generate the captured image may be used to generate the captured image.

That is, the captured image based on the third fluorescence Lw4 may be generated on the basis of the image signal of the third fluorescence image frame from the second imaging element 522b. For example, the captured image based on the third fluorescence Lw4 may be generated on the basis of the image signal of the third fluorescence image frame from both the second imaging element 522b and the third imaging element 522c.

In addition, the captured image based on the second fluorescence Lw3 may be generated on the basis of the image signal of the second fluorescence image frame from the third imaging element 522c. For example, the captured image based on the second fluorescence Lw3 may be generated on the basis of the image signal of the second fluorescence image frame from both the second imaging element 522b and the third imaging element 522c.

The captured image generated in this manner may be used to generate the output image or may not be used to generate the output image.

The captured image that is not used to generate the output image can be used for any purpose (for example, correction processing regarding brightness of the output image or processing regarding adjustment of a focal position).

### [10th Embodiment]

In the present embodiment, the same or corresponding elements as those in the first to ninth embodiments described above are denoted by the same reference signs, and a detailed description thereof will be omitted.

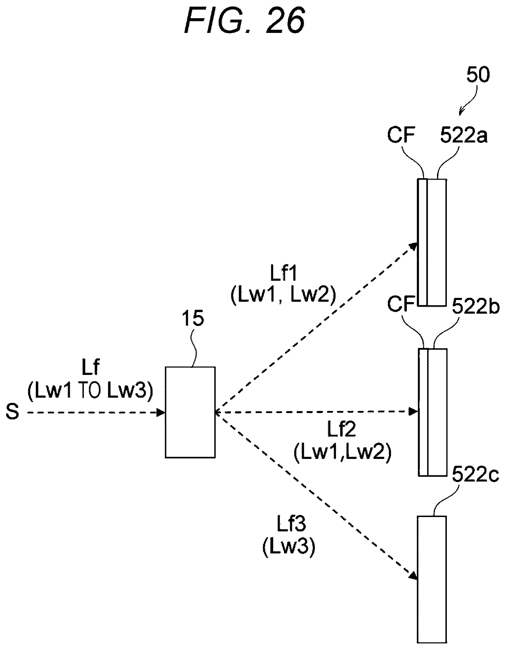

Fig. 26 is a diagram for explaining types of light incident on the imaging element (first imaging element 522a, second imaging element 522b, and third imaging element 522c) according to the 10th embodiment.

The camera head 50 (in particular, the imaging section 52) of the present embodiment includes a three-plate type imaging module (see Figs. 5 and 6), and includes a first imaging element 522a and a second imaging element 522b each having a color filter CF, and a third imaging element 522c not having the color filter CF.

The color filter CF provided in each of the first imaging element 522a and the second imaging element 522b of the present embodiment transmits the broadband reflected light Lw1 and the first fluorescence Lw2 received by each of the first imaging element 522a and the second imaging element 522b as described later, but may or may not transmit light (for example, the second fluorescence Lw3) in a wavelength band different from the broadband reflected light Lw1 and the first fluorescence Lw2. Note that the third imaging element 522c of the present example does not include the color filter CF, but the third imaging element 522c may include the color filter CF capable of transmitting the second fluorescence Lw3.

The first imaging element 522a has relatively lower sensitivity and higher resolution (for example, 4K resolution) than the second imaging element 522b and the third imaging element 522c, while the second imaging element 522b and the third imaging element 522c have relatively higher sensitivity and lower resolution (for example, HD resolution) than the first imaging element 522a. The second imaging element 522b and the third imaging element 522c may have the same characteristics or different characteristics from each other. However, the resolution and sensitivity of the first imaging element 522a to the third imaging element 522c are not limited thereto, and the relationship between the resolution and sensitivity of the first imaging element 522a to the third imaging element 522c is not limited thereto.

The light source apparatus 10 (see Fig. 1A) emits light from at least one of the broadband light source 11, the first narrowband light source 12, or the second narrowband light source 13, and can irradiate the observation target S with at least one of broadband light, first narrowband light, or second narrowband light.

Therefore, the observation light Lf from the observation target S may include broadband reflected light Lw1 which is reflected light of broadband light, first fluorescence Lw2 emitted from the first substance excited by the first narrowband light, and second fluorescence Lw3 emitted from the second substance excited by the second narrowband light. Note that the broadband reflected light Lw1 and the first fluorescence Lw2 are light included in the first wavelength band, and the second fluorescence Lw3 is light included in the second wavelength band outside the first wavelength band.

The observation light Lf incident on the optical element 15 is separated into the first light flux Lf1, the second light flux Lf2, and the third light flux Lf3 by the optical element 15. The optical element 15 of the present embodiment guides a part of light included in the first wavelength band to the first imaging element 522a as a first light flux Lf1, guides a part of light included in the first wavelength band to the second imaging element 522b as a second light flux Lf2, and guides light included in the second wavelength band to the third imaging element 522c as a third light flux Lf3.

As described above, in the present embodiment, the first light flux Lf1 in which the light in the first wavelength band is partially suppressed and the light in the wavelength band of the second fluorescence Lw3 is partially, substantially, or completely suppressed in the observation light Lf is guided to the first imaging element 522a by the optical element 15. That is, light including at least a part of the broadband reflected light Lw1 is guided to the first imaging element 522a as the first light flux Lf1. Furthermore, in the observation light Lf, the second light flux Lf2 in which the light in the first wavelength band is partially suppressed and the light in the wavelength band of the second fluorescence Lw3 is partially, substantially, or completely suppressed is guided to the second imaging element 522b. That is, light including at least a part of the broadband reflected light Lw1 and the first fluorescence Lw2 is guided to the second imaging element 522b as the second light flux Lf2. Furthermore, the third light flux Lf3 in which the light in the first wavelength band is partially, substantially, or completely suppressed in the observation light Lf is guided to the third imaging element 522c. That is, light including at least the second fluorescence Lw3 is guided to the third imaging element 522c as the third light flux Lf3.

The medical observation system 100 of the present embodiment having the above-described configuration can acquire various captured images of the observation target S according to the following observation modes (first to third modes).

### <First Mode>

The first mode of the present embodiment is an observation mode of irradiating the observation target S with the broadband light and the first narrowband light to acquire a captured image based on the broadband reflected light Lw1 and the first fluorescence Lw2 from the observation target S.

That is, the control apparatus 90 (the control section 94 (see Fig. 7)) controls the light source apparatus 10 (the broadband light source 11 and the first narrowband light source 12 (see Fig. 1A)), the broadband light and the first narrowband light are emitted from the light source apparatus 10 in a time division manner, and the broadband light and the first narrowband light are emitted to the observation target S in a time division manner. Note that, in this mode, the second narrowband light source 13 is placed in the OFF state, and the second narrowband light is not emitted from the light source apparatus 10.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. As a result, the first imaging element 522a sequentially receives the first light flux Lf1 including the broadband reflected light Lw1 and the first light flux Lf1 including the first fluorescence Lw2.

In addition, the optical element 15 sequentially guides the second light flux Lf2 including the broadband reflected light Lw1 from the observation target S irradiated with the broadband light and the second light flux Lf2 including the first fluorescence Lw2 from the observation target S irradiated with the first narrowband light to the second imaging element 522b. As a result, the second imaging element 522b sequentially receives the second light flux Lf2 including the broadband reflected light Lw1 and the second light flux Lf2 including the first fluorescence Lw2.

Then, each of the first imaging element 522a and the second imaging element 522b sequentially and repeatedly outputs the image signal based on the broadband reflected light Lw1 and the image signal based on the first fluorescence Lw2 under the control of the control apparatus 90 (control section 94 (see Fig. 7)).

Then, under the control of the control section 94, the image generation section 93 (particularly, the image processing section 931 (see Fig. 7)) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity color image) of the observation target S based on the first fluorescence Lw2 from the image signal based on the first fluorescence Lw2 output from the second imaging element 522b. The "high-resolution color image" mentioned here is a color image acquired by a high-resolution imaging element, and the "high-sensitivity color image" is a color image acquired by a high-sensitivity imaging element.

As described above, in this mode, in the common time frame, the normal light captured image which is the reflected image of the visible light (white light) and the first fluorescence captured image in which the first substance in the observation target S is emphasized, that is, the fluorescence is emitted from the first substance in the observation target S excited by the narrowband light are acquired. Therefore, the user can compare and observe the normal light captured image and the first fluorescence captured image of the observation target S and observe a composite image (superimposed image) formed from these images via the display apparatus 70 (see Fig. 1A).

Note that, although the image signal output from the first imaging element 522a is used to generate the captured image based on the broadband reflected light Lw1 in the above-described example, the image signal output from the second imaging element 522b may be used.

That is, the control section 94 may control the imaging section 52 (imaging element 522) and the image generation section 93 to generate an image based on the broadband reflected light Lw1 on the basis of one or both of the image signal output from the first imaging element 522a that has received the first light flux Lf1 including the broadband reflected light Lw1 and the image signal output from the second imaging element 522b that has received the second light flux Lf2 including the broadband reflected light Lw1.

For example, in a case where priority is given to resolution over sensitivity, a captured image (high-resolution color image) based on the broadband reflected light Lw1 may be generated on the basis of an image signal output from the first imaging element 522a. On the other hand, in a case where sensitivity is prioritized over resolution, a captured image (high-sensitivity color image) based on the broadband reflected light Lw1 may be generated on the basis of an image signal output from the second imaging element 522b. Furthermore, the captured image based on the broadband reflected light Lw1 may be generated on the basis of the image signal output from the first imaging element 522a and the image signal output from the second imaging element 522b. The "high-resolution color image" mentioned here is a color image acquired by a high-resolution imaging element, and the "high-sensitivity color image" is a color image acquired by a high-sensitivity imaging element.

The control section 94 may determine which one or both of the image signal output from the first imaging element 522a and the image signal output from the second imaging element 522b is used to generate the image based on the broadband reflected light Lw1 on the basis of the instruction from the user received via the input section 95.

Note that, although the image signal output from the second imaging element 522b is used to generate the captured image based on the first fluorescence Lw2 in the above-described example, the image signal output from the first imaging element 522a may be used. That is, the control section 94 may control the imaging section 52 (imaging element 522) and the image generation section 93 to generate an image based on the first fluorescence Lw2 on the basis of one or both of the image signal output from the first imaging element 522a that has received the first light flux Lf1 including the first fluorescence Lw2 and the image signal output from the second imaging element 522b that has received the second light flux Lf2 including the first fluorescence Lw2.

The control section 94 may determine which one or both of the image signal output from the first imaging element 522a and the image signal output from the second imaging element 522b is used for generation of the image based on the first fluorescence Lw2 on the basis of the instruction from the user received via the input section 95.

### <Second Mode>

The second mode of the present embodiment is an observation mode of irradiating the observation target S with the broadband light and the second narrowband light to acquire a captured image based on the broadband reflected light Lw1 and the second fluorescence Lw3 from the observation target S.

That is, the control apparatus 90 (the control section 94) controls the light source apparatus 10 (the broadband light source 11 and the second narrowband light source 13), the broadband light and the second narrowband light are continuously emitted from the light source apparatus 10, and the observation target S is continuously irradiated with the broadband light and the second narrowband light. Note that, in this mode, the first narrowband light source 12 is placed in the OFF state, and the first narrowband light is not emitted from the light source apparatus 10.

Then, the optical element 15 continuously guides the first light flux Lf1 including the broadband reflected light Lw1 from the observation target S irradiated with the broadband light to the first imaging element 522a, and continuously guides the second light flux Lf2 including the broadband reflected light Lw1 from the observation target S irradiated with the broadband light to the second imaging element 522b. In addition, the optical element 15 continuously guides the third light flux Lf3 including the second fluorescence Lw3 from the observation target S irradiated with the second narrowband light to the third imaging element 522c.

As a result, the first imaging element 522a continuously receives the first light flux Lf1 including the broadband reflected light Lw1, and the second imaging element 522b continuously receives the second light flux Lf2 including the broadband reflected light Lw1. In addition, the third imaging element 522c continuously receives the third light flux Lf3 including the second fluorescence Lw3.

Then, under the control of the control apparatus 90 (control section 94), the first imaging element 522a and the second imaging element 522b continuously repeatedly output an image signal based on the broadband reflected light Lw1, and the third imaging element 522c continuously repeatedly outputs an image signal based on the second fluorescence Lw3.

Then, under the control of the control section 94, the image generation section 93 (image processing section 931) generates a captured image (high-resolution color image) of the observation target S based on the broadband reflected light Lw1 from the image signal based on the broadband reflected light Lw1 output from the first imaging element 522a. In addition, under the control of the control section 94, the image generation section 93 (image processing section 931) generates a captured image (high-sensitivity monochrome image) of the observation target S based on the second fluorescence Lw3 from the image signal based on the second fluorescence Lw3 output from the third imaging element 522c. The "high-resolution color image" mentioned here is a color image acquired by the high-resolution imaging element, and the "high-sensitivity monochrome image" is a monochrome image acquired by the high-sensitivity imaging element.

As described above, in this mode, in the common time frame, the normal light captured image which is the reflected image of the visible light (white light) and the second fluorescence captured image in which the second substance in the observation target S is emphasized, that is, the fluorescence is emitted from the second substance in the observation target S excited by the narrowband light are acquired. Therefore, the user can compare and observe the normal light captured image and the second fluorescence captured image of the observation target S and observe a composite image (superimposed image) formed from these images via the display apparatus 70.

Note that, in the above example, the broadband light and the second narrowband light are constantly emitted from the light source apparatus 10, but the light source apparatus 10 may turn off the emission of each of the broadband light and the second narrowband light in the middle under the control of the control apparatus 90 (control section 94). For example, the light source apparatus 10 may repeatedly turn on and off the emission of the broadband light and the second narrowband light, and may emit the broadband light and the second narrowband light in a time division manner.

However, by constantly emitting the broadband light and the second narrowband light by the light source apparatus 10, a bright captured image can be acquired, an increase in noise due to gain adjustment can be suppressed, and a substantial decrease in frame rate can be prevented.

Note that, although the image signal output from the first imaging element 522a is used to generate the captured image based on the broadband reflected light Lw1 in the above-described example, the image signal output from the second imaging element 522b may be used. That is, the control section 94 may control the imaging section 52 (imaging element 522) and the image generation section 93 to generate an image based on the broadband reflected light Lw1 on the basis of one or both of the image signal output from the first imaging element 522a that has received the first light flux Lf1 including the broadband reflected light Lw1 and the image signal output from the second imaging element 522b that has received the second light flux Lf2 including the broadband reflected light Lw1.

The control section 94 may determine which one or both of the image signal output from the first imaging element 522a and the image signal output from the second imaging element 522b is used to generate the image based on the broadband reflected light Lw1 on the basis of the instruction from the user received via the input section 95.

### <Third Mode>

The third mode of the present embodiment is an observation mode of irradiating the observation target S with the broadband light, the first narrowband light, and the second narrowband light to acquire a captured image based on the broadband reflected light Lw1, the first fluorescence Lw2, and the second fluorescence Lw3 from the observation target S.

That is, the control apparatus 90 (control section 94) controls the light source apparatus 10 (broadband light source 11, first narrowband light source 12, and second narrowband light source 13), the broadband light and the first narrowband light are emitted from the light source apparatus 10 in a time division manner, and the second narrowband light is continuously emitted. As a result, the broadband light and the first narrowband light are emitted to the observation target S in a time division manner, and the second narrowband light is continuously emitted to the observation target S.

Then, the optical element 15 sequentially guides the first light flux Lf1 including the broadband reflected light Lw1 from the observation target S irradiated with the broadband light and the first light flux Lf1 including the first fluorescence Lw2 from the observation target S irradiated with the first narrowband light to the first imaging element 522a. In addition, the optical element 15 sequentially guides the second light flux Lf2 including the broadband reflected light Lw1 from the observation target S irradiated with the broadband light and the second light flux Lf2 including the first fluorescence Lw2 from the observation target S irradiated with the first narrowband light to the second imaging element 522b. In addition, the optical element 15 continuously guides the third light flux Lf3 including the second fluorescence Lw3 from the observation target S irradiated with the second narrowband light to the third imaging element 522c.

As a result, the first imaging element 522a sequentially receives the first light flux Lf1 including the broadband reflected light Lw1 and the first light flux Lf1 including the first fluorescence Lw2. In addition, the second imaging element 522b sequentially receives the second light flux Lf2 including the broadband reflected light Lw1 and the second light flux Lf2 including the first fluorescence Lw2. In addition, the third imaging element 522c continuously receives the third light flux Lf3 including the second fluorescence Lw3.

Then, the first imaging element 522a and the second imaging element 522b sequentially and repeatedly output the image signal based on the broadband reflected light Lw1 and the image signal based on the first fluorescence Lw2 under the control of the control apparatus 90 (control section 94). On the other hand, the third imaging element 522c continuously and repeatedly outputs an image signal based on the second fluorescence Lw3 under the control of the control apparatus 90 (control section 94).

Then, under the control of the control section 94, the image generation section 93 (image processing section 931) generates a captured image (high-resolution color image) of the observation target S based on the broadband reflected light Lw1 on the basis of the image signal output from the first imaging element 522a that has received the first light flux Lf1 including the broadband reflected light Lw1. In addition, under the control of the control section 94, the image generation section 93 (image processing section 931) generates a captured image (high-sensitivity color image) of the observation target S based on the first fluorescence Lw2 on the basis of the image signal output from the second imaging element 522b that has received the second light flux Lf2 including the first fluorescence Lw2. In addition, under the control of the control section 94, the image generation section 93 (image processing section 931) generates a captured image (high-sensitivity image) of the observation target S based on the second fluorescence Lw3 on the basis of the image signal output from the third imaging element 522c that has received the third light flux Lf3 including the second fluorescence Lw3. The "high-resolution color image" mentioned here is a color image acquired by a high-resolution imaging element, the "high-sensitivity color image" is a color image acquired by a high-sensitivity imaging element, and the "high-sensitivity image" is an image acquired by a high-sensitivity imaging element.

As described above, in this mode, in the common time frame, the normal light captured image which is the reflected image of the visible light (white light), and the first fluorescence captured image and the second fluorescence captured image which are images in which the first substance and the second substance in the observation target S are emphasized, that is, fluorescence is emitted from the first substance and the second substance in the observation target S excited by the narrowband light are acquired. Therefore, the user can compare and observe the normal light captured image, the first fluorescence captured image, and the second fluorescence captured image of the observation target S, and can observe a composite image (superimposed image) formed from these images.

Note that, although the image signal output from the first imaging element 522a is used to generate the captured image based on the broadband reflected light Lw1 in the above-described example, the image signal output from the second imaging element 522b may be used. That is, the control section 94 may control the imaging section 52 (imaging element 522) and the image generation section 93 to generate an image based on the broadband reflected light Lw1 on the basis of one or both of the image signal output from the first imaging element 522a that has received the first light flux Lf1 including the broadband reflected light Lw1 and the image signal output from the second imaging element 522b that has received the second light flux Lf2 including the broadband reflected light Lw1.

The control section 94 may determine which one or both of the image signal output from the first imaging element 522a and the image signal output from the second imaging element 522b is used to generate the image based on the broadband reflected light Lw1 on the basis of the instruction from the user received via the input section 95.

Note that, although the image signal output from the second imaging element 522b is used to generate the captured image based on the first fluorescence Lw2 in the above-described example, the image signal output from the first imaging element 522a may be used. That is, the control section 94 may control the imaging section 52 (imaging element 522) and the image generation section 93 to generate an image based on the first fluorescence Lw2 on the basis of one or both of the image signal output from the first imaging element 522a that has received the first light flux Lf1 including the first fluorescence Lw2 and the image signal output from the second imaging element 522b that has received the second light flux Lf2 including the first fluorescence Lw2.

The control section 94 may determine which one or both of the image signal output from the first imaging element 522a and the image signal output from the second imaging element 522b is used for generation of the image based on the first fluorescence Lw2 on the basis of the instruction from the user received via the input section 95.

### [Medical Observation Method]

Next, a medical observation method will be exemplified. The medical observation method described below can be performed by an arbitrary medical observation system 100 and a control apparatus 90 (image generation apparatus). Therefore, the medical observation system 100 and the control apparatus 90 based on each of the above-described embodiments and modifications may perform the medical observation method described below, or the medical observation system 100 and the control apparatus 90 different from each of the above-described embodiments and modifications may perform the medical observation method described below.

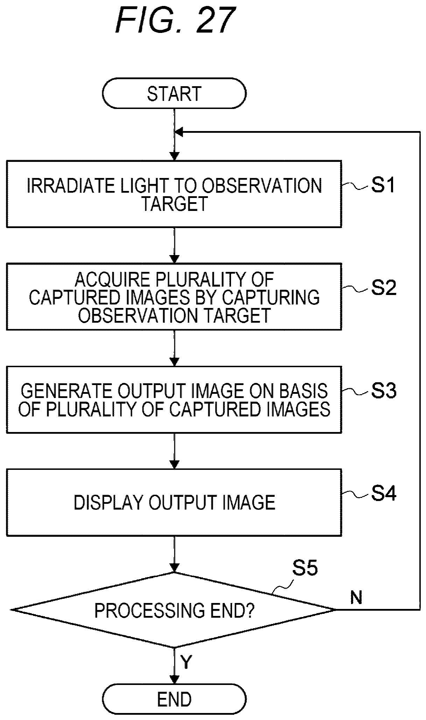

Fig. 27 is a flowchart illustrating an example of a medical observation method performed by the medical observation system 100.

First, under the control of the control section 94 of the control apparatus 90, while the observation target is irradiated with light emitted from the light source apparatus 10 and emitted from the insertion device 20 (S1 in Fig. 27), imaging of the observation target by the camera head 50 is performed, and a plurality of captured images is acquired (S2).

As described above, the light with which the observation target is irradiated may include visible light (for example, broadband light such as white light) and excitation light (narrowband light in a visible light wavelength band or narrowband light in an invisible light wavelength band). In addition, the plurality of captured images may include a captured image based on visible light reflected by the observation target and a captured image based on fluorescence from the observation target.

A specific manner of light emission and imaging is not limited. For example, light emission of a plurality of types of light in the light source apparatus 10 and light reception (exposure) of a plurality of types of light in the imaging element 522 of the camera head 50 may be performed intermittently, may be performed continuously, may be performed in a time division manner, or may be performed simultaneously.

Then, under the control of the control section 94, the image generation section 93 generates an output image on the basis of the plurality of captured images (S3), and the output image is displayed on the display apparatus 70 (S4). A user such as a doctor can confirm the state and characteristics of the tissue of the subject to be observed by viewing the output image displayed on the display apparatus 70.

The above-described series of processing (S1 to S4) is repeatedly continued while the end of the processing is not instructed from the user (N in S5), and is ended in a case where the end of the processing is instructed from the user (Y in S5). The control section 94 can receive such a processing end instruction from the user via the input section 95 (see Fig. 7).

Regarding the output image displayed on the display apparatus 70 as described above, for example, for a captured image (that is, a "normal light captured image") acquired by capturing an image of an observation target under irradiation of white light, high-resolution imaging may be required in order to provide a high-definition image. On the other hand, since the intensity of fluorescence from the observation target is not necessarily strong, high-sensitivity imaging may be required to acquire a captured image based on fluorescence. Although both high-resolution imaging and high-sensitivity imaging are satisfied by using a high-resolution and high-sensitivity imaging element, such a high-performance imaging element is expensive, and it may be difficult to prepare such a high-performance imaging element.

On the other hand, it is also possible to perform high-resolution imaging and high-sensitivity imaging by using a high-sensitivity imaging element and a high-resolution imaging element prepared as separate imaging elements. For example, observation light from a subject may be guided to a high-sensitivity imaging element or a high-resolution imaging element on the basis of a wavelength band. That is, desired high-resolution imaging and high-sensitivity imaging can be performed by guiding observation light in a certain wavelength band (for example, a light flux including reflected light of white light) to a high-resolution imaging element and guiding observation light in another wavelength band (for example, a light flux including fluorescence) to a high-sensitivity imaging element.

In a case where a plurality of imaging elements having different characteristics is used as described above, sensor sizes of the imaging elements may be different. In such a case, the range (angle of view) of the observation target captured in the captured image may be different among the plurality of imaging elements, and the number of pixels (size) of the captured image may be different among the imaging elements. In a case where such a captured image is displayed while being switched on one display apparatus, the user needs to perform visual field matching work between displayed images in the user's head. With such visual field matching between the display images, observing the observation target while matching or associating the position and range of the observation target (for example, the identification target such as a lesion) between the display images is troublesome work that puts a burden on the user, and accurate recognition of the identification target can be inhibited.

In addition, the plurality of captured images having different angles of view may include not only the region of the observation target included in common in the plurality of captured images but also the region of the observation target included in a certain captured image but not included in other captured images. For example, the observation target region included in the normal light captured image may not be included in the fluorescence captured image. In the superimposed image generated by superimposing and combining the normal light captured image and the fluorescence captured image, an observation target region included only in the normal light captured image is represented as the normal light captured image, but is not represented as the fluorescence captured image. That is, the region of the observation target included only in the normal light captured image can be recognized as the normal light captured image by the user such as a doctor in the superimposed image. On the other hand, since the observation target region included only in the normal light captured image is not included in the fluorescence captured image, even if the region contains the specific substance and emits fluorescence, the fluorescence captured image of the region is not included in the superimposed image. Therefore, even if the region of the observation target included only in the normal light captured image emits fluorescence, the user viewing the superimposed image may erroneously recognize the region as a "tissue that does not emit fluorescence (that is, a tissue that does not contain a specific substance)" and erroneously diagnose the region.

As described above, in a case where a plurality of captured images having different angles of view is superimposed, not only an observation target region (common image region) commonly included in all the captured images but also an observation target region (non-common image region) included in a certain captured image but not included in other captured images can be included in the superimposed image. It is not necessarily easy for a user such as a doctor to accurately distinguish such common image regions and non-common image regions in the superimposed image.

Therefore, in the output image generated on the basis of the plurality of captured images, it is desirable to generate the output image so as to be advantageous for the user to accurately recognize the image of the region to be observed that is included in common in the plurality of captured images.

### [Image Generation Method]

Next, a specific example of an image generation method regarding generation of an output image will be described. The image generation method described below can be implemented in any medical observation system 100, control apparatus 90 (image generation apparatus), and medical observation method. Therefore, the image generation method described below may be performed in the medical observation system 100, the control apparatus 90, and the medical observation method based on each of the above-described embodiments and modifications, or the image generation method described below may be performed in the medical observation system 100, the control apparatus 90, and the medical observation method different from each of the above-described embodiments and modifications.

In the medical field, for example, an endoscope apparatus is used to observe a subject in order to support surgery. The endoscope apparatus can capture an image of a subject under irradiation with white light to obtain a captured image of the subject. On the other hand, fluorescence observation is effective for a subject tissue that is difficult to identify from a captured image using white light. For example, by irradiating a subject to which an agent capable of emitting fluorescence is administered with excitation light and observing fluorescence emitted from the subject, it is possible to visually grasp the characteristics and state of the subject tissue.

In particular, according to the superimposed image obtained by combining the white light captured image and the fluorescence captured image, various characteristics and states of the subject can be simultaneously visualized.

A user such as a doctor observes a subject via an image of the subject displayed on a display apparatus. In particular, in a case where a plurality of images (for example, a normal light captured image captured and acquired under white light irradiation and a fluorescence captured image captured and acquired under excitation light irradiation) of different types related to the subject is displayed on the display apparatus, it is required to devise how to illustrate the plurality of images so as not to lead to erroneous diagnosis when performing surgery.

That is, a technique advantageous for devising and displaying a plurality of images related to the observation target is useful for the user.

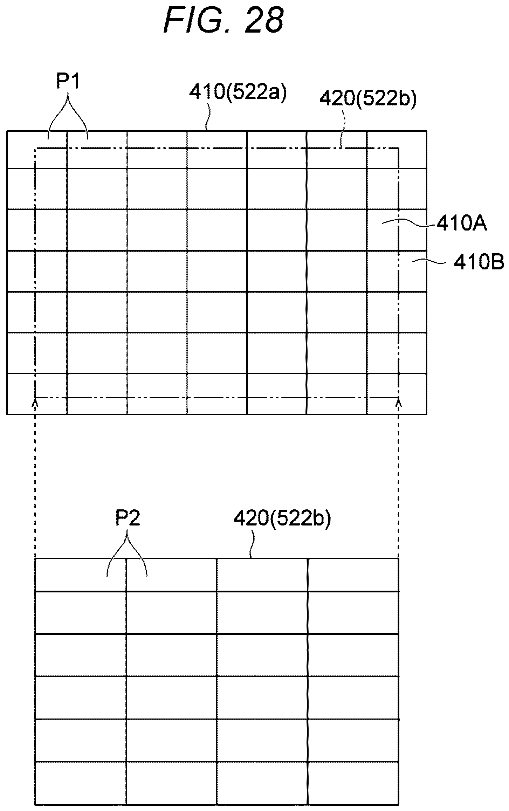

Fig. 28 illustrates an example of a physical size relationship between the imaging region 410 (effective pixel region) of the first imaging element 522a and the imaging region 420 (effective pixel region) of the second imaging element 522b. In Fig. 28, a rectangle superimposed on the imaging region 410 (solid line) of the first imaging element 522a and indicated by a two-dot chain line indicates a range corresponding to the imaging region 420 of the second imaging element 522b illustrated in Fig. 28.

A first image (captured image) is generated on the basis of an image signal (pixel data) output from the imaging region 410 of the first imaging element 522a, and a second image (captured image) is generated on the basis of an image signal output from the imaging region 420 of the second imaging element 522b.

In the example illustrated in Fig. 28, the first imaging element 522a and the second imaging element 522b are imaging elements of different types, and the imaging region 410 of the first imaging element 522a has a physically larger size than the imaging region 420 of the second imaging element 522b.

The angle of view of the first imaging element 522a is wider than the angle of view of the second imaging element 522b, and the range of the observation target imaged by the first imaging element 522a is wider than the range of the observation target imaged by the second imaging element 522b. That is, the entire range of the observation target imaged by the second imaging element 522b is included in the range of the observation target imaged by the first imaging element 522a. Therefore, the observation target included in the first image includes the entire range of the observation target included in the second image.

As described above, the first image is an image obtained by imaging the first range of the observation target using the first imaging element 522a. On the other hand, the second image is an image obtained by imaging a second range different from the first range of the observation target using the second imaging element 522b different in type from the first imaging element 522a. Here, one of the first range and the second range is wider than the other, and the second range is included in the first range in the example illustrated in Fig. 28. In addition, the "type" mentioned here is, for example, a physical size, an image size, or a model number of the imaging element, but is not limited thereto. Then, an output image is generated on the basis of the first image and the second image by an image generation method performed by the control apparatus 90 (image generation apparatus) as described later. The output image generated in this manner is, for example, an image obtained by performing processing of replacing or deleting one or more pixel signals among the pixel signals of the first image and the second image on the basis of the first range and the second range of the observation target.

Light from a site to be observed, which is included in both the first image and the second image, is incident on both the first imaging element 522a and the second imaging element 522b. On the other hand, light from a site to be observed, which is captured in the first image but not in the second image, is incident on the first imaging element 522a, but is not incident on the second imaging element 522b.

As described above, the imaging region 410 of the first imaging element 522a includes the common light receiving region 410A that receives light from the common site of the observation target and the non-common light receiving region 410B that receives light from the non-common target site of the observation target. On the other hand, the imaging region 420 of the second imaging element 522b includes only the common light receiving region 410A that receives light from the common site of the observation target.

As described above, the common light receiving region 410A of the first imaging element 522a is a region optically corresponding to the entire imaging region 420 of the second imaging element 522b, and the same site as the site of the observation target imaged by the second imaging element 522b is imaged in the common light receiving region 410A. On the other hand, the non-common light receiving region 410B of the first imaging element 522a is a region that does not optically correspond to the imaging region 420 of the second imaging element 522b, and a site of the observation target that is not imaged by the second imaging element 522b is imaged in the non-common light receiving region 410B.

As described above, the first imaging element 522a and the second imaging element 522b respectively acquire the first image and the second image in which the same observation target is captured and the ranges of the observation targets in which the same observation target is captured are different from each other.

In the example illustrated in Fig. 28, the number of pixels P1 of the first imaging element 522a (imaging region 410) is larger than the number of pixels P2 of the second imaging element 522b (imaging region 420). As an example, the number of pixels of the first imaging element 522a may be 3840×2160 corresponding to 4K resolution, and the number of pixels of the second imaging element 522b may be 1920×1080 corresponding to full high definition (HD).

However, the number of pixels of the first imaging element 522a and the number of pixels of the second imaging element 522b are not limited. The number of pixels of the imaging element is related not only to the physical size of the imaging region but also to the size of each pixel (that is, the pixel size). Therefore, the number of pixels of the first imaging element 522a may be the same as the number of pixels of the second imaging element 522b, or may be smaller than the number of pixels of the second imaging element 522b. As described above, the magnitude relationship of the number of pixels between the first imaging element 522a and the second imaging element 522b is not limited.

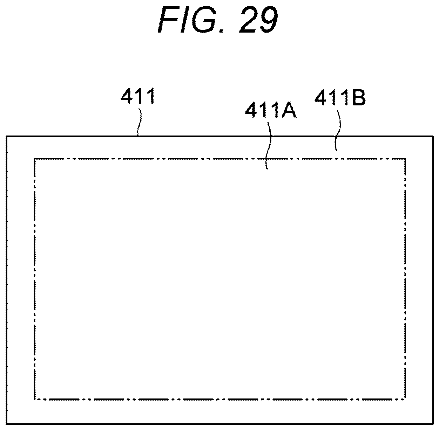

Fig. 29 is a schematic diagram illustrating an example of a first image 411 generated on the basis of an image signal from the first imaging element 522a illustrated in Fig. 28.

The first image 411 illustrated in Fig. 29 includes a common image region 411A and a non-common image region 411B adjacent to the common image region 411A. An image based on an image signal output from the common light receiving region 410A (see Fig. 28) of the first imaging element 522a is captured in the common image region 411A, and an image based on an image signal output from the non-common light receiving region 410B (see Fig. 28) is captured in the non-common image region 411B.

Therefore, in the common image region 411A, an image of a site to be observed, which is captured by both the first imaging element 522a and the second imaging element 522b and is included in the entire second image captured and acquired by the second imaging element 522b, is included. On the other hand, in the non-common image region 411B, an image of a site to be observed, which is not included in the second image, is included.

Note that, in the example illustrated in Fig. 29, the non-common image region 411B surrounds the entire common image region 411A (up, down, left, and right), but the range of the non-common image region 411B is not limited, and the non-common image region 411B does not necessarily surround the entire common image region 411A. In addition, it is not always necessary to specify specific ranges of the common image region 411A and the non-common image region 411B in the first image 411. Therefore, image generation section 93 (for example, superimposed image generation section 934) may or may not perform the processing of specifying the ranges of common image region 411A and non-common image region 411B in first image 411.

In each image generation example described below, a case where two imaging elements 522a and 522b illustrated in Fig. 28 are provided in the imaging section 52 of the camera head 50 will be described. That is, in each of the following image generation examples, an output image is generated from the captured image (that is, the first image and the second image) generated on the basis of the image signal output from each of the two imaging elements 522a and 522b, and the output image is displayed on the display apparatus 70.

Furthermore, in each of the following image generation examples, the first imaging element 522a acquires a captured image (normal light captured image; first image) by capturing an image of the observation target irradiated with visible light (particularly, white light; first light) in the first wavelength band from the broadband light source (first light source) 11. On the other hand, the second imaging element 522b acquires a captured image (fluorescence captured image; second image) by capturing an image of an observation target irradiated with excitation light that excites a specific substance so as to emit fluorescence, which is excitation light (second light) from the first narrowband light source (second light source) 12 and is visible fluorescence or invisible light. The excitation light is light in a wavelength band at least partially different from the first wavelength band. Therefore, the first image acquired by the first imaging element 522a includes a captured image based on reflected light of visible light (white light) from the observation target. On the other hand, the second image acquired by the second imaging element 522b includes a captured image based on fluorescence (that is, visible fluorescence having at least a partial wavelength different from the first wavelength band, or fluorescence in an invisible light wavelength band) from the observation target irradiated with the excitation light.

Note that the present disclosure technology is not limited to the following image generation examples, and can be appropriately applied to other image generation examples not illustrated below. For example, the imaging section 52 does not necessarily need to include the two imaging elements 522a and 522b illustrated in Fig. 28, and for example, even in a case where the imaging section 52 includes three or more imaging elements 522, the technology illustrated in each of the following image generation examples can be effectively applied. Furthermore, the wavelength characteristic of the light received by the imaging element 522 is not limited. For example, even in a case where two or more types of fluorescence emitted from the observation target irradiated with the excitation light and having different wavelength bands are received by one or a plurality of imaging elements 522, the technology illustrated in each of the following image generation examples can be effectively applied.

### [First Image Generation Example]

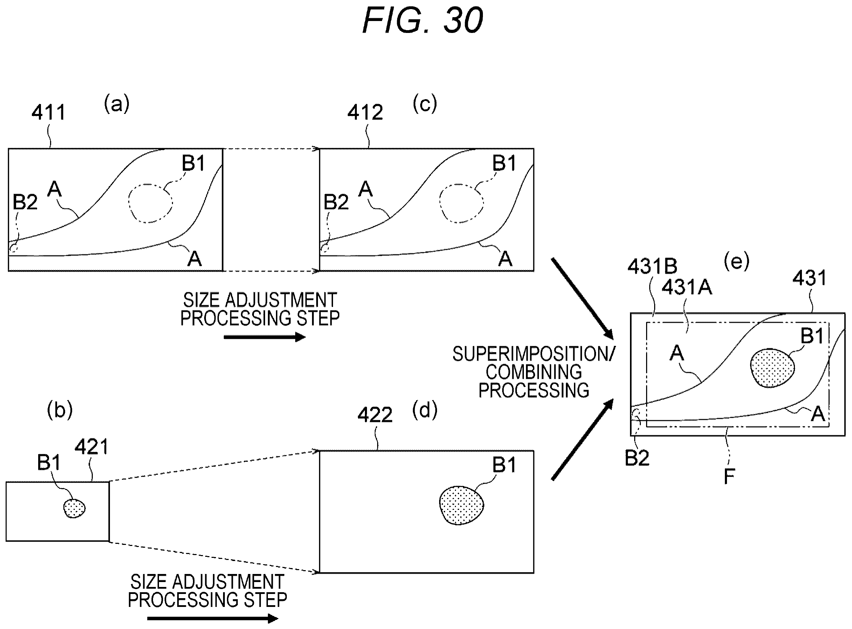

Fig. 30 is a diagram for explaining an example of generation processing of the first images 411 and 412, the second images 421 and 422, and the superimposed image 431 in the first image generation example.

(a) and (c) of Fig. 30 illustrate the first images 411 and 412 generated on the basis of the image signal from the first imaging element 522a, (a) illustrates the first image 411 before the size adjustment processing step, and (c) illustrates the first image 412 after the size adjustment processing step. (b) and (d) of Fig. 30 illustrate the second images 421 and 422 generated on the basis of the image signal from the second imaging element 522b, (b) illustrates the second image 421 before the size adjustment processing step, and (d) illustrates the second image 422 after the size adjustment processing step. (e) of Fig. 30 illustrates the superimposed image 431 generated on the basis of the first image 412 and the second image 422 after the size adjustment processing step. The processing of generating various images illustrated in Fig. 30 is appropriately performed by the image generation section 93 under the control of the control section 94 in the control apparatus 90 (see Fig. 7).

As an example, an organ having the fluorescent coloring sites B1 and B2 as identification targets is imaged by the first imaging element 522a and the second imaging element 522b as observation targets. Note that (a) and (c) of Fig. 30 illustrate the fluorescent coloring sites B1 and B2 as diagrams surrounded by a two-dot chain line, but the diagram is merely a reference diagram illustrating the corresponding ranges and positions of the fluorescent coloring sites B1 and B2. That is, the first images 411 and 412 in (a) and (c) of Fig. 30 do not include the fluorescent coloring sites B1 and B2 as identifiable images. Similarly, (e) of Fig. 30 illustrates the fluorescent coloring site B2 as a diagram surrounded by a two-dot chain line, but the diagram is merely a reference diagram illustrating the corresponding range and position of the fluorescent coloring site B2. That is, the superimposed image 431 in (e) of Fig. 30 does not include the fluorescent coloring site B2 as an identifiable image. However, as will be described later, the superimposed image 431 in (e) of Fig. 30 includes the fluorescent coloring site B1 as an identifiable image.

In the present image generation example, in the size adjustment processing step, captured images 412 and 422 (see (c) and (d) of Fig. 30) having desired sizes are generated from captured images 411 and 421 (see (a) and (b) of Fig. 30) based on the image signal output from the imaging element 522. Then, an output image is generated on the basis of the first image 412 and the second image 422 after the size adjustment processing step.

Specifically, the enlargement/reduction processing is performed on the first image 411 and the second image 421, and the sizes (the number of pixels) of both or one of the first image 411 and the second image 421 are adjusted, so that the first image 412 and the second image 422 after the size adjustment processing step are provided. In the enlargement processing, pixel interpolation is performed, the number of pixels constituting the image is increased, and the overall size of the image is increased. In the reduction processing, pixel thinning is performed, the number of pixels constituting an image is reduced, and the overall size of the image is reduced.

The enlargement/reduction magnification in the enlargement/reduction processing can be determined in consideration of generation of the superimposed image 431 to be described later. Specifically, the enlargement and reduction magnification is determined such that the size of the region (common image region) of the observation target (including the identification target) commonly captured in the first image 411 and the second image 421 is matched between the first image 411 and the second image 421. As a result, size adjustment of one or both of the first image 411 and the second image 421 is performed such that the number of pixels of the common image region matches between the first image 412 and the second image 422.

In the examples of (a) and (b) of Fig. 30, before the size adjustment processing step, the size (the number of pixels) of the second image 421 based on the image signal output from the second imaging element 522b is smaller than the size of the first image 411 based on the image signal output from the first imaging element 522a. Therefore, the enlargement magnification of the enlargement processing on the second image 421 is determined such that the entire second image 422 has the same size (number of pixels) as the common image region (see reference sign "411A" in Fig. 29) of the first image 412 after the size adjustment processing step.

Therefore, after the size adjustment processing step, the common image region of the first image 412 has the same angle of view as the entire second image 422, and the observation target is illustrated in the common image region of the first image 412 and the second image 422 in the same range and the same size.

Note that, in the example illustrated in Fig. 30, the first image 411 may be enlarged or reduced, or may not be enlarged or reduced in the size adjustment processing step. Therefore, the first images 411 and 412 (see (a) and (c) of Fig. 30) based on the image signal from the first imaging element 522a may have the same number of pixels before and after the size adjustment processing step. In this case, the enlargement/reduction processing on the first image 411 is unnecessary, and the size adjustment processing of the first image 411 is not substantially performed in the size adjustment processing step. Note that, also in this case, the first image before the size adjustment processing step is denoted by reference sign "411", and the first image after the size adjustment processing step is denoted by reference sign "412".

As described above, the range of the observation target appearing in the second images 421 and 422 is narrower than the range of the observation target appearing in the first images 411 and 412, and a region of the observation target (non-common image region 411B in Fig. 29) not appearing in the second images 421 and 422 is included in the first images 411 and 412. Therefore, the total number of pixels does not match between the first image 412 and the second image 422 after the size adjustment processing step, and the number of pixels of the second image 422 is smaller than the number of pixels of the first image 412.

Then, the superimposed image 431 is generated on the basis of the first image 412 and the second image 422 after the size adjustment processing step (see (e) of Fig. 30).

That is, the first image 412 and the second image 422 are superimposed and combined such that the size and position of the observation target region commonly included in the first image 412 and the second image 422 coincide with each other. As an example, the superimposition/combining processing of the first image 412 and the second image 422 may be performed such that the center of the second image 422 is aligned with the center of the common image region of the first image 412.

As described above, after the size adjustment processing step, the number of pixels of the common image region of the first image 412 coincides with the total number of pixels of the second image 422. As described above, since the observation target regions commonly captured between the first image 412 (particularly, the common image region) and the second image 422 correspond to each other in units of pixels, the superimposition/combining processing of the first image 412 and the second image 422 can be appropriately performed in units of pixels.

As described above, the superimposed image 431 is generated on the basis of the first image 412 and the second image 422 adjusted so that the image sizes (the number of pixels) of the observation target region (in particular, the fluorescent coloring site B1 as the identification target) coincide with each other, whereby the identification target is appropriately displayed in the superimposed image 431.

Note that, as described above, even after the size adjustment processing step, the overall size of the first image 412 is larger than the overall size of the second image 422 (see (c) and (d) of Fig. 30). Therefore, the superimposed image 431 includes a superimposed region 431A to which an image based on the first image 412 and the second image 422 is assigned, and a non-superimposed region 431B to which an image based on the first image 412 but not based on the second image 422 is assigned.

The superimposed composite image of the observation target region, which is commonly included in the first image 412 and the second image 422, is allocated to the superimposed region 431A of the superimposed image 431. On the other hand, an image of an observation target region (that is, a non-common image region of the first image 412 (see reference sign "411B" in Fig. 29)), which is included only in the first image 412 and is not included in the second image 422, is allocated to the non-superimposed region 431B adjacent to the superimposed region 431A. Note that, in the example illustrated in (e) of Fig. 30, the non-superimposed region 431B surrounds the entire superimposed region 431A, but the range of the non-superimposed region 431B is not limited, and the non-superimposed region 431B may not necessarily surround the entire superimposed region 431A.

As described above, the second image 422 is reflected in the image allocated to the superimposed region 431A of the superimposed image 431, but is not reflected in the image allocated to the non-superimposed region 431B.

In the example illustrated in Fig. 30, the reflected light of the white light with which the fluorescent coloring sites B1 and B2 are irradiated has wavelength characteristics similar to the reflected light of the white light with which the sites around the fluorescent coloring sites B1 and B2 are irradiated. Therefore, in the first images 411 and 412 which are normal light captured images, the organ A to be observed is included as an identifiable image, but the fluorescent coloring sites B1 and B2 are not included as an identifiable image. Therefore, it is difficult for the user to visually distinguish the fluorescent coloring sites B1 and B2 from the surrounding sites in the first images 411 and 412 (normal light captured image) acquired by the first imaging element 522a (see (a) of Fig. 30).

On the other hand, the fluorescent coloring sites B1 and B2 of the present example emit fluorescence that can be imaged by the second imaging element 522b by being irradiated with excitation light (second light) from the first narrowband light source (second light source) 12. Therefore, the organ A to be observed is not included as an identifiable image in the second image 421 (see (b) of Fig. 30) which is the fluorescence captured image. On the other hand, the second image 421 includes the fluorescent coloring site (in particular, the fluorescent coloring site B1 located in the region of the observation target, which is commonly included in the first image 412 and the second image 422) as an identifiable image. Therefore, the user can visually identify the fluorescent coloring site B1 from the surrounding site in the second image 421 (fluorescence captured image) acquired by the second imaging element 522b.

On the other hand, the fluorescent coloring site B2 existing in the region of the organ, which is illustrated as the normal light captured image in the non-superimposed region 431B, is not illustrated as the fluorescence captured image in the superimposed image 431. Therefore, the user viewing the superimposed image 431 may erroneously recognize that such a fluorescent coloring site does not exist even if the fluorescent coloring site exists in the region of the organ illustrated in the non-superimposed region 431B.

Further, a boundary between the superimposed region 431A and the non-superimposed region 431B in the superimposed image 431 is not necessarily visually clear. Note that, in (e) of Fig. 30, the boundary F between the superimposed region 431A and the non-superimposed region 431B is indicated by a two-dot chain line, but the boundary F is a virtual line added for convenience and is not a line explicitly displayed from the beginning in the superimposed image 431. Therefore, it is not always easy for the user to clearly identify the image range related to the second image 422 in the superimposed image 431, and there is a possibility that the user erroneously recognizes at least a partial range of the non-superimposed region 431B as the superimposed region 431A.

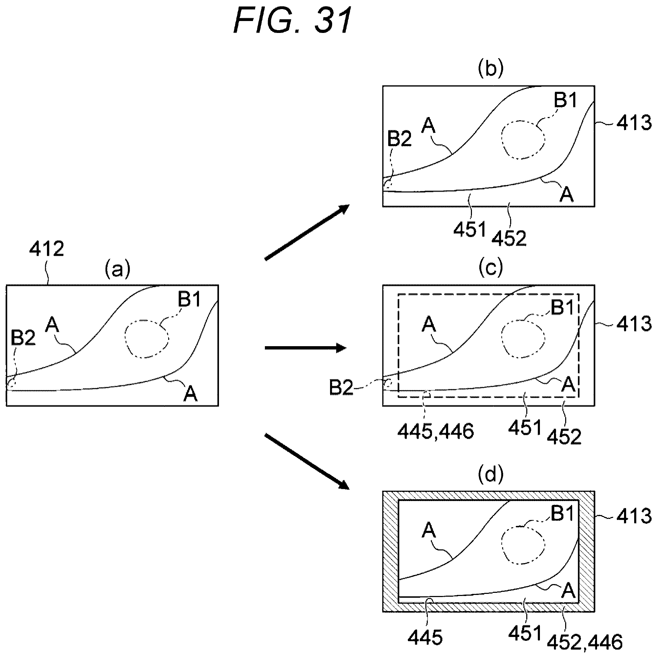

Figs. 31 to 33 are diagrams for explaining an example of output image generation processing in the first image generation example.

Fig. 31 illustrates an example of a first output image 413 generated mainly from a first image 412 (normal light captured image) which is a captured image by the first imaging element 522a. (a) of Fig. 31 illustrates the first image 412 that is the original image of the first output image 413 (see (b) to (d) of Fig. 31). The processing of generating various images illustrated in Fig. 31 is appropriately performed by the image generation section 93 under the control of the control section 94 in the control apparatus 90 (see Fig. 7).

In the example illustrated in Fig. 31, the first output image 413 based on the first image 412 is generated, and can be displayed on the display apparatus 70 simultaneously or switchably with other images.

The first output image 413 of the present image generation example includes a first image region 451 and a second image region 452 different from the first image region 451. In particular, the first image region 451 is generated on the basis of the first image 412 and the second image 422, and an image of a region to be observed, which is commonly included in the first image 412 and the second image 422, is allocated. Here, "the first image region 451 is generated on the basis of the first image 412 and the second image 422" can mean that the range and size of the image of the observation target region allocated to the first image region 451 are determined on the basis of the first image 412 and the second image 422.

For example, the first output image 413 based on the first image 412 may be the same image as the first image 412 (see (a) of Fig. 31) of the original image (see (b) of Fig. 31). In this case, the boundary between the first image region 451 and the second image region 452 is not indicated in the first output image 413, and the user can visually recognize the first output image 413 through the display apparatus 70 without being conscious of the boundary.

Alternatively, as illustrated in (c) and (d) of Fig. 31, the pixel replacement processing may be performed such that the boundary-enhanced image 446 indicating the boundary 445 of the first image region 451 is included in the first output image 413.

The pixel replacement processing described herein includes the entire processing of changing the value of one or a plurality of pixels. The pixel values before and after the pixel replacement processing may be values unrelated to each other, or the pixel value after the pixel replacement processing may be determined on the basis of the pixel value before the pixel replacement processing. Therefore, pixel combining processing in which pixel values after pixel replacement processing are derived on the basis of a plurality of pixel values before the pixel replacement processing is also included in the pixel replacement processing.

In the first output image 413 of the example illustrated in (c) of Fig. 31, as indicating the boundary 445 between the first image region 451 and the second image region 452, the boundary-enhanced image 446 having a line shape (for example, a dotted line shape) extends along the boundary 445. For example, the boundary-enhanced image 446 can be included in the first output image 413 by performing pixel replacement processing on a corresponding pixel of the first output image 413 such that the corresponding pixel exhibits a specific color indicating the boundary-enhanced image 446.

The boundary-enhanced image 446 extending along the boundary 445 of the first image region 451 as described above may be located only in the first image region 451, only in the second image region 452, or both in the first image region 451 and the second image region 452. Note that it is preferable to reduce the occupied region (for example, the line width) of the boundary-enhanced image 446 from the viewpoint of suppressing a reduction in the amount of information in the first output image 413.

On the other hand, in the example illustrated in (d) of Fig. 31, a unique image is allocated to the second image region 452 as the boundary-enhanced image 446, whereby the boundary 445 of the first image region 451 is indicated in the first output image 413.

The boundary-enhanced image 446 allocated to the second image region 452 is an image that can be visually distinguished and identified from the image (that is, the first image 412) allocated to the first image region 451, and can have an arbitrary color, pattern, and design. Therefore, the boundary-enhanced image 446 may be, for example, a mask image of a single color, an image of a hatched pattern, or an image of a checkerboard design. In a case where the switching display of the first output image 413 and the other image (for example, the superimposed image) is performed, since the size of the observation target does not change between the display images, the user such as a doctor can smoothly advance medical practice such as surgery.

In addition, in the second image region 452, an image based on the first image 412 (for example, an image of a non-common image region) may be illustrated together with the boundary-enhanced image 446. For example, an image (semitransparent image) of a non-common image region of the first image 412 combined with the boundary-enhanced image 446 by alpha blending may be allocated to the second image region 452 of the first output image 413.

As described above, in the first output image 413 generated on the basis of the normal light captured image, the organ A is included as an identifiable image, but the fluorescent coloring sites B1 and B2 are not included as an identifiable image. Therefore, the user can identify the organ A from the first output image 413, but it is difficult to identify the fluorescent coloring sites B1 and B2.

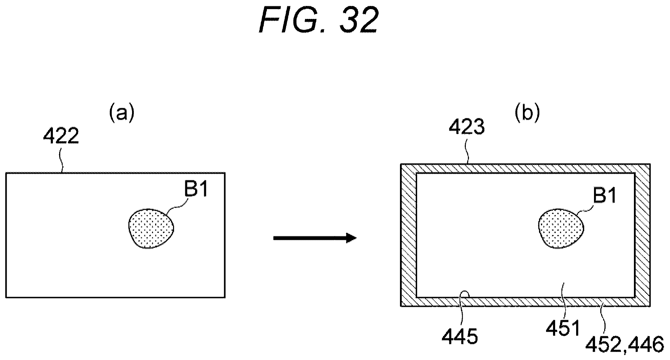

Fig. 32 illustrates an example of a second output image 423 generated mainly from a second image 422 (fluorescence captured image) which is a captured image by the second imaging element 522b. (a) of Fig. 32 illustrates the second image 422 that is the original image of the second output image 423 (see (b) of Fig. 32). The processing of generating various images illustrated in Fig. 32 is appropriately performed by the image generation section 93 under the control of the control section 94 in the control apparatus 90 (see Fig. 7).

In the example illustrated in Fig. 32, the second output image 423 based on the second image 422 is generated, and can be displayed on the display apparatus 70 simultaneously or switchably with other images.

As illustrated in (b) of Fig. 32, the second output image 423 based on the second image 422 may have the first image region 451 to which the second image 422 is assigned, and the second image region 452 to which a specific image that acts as the boundary-enhanced image 446 is assigned.

In this case, the boundary-enhanced image 446 allocated to the second image region 452 is an image that can be visually distinguished and identified from the image (that is, the second image 422) allocated to the first image region 451, and can have an arbitrary color, pattern, and design. For example, an image (for example, a black mask image) unrelated to first image 412 and second image 422 may be used as the boundary-enhanced image 446. In a case where the switching display of the second output image 423 and the other image (for example, the superimposed image) is performed, the size of the observation target does not change between the display images, so that the user such as a doctor can smoothly advance medical practice such as surgery.

An image processing method for generating such a second output image 423 is not limited. For example, the second output image 423 may be generated by performing pixel addition processing of adding the boundary-enhanced image 446 around the second image 422 by the image generation section 93.

In the second output image 423 generated on the basis of the second image 422 which is the fluorescence captured image, the organ A to be observed is not included as an identifiable image, but the fluorescent coloring site (the fluorescent coloring site B1 located in the first image region 451) is included as an identifiable image. Therefore, although it is difficult for the user to identify the organ A from the second output image 423, the user can identify the fluorescent coloring site B1.

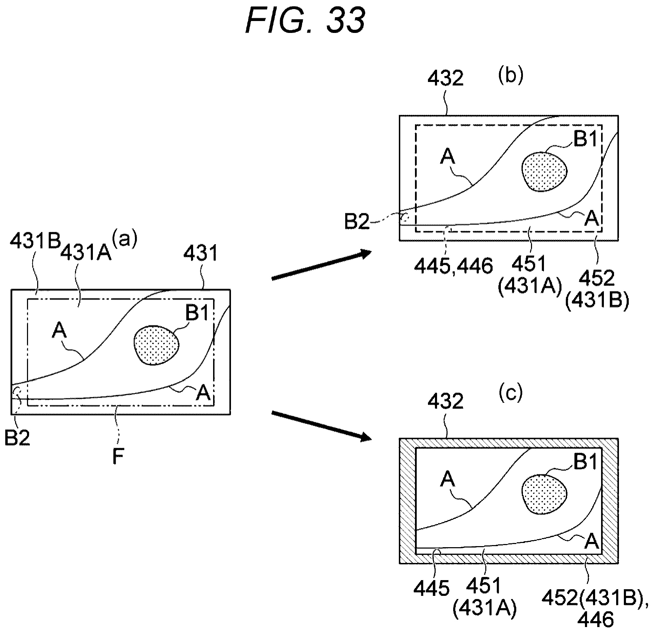

Fig. 33 illustrates an example of a superimposed output image 432 generated from a first image 412 (normal light captured image) and a second image 422 (fluorescence captured image) which are captured images by the first imaging element 522a and the second imaging element 522b. (a) of Fig. 33 illustrates the superimposed image 431 that is an original image of the superimposed output image 432 (see (b) and (d) of Fig. 33). The processing of generating various images illustrated in Fig. 33 is appropriately performed by the image generation section 93 under the control of the control section 94 in the control apparatus 90 (see Fig. 7).

In the example illustrated in Fig. 33, the superimposed output image 432 based on the superimposed image 431 is generated, and can be displayed on the display apparatus 70 simultaneously or switchably with other images.

As illustrated in (b) and (c) of Fig. 33, the superimposed output image 432 based on the superimposed image 431 may have a first image region 451 to which the superimposed image is assigned and a second image region 452 to which an image different from the first image region 451 is assigned. In particular, the pixel replacement processing may be performed such that the boundary-enhanced image 446 indicating the boundary 445 of the first image region 451 is included in the superimposed output image 432.

As described above, the superimposed image allocated to the first image region 451 of the superimposed output image 432 is generated on the basis of the image of the common image region in the first image 412 and the image of the common image region in the second image 422 having the same number of pixels.

In the example illustrated in (b) of Fig. 33, the boundary-enhanced image 446 having a line shape (for example, a dotted line shape) extends along the boundary 445 between the first image region 451 and the second image region 452. For example, the boundary-enhanced image 446 can be included in the superimposed output image 432 by performing pixel replacement processing on a corresponding pixel of the superimposed output image 432 so that the corresponding pixel exhibits a specific color indicating the boundary-enhanced image 446. The boundary-enhanced image 446 extending along the boundary 445 of the first image region 451 as described above may be located only in the first image region 451, only in the second image region 452, or both in the first image region 451 and the second image region 452.

On the other hand, in the example illustrated in (c) of Fig. 33, a unique image is allocated to the second image region 452 as the boundary-enhanced image 446, whereby the boundary 445 of the first image region 451 is indicated in the superimposed output image 432. The boundary-enhanced image 446 assigned to the second image region 452 is an image that can be visually distinguished and identified from the image assigned to the first image region 451 (that is, the superimposed image of the first image 412 and the second image 422), and may have any color, pattern, and design. In particular, using an image (for example, a black mask image) unrelated to the first image 412 and the second image 422 as the boundary-enhanced image 446 is advantageous in preventing erroneous recognition of the superimposed output image 432.

In addition, in the second image region 452, an image based on the first image 412 (for example, an image of the non-common image region 411B (see Fig. 29)) may be illustrated together with the boundary-enhanced image 446.

An image processing method for generating such a superimposed output image 432 is not limited. For example, it may be generated by performing pixel replacement processing of replacing data of a plurality of pixels of the superimposed image 431 (particularly, a plurality of pixels constituting the non-superimposed region 431B) with data of the boundary-enhanced image 446. Alternatively, the superimposed output image 432 may be generated by performing pixel replacement processing of combining the superimposed image 431 (particularly, the image of the non-superimposed region 431B) and the boundary-enhanced image 446.

For example, in a case where the above-described switching display of the second output image 423 (see Fig. 32) and the superimposed output image 432 (see Fig. 33) is performed in the display apparatus 70, the image generation section 93 of the control apparatus 90 outputs the second output image 423 and the superimposed output image 432 to the display apparatus 70. Here, the superimposed output image 432 is "an image obtained by performing processing of replacing or deleting one or more pixel signals among the pixel signals of the first image 412 and the second image 422 on the basis of a first range that is a range of an observation target imaged by the first imaging element 522a for acquiring the first images 411 and 412 and a second range that is a range of an observation target imaged by the second imaging element 522b for acquiring the second images 421 and 422". In addition, the second output image 423 is an image generated by adding one or more pixel signals (that is, pixel data (pixel value)) to an image generated from at least some pixel signals of the pixel signals of the second image 422.

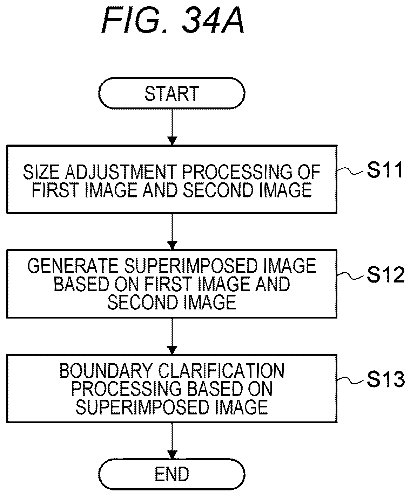

Figs. 34A to 34C are flowcharts illustrating a generation processing example of the superimposed output image 432.

Fig. 34A is a flowchart of a generation processing example corresponding to the examples illustrated in Figs. 30 to 33 described above. That is, after the execution of the size adjustment processing step for the first image 411 and the second image 421 (S11 in Fig. 34A); (a) to (d) of Fig. 30), the superimposed image 431 is generated on the basis of the first image 412 and the second image 422 subjected to the size adjustment processing (S12; (e) in Fig. 30). Then, the boundary-enhanced image 446 indicating the boundary of the superimposed region 431A (eventually, the boundary 445 of the first image region 451 of the superimposed output image 432) is applied to the superimposed image 431, thereby generating the superimposed output image 432 (S13; Fig. 33).

However, the superimposed output image 432 can also be generated by another processing flow.

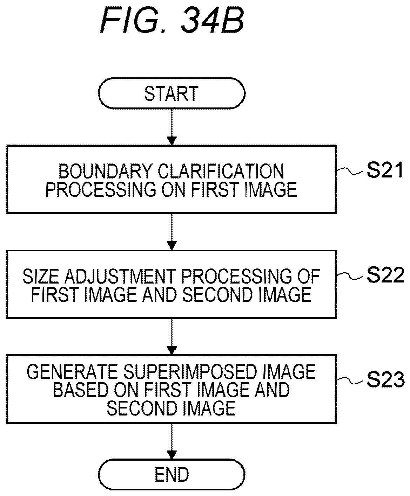

For example, as illustrated in Fig. 34B, after the boundary clarification processing for clarifying the boundary of the common image region 411A (see Fig. 29) is performed on the first image 411 (S21), the size adjustment processing step may be performed on the first image 411 and the second image 421 (S22). In this case, the superimposed image 431 (S23) generated on the basis of the first image 412 and the second image 422 after the size adjustment processing step can be used as the superimposed output image 432 (see Fig. 33).

In the present example, the "image that emphasizes the boundary between the common image region 411A and the non-common image region 411B" given to the first image 411 by the boundary clarification processing (S21) on the first image 411 finally configures the boundary-enhanced image 446 in the superimposed output image 432. The "image that emphasizes the boundary between the common image region 411A and the non-common image region 411B" mentioned here is not limited, and may be a linear image extending along the boundary between the common image region 411A and the non-common image region 411B. Alternatively, a specific image that can be visually distinguished and identified from the image allocated to the common image region 411A may be allocated to the non-common image region 411B as the "image that emphasizes the boundary between the common image region 411A and the non-common image region 411B".

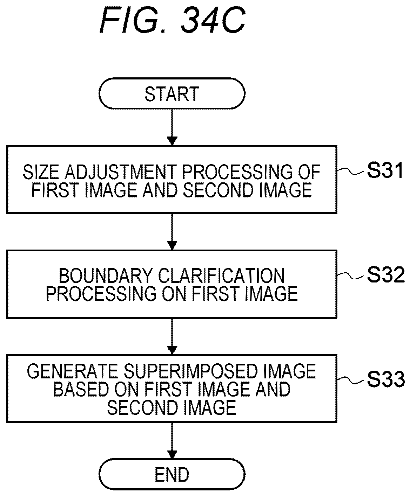

In addition, as illustrated in Fig. 34C, after the size adjustment processing step is performed on the first image 411 and the second image 421 (S31), the boundary clarification processing (S32) for clarifying the boundary of the common image region 411A may be performed on the first image 412. In this case, the superimposed image 431 (S33) generated on the basis of the first image 412 and the second image 422 after the boundary clarification processing can be used as the superimposed output image 432 (see Fig. 33).

Also in the present example, the "image that emphasizes the boundary between the common image region 411A and the non-common image region 411B" given to the first image 412 by the boundary clarification processing (S32) on the first image 412 finally configures the boundary-enhanced image 446 in the superimposed output image 432.

Note that, in the size adjustment processing (for example, enlargement processing) of the first image 411 and the second image 421, data of new pixels (interpolation pixels) may be generated on the basis of data of a plurality of pixels in the original image depending on a processing algorithm. In such a size adjustment processing, if the pixel data of the above-described "image that emphasizes the boundary" that is not originally included in the first image 411 and the second image 421 is used to create data of interpolation pixels, the boundary-enhanced image 446 in the superimposed output image 432 may become unclear. Therefore, from the viewpoint of realizing the clear boundary-enhanced image 446 in the superimposed output image 432, it is preferable to perform the boundary clarification processing after the size adjustment processing (for example, enlargement processing) of the first image 411 and the second image 421 (see Figs. 34A and 34C described above).

As described above, according to the present image generation example, in the first image region 451 of the output images 413, 423, and 432, an image based on both or one of the first images 411 and 412 and the second images 421 and 422, which is an image of a region to be observed and is commonly included in the first images 411 and 412 and the second images 421 and 422, is allocated. In the output images 413, 423, and 432, the boundary-enhanced image 446 indicating the boundary 445 of the first image region 451 can be included.

The user can clearly grasp the boundary 445 of the first image region 451 in the output images 413, 423, and 432 on the basis of the boundary-enhanced image 446 in the output images 413, 423, and 432. Therefore, in the output images 413, 423, and 432, the user can accurately recognize the image of the observation target region commonly included in the first images 411 and 412 and the second images 421 and 422.

In addition, the first output image 413, the second output image 423, and the superimposed output image 432 can be generated so as to have the same number of pixels and resolution as a whole. In particular, the first output image 413, the second output image 423, and the superimposed output image 432 are caused to coincide with each other in the number of pixels and the resolution of the first image region 451 to which the image of the observation target region is assigned, the first image region 451 being included in common in the first images 411 and 412 and the second images 421 and 422.

In this case, in the first image region 451 of each output image, the same range of the observation target is indicated by the same size. Therefore, even in a case where the plurality of output images 413, 423, and 432 is switched and displayed on the common display apparatus 70, the user can identify the observation target in each output image without feeling strange, and can easily visually compare the observation target between the output images.

Note that the image generation section 93 (display control section 935 (see Fig. 7)) of the control apparatus 90 may change the display angle of view of the output images 413, 423, and 432 in the display apparatus 70 on the basis of an instruction of digital zoom from the user via the input section 95. In particular, in a mode in which the boundary-enhanced image 446 is displayed in the second image region 452 and the second image region 452 is used as the mask region (see (d) of Fig. 31, (b) of Fig. 32, and (c) of Fig. 33), in a case where the display angle of view of the output images 413, 423, and 432 is changed, the output images 413, 423, and 432 may be generated such that only the image of the first image region 451 inside the mask region is displayed at the angle of view based on the user's instruction without changing the display size of the mask region (second image region 452) in the display apparatus 70.

Alternatively, in the display apparatus 70, the entire display sizes of the output images 413, 423, and 432 including the mask region (second image region 452) may be changed, and the output images 413, 423, and 432 may be generated such that the output images 413, 423, and 432 are displayed at the angle of view based on the user's instruction. In this case, it is also possible to adjust the display angle of view of the output images 413, 423, and 432 so that the first image region 451 is displayed on the entire display apparatus 70, leading to improvement of the visibility of the output images 413, 423, and 432.

### [Second Image Generation Example]

In the present image generation example, the same or corresponding elements as those in the above-described first image generation example are denoted by the same reference signs, and a detailed description thereof will be omitted.

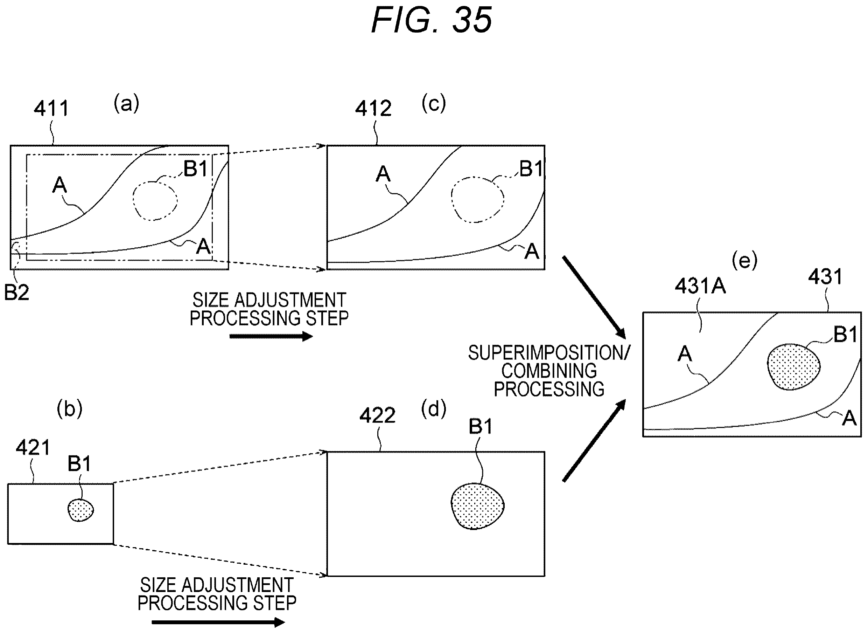

Fig. 35 is a diagram for explaining an example of generation processing of the first images 411 and 412, the second images 421 and 422, and the superimposed image 431 in the second image generation example.

(a) and (c) of Fig. 35 illustrate the first images 411 and 412 generated on the basis of the image signal from the first imaging element 522a, (a) illustrates the first image 411 before the size adjustment processing step, and (c) illustrates the first image 412 after the size adjustment processing step. (b) and (d) of Fig. 35 illustrate the second images 421 and 422 generated on the basis of the image signal from the second imaging element 522b, (b) illustrates the second image 421 before the size adjustment processing step, and (d) illustrates the second image 422 after the size adjustment processing step. (e) of Fig. 35 illustrates the superimposed image 431 generated on the basis of the first image 412 and the second image 422 after the size adjustment processing step. The processing of generating various images illustrated in Fig. 35 is appropriately performed by the image generation section 93 under the control of the control section 94 in the control apparatus 90 (see Fig. 7).

Similarly to the first image generation example described above, also in the present image generation example, as an example, an organ having the fluorescent coloring sites B1 and B2 as identification targets is imaged by the first imaging element 522a and the second imaging element 522b as observation targets. Note that (a) and (c) of Fig. 35 illustrate the fluorescent coloring sites B1 and B2 surrounded by a two-dot chain line, but the diagram is merely a reference diagram illustrating the corresponding ranges and positions of the fluorescent coloring sites B1 and B2. That is, the first images 411 and 412 in (a) and (c) of Fig. 35 do not include the fluorescent coloring sites B1 and B2 as identifiable images.

Also in the present image generation example, in the size adjustment processing step, the captured images 412 and 422 (see (c) and (d) of Fig. 35) having desired sizes are generated from the captured images 411 and 412 (see (a) and (b) of Fig. 35) based on the image signal output from the imaging element 522. Then, an output image is generated on the basis of the first image 412 and the second image 422 after the size adjustment processing step.

In particular, in the present image generation example, the image of the observation target region (common target region) (that is, the image of the common image region 411A (see Fig. 29)) commonly captured in the first image 411 and the second image 421 is extracted from the first image 411, and the size adjustment processing of the extracted portion is performed. Here, the processing of extracting the image of the common image region 411A corresponds to pixel deletion processing of deleting the pixels of the non-common image region 411B from the first image 411.

Thereafter, the enlargement/reduction processing is performed on both or one of the first image 411 (the image of the common image region 411A) and the second image 421 of the extracted portion, and the size (the number of pixels) of both or one of the first image 411 and the second image 421 is adjusted (size adjustment processing step). Also in the present image generation example, the enlargement and reduction magnification in the enlargement/reduction processing is determined to be a magnification at which the size of the common image region is matched between the first image 412 and the second image 422. Therefore, one or both of the first image 412 and the second image 422 are sized such that the number of pixels of the common image region matches between the first image 411 and the second image 421.

Then, an output image is generated on the basis of the "extracted first image 412 after the size adjustment processing step" and the "second image 422".

In the example illustrated in Fig. 35, before the size adjustment processing step, the overall size (the number of pixels) of the second image 421 is smaller than the size of the partial image based on the image signal output from the common image region 411A of the first imaging element 522a in the first image 411.

Then, in the size adjustment processing step, the extracted partial image of the first image 411 (the image of the common image region 411A) is enlarged to constitute the entire first image 412. On the other hand, the entire second image 421 is enlarged. The first image 412 and the second image 422 after the size adjustment processing include only the image of the observation target region (common image region) that is commonly captured, and have the same number of pixels and resolution.

Then, the superimposed image 431 is generated on the basis of the first image 412 and the second image 422 after the size adjustment processing step (see (e) of Fig. 35). That is, the first image 412 and the second image 422 are superimposed and combined such that the size and position of the observation target region commonly included in the first image 412 and the second image 422 coincide with each other.

As described above, in the first image 412 and the second image 422 after the size adjustment processing step, the same range of the observation target is captured with the same size (number of pixels) and has the same size and resolution as a whole. Therefore, the superimposed image 431 includes only the superimposed region 431A to which the images based on the first image 412 and the second image 422 are allocated, and does not include the non-superimposed region 431B (see (e) of Fig. 30).

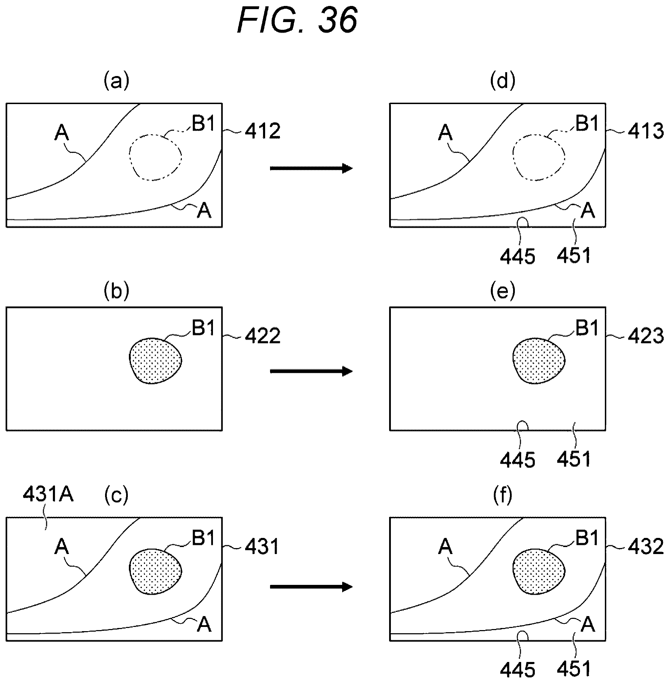

Fig. 36 is a diagram for explaining an example of output image generation processing in the second image generation example.

(a) of Fig. 36 illustrates the first image 412 that is the original image of the first output image 413 (see (d) of Fig. 36). (b) of Fig. 36 illustrates the second image 422 that is the original image of the second output image 423 (see (e) of Fig. 36). (c) of Fig. 36 illustrates the superimposed image 431 that is the original image of the superimposed output image 432 (see (f) of Fig. 36). The processing of generating various images illustrated in Fig. 36 is appropriately performed by the image generation section 93 under the control of the control section 94 in the control apparatus 90 (see Fig. 7).

Also in the present image generation example, the first output image 413 based on the first image 412, the second output image 423 based on the second image 422, and the superimposed output image 432 based on the superimposed image 431 are generated, and can be displayed on the display apparatus 70 simultaneously or switchably. In particular, as in the example illustrated in Fig. 36, the first output image 413, the second output image 423, and the superimposed output image 432 may be the same images as the first image 412, the second image 422, and the superimposed image 431 of the original image, respectively.

The output images 413, 423, and 432 illustrated in (d) to (f) of Fig. 36 include only the first image region 451 to which the image of the common image region is allocated, and do not include the second image region 452 different from the first image region 451. In this case, a contour portion of each of the output images 413, 423, and 432 is a portion indicating a boundary of the first image region 451 to which the image of the common image region is allocated. Therefore, in the output images 413, 423, and 432 of the present image generation example, an "additional image for indicating the boundary 445 of the first image region 451" such as the boundary-enhanced image 446 (see (c) and (d) of Fig. 31 and the like) in the above-described first image generation example is unnecessary.

As described above, according to the present image generation example, the output images 413, 423, and 432 (see (d) to (f) of Fig. 36) including only the first image region 451 can be generated. Since the entire output images 413, 423, and 432 are occupied by the first image region 451 in this manner, when the output images 413, 423, and 432 are displayed on the display apparatus 70, all the images in the first image region 451 can be displayed without waste in the display region of the display apparatus 70.

### [Modification]

In the first image generation example and the second image generation example described above, the output image is generated on the basis of the entire second image 421, but the output image may be generated on the basis of a partial range of the second image 421. For example, in a case where the observation target is included only in a partial region of the second image 421, the image generation section 93 may extract a partial range of the second image 421 including the region in which the observation target is included, and perform size adjustment processing on the image of the extraction range to generate the second image 421.

In addition, in general, surgery using a medical observation system may be performed while switching between several observation modes (for example, "normal light image observation mode based on normal light captured image" and "superimposed image observation mode based on superimposed image of normal light captured image and fluorescence captured image"). In such a case, if the appearance of the operative field image changes due to the change in the observation mode, it is difficult to smoothly perform the surgery. Therefore, in a case where the normal light image observation mode is switched to the superimposed image observation mode, the image generation section 93 may generate the superimposed image by superimposing the fluorescence captured image on the normal light captured image captured and acquired using the parameter of the normal light image observation mode under the control of the control section 94. In this case, it is possible to effectively suppress the change in the appearance of the operative field image when the normal light image observation mode is switched to the superimposed image observation mode, which is advantageous for maintaining the appearance of the operative field image and smoothly performing the surgery. When switching is performed between a plurality of observation modes in this manner (for example, when the normal light image observation mode is switched to the superimposed image observation mode or when the superimposed image observation mode is switched to the normal light image observation mode), various parameters used for image generation (image processing) may be maintained between the modes to be switched. In a case where the mode is switched from a certain observation mode to another observation mode under the control of the control section 94, the image generation section 93 may use at least some of the parameters used in the certain observation mode while maintaining the parameters in the other mode. Examples of the parameter here include image processing parameters such as white balance (WB), color tone, color mode, and color matrix adjustment of an image. Note that, when the normal light captured image in the superimposed image observation mode is acquired, the parameter of the normal light image observation mode may be used as it is, or a parameter set on the basis of the parameter of the normal light image observation mode may be used. The parameter used to acquire the fluorescence captured image in the superimposed image observation mode may be determined on the basis of the parameter related to the normal light captured image, or may be set independently of the parameter related to the normal light captured image.

Furthermore, in each of the above-described embodiments, the light source apparatus 10 is connected to the insertion device 20 configured as the endoscope main body, but the light source apparatus 10 may be connected to another arbitrary light emission device.

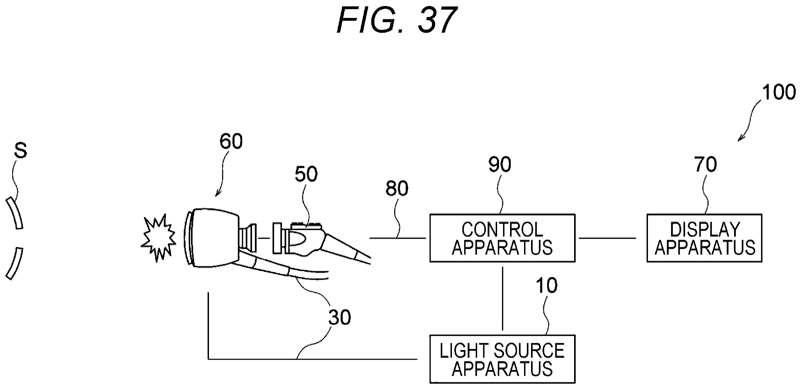

Fig. 37 is a conceptual diagram illustrating an example of a medical observation system 100 configured as an operative field illumination observation apparatus in which a ring light 60 (open field illumination apparatus for living body observation) is connected to a light source apparatus 10.

In the example illustrated in Fig. 37, the light source apparatus 10 and the camera head 50 are connected to the ring light 60. The light emitted by the light source apparatus 10 is sent to the ring light 60 via the light guide 30, and is emitted from the ring light 60 toward the observation target S. The ring light 60 is different from the insertion device 20 (see Figs. 1A and 1B) described above, which is intended to emit light inside the subject and irradiate the observation target with the light, in that the ring light is used to emit light outside the observation target.

For example, in each of the above-described embodiments, it is also possible to observe the observation target S according to the above-described observation mode by using the ring light 60 of the present example connected to the light source apparatus 10 instead of the insertion device 20.

Note that the ring light 60 illustrated in Fig. 37 is also connected to the camera head 50, and observation light from the observation target S enters the camera head 50 through the ring light 60. However, the light emission device such as the ring light 60 connected to the light source apparatus 10 may not be connected to the camera head 50, and may be provided separately from the camera head 50 (particularly, the imaging section).

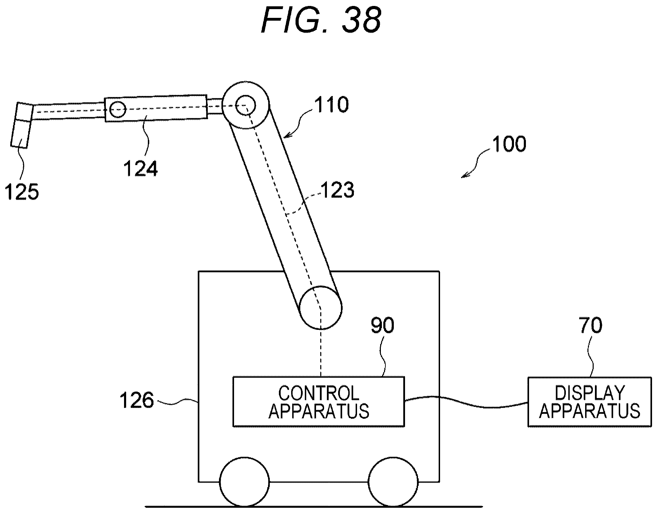

Fig. 38 is a diagram illustrating an example of the medical observation system 100 configured as a microscope system. The medical observation system 100 illustrated in Fig. 38 is a surgical microscope system having a function of enlarging and capturing a visual field region of an observation target and displaying an output image generated on the basis of a captured image.

The medical observation system 100 (surgical microscope system) of the present example includes a microscope apparatus 110 that captures an image of an observation target and the display apparatus 70 that displays an image captured by the microscope apparatus 110. The display apparatus 70 illustrated in Fig. 38 is provided separately from the microscope apparatus 110, but may be provided integrally with the microscope apparatus 110.

The microscope apparatus 110 includes a microscope portion 125 that enlarges and captures an image of a minute site to be observed, a support portion 124 having an arm that rotatably supports the microscope portion 125, and a base portion 126 that rotatably supports the support portion 124 and is movable on a floor surface. The base portion 126 includes the control apparatus 90 that controls the operation of the medical observation system 100. The control apparatus 90 is connected to the microscope portion 125 via the transmission cable 123. The base portion 126 may have a configuration fixed to a ceiling, a wall surface, or the like.

The microscope portion 125 includes the above-described imaging section (see Fig. 7; not illustrated in Fig. 38), an operation section (not illustrated) such as a switch that receives an input of an operation instruction of the microscope apparatus 110, and a cover glass (not illustrated) for protecting the inside. The user can move the microscope portion 125 while operating the operation section of the microscope portion 125.

In the present example, the captured image of the observation target is acquired by the microscope portion 125. Then, similarly to each of the above-described embodiments, the control apparatus 90 generates an output image from the captured image, and the output image is displayed on the display apparatus 70.

In the microscope apparatus 110 of the present example, a light emission device (not illustrated) that is connected to a light source apparatus (not illustrated) and emits light from the light source apparatus may be provided integrally with the microscope portion 125 (imaging section), or may be provided separately from the microscope portion 125. As an example, the light emission device may be provided on the base portion 126, may be provided on the support portion 124, or may be provided separately from the microscope apparatus 110.

It should be noted that the embodiments and modifications disclosed herein are merely illustrative in all respects and are not to be construed as limiting. The above-described embodiments and modifications can be omitted, replaced, and changed in various forms without departing from the scope and spirit of the appended claims. For example, the above-described embodiments and modifications may be combined in whole or in part, and embodiments other than the above-described embodiments and modifications may be combined with the above-described embodiments or modifications. Furthermore, the effects of the present disclosure described in the present specification are merely examples, and other effects may be provided.

The technical category embodying the above technical idea is not limited. For example, the above-described technical idea may be embodied by a computer program for causing a computer to execute one or a plurality of procedures (steps) included in a method of manufacturing or using the above-described apparatus. In addition, the above-described technical idea may be embodied by a computer-readable non-transitory recording medium in which such a computer program is recorded.

### [Supplementary Note]

The present disclosure can also have the following configurations.

### [Item 1]

A medical observation system including:
a light source apparatus that emits broadband light of a first wavelength band, first narrowband light that excites a first substance that emits first fluorescence of a wavelength band included in the first wavelength band, and second narrowband light that excites a second substance that emits second fluorescence of a wavelength band not included in the first wavelength band; and
a control section that controls the light source apparatus, in which
the control section is configured to:
   control the light source apparatus such that the broadband light and the first narrowband light are emitted to an observation target in a time division manner in a first mode; and
   control the light source apparatus such that the broadband light and the second narrowband light are emitted to the observation target in a second mode different from the first mode.

### [Item 2]

The medical observation system according to item 1, further including:
an imaging section including a first imaging element and a second imaging element; and
an optical element that separates light from the observation target into a plurality of light fluxes including a first light flux and a second light flux, guides the first light flux to the first imaging element, and guides the second light flux to the second imaging element.

### [Item 3]

The medical observation system according to item 2, in which
the second imaging element has higher sensitivity than the first imaging element.

### [Item 4]

The medical observation system according to item 2 or 3, in which
the first imaging element includes a color filter.

### [Item 5]

The medical observation system according to any one of items 2 to 4, in which
the second imaging element has no color filter.

### [Item 6]

The medical observation system according to any one of items 2 to 5, in which
light in a wavelength band of the second fluorescence is partially, substantially, or completely suppressed in the first light flux, and
light in the first wavelength band is partially, substantially, or completely suppressed in the second light flux.

### [Item 7]

The medical observation system according to item 6, in which
the optical element is configured to:
in the first mode, sequentially guide the first light flux including the reflected light from the observation target irradiated with the broadband light and the first light flux including the first fluorescence from the observation target irradiated with the first narrowband light to the first imaging element; and
in the second mode, guide the first light flux including the reflected light from the observation target irradiated with the broadband light to the first imaging element, and guide the second light flux including the second fluorescence from the observation target irradiated with the second narrowband light to the second imaging element.

### [Item 8]

The medical observation system according to item 7, in which
in a third mode different from the first mode and the second mode,
the control section controls the light source apparatus such that the broadband light and the first narrowband light are emitted to the observation target in a time division manner and the second narrowband light is emitted to the observation target, and
the optical element sequentially guides the first light flux including the reflected light from the observation target irradiated with the broadband light and the first light flux including the first fluorescence from the observation target irradiated with the first narrowband light to the first imaging element, and guides the second light flux including the second fluorescence from the observation target irradiated with the second narrowband light to the second imaging element.

### [Item 9]

The medical observation system according to item 7 or 8, in which
the light source apparatus emits third narrowband light that excites a third substance emitting third fluorescence in a wavelength band that is not included in the first wavelength band and is at least partially different from a wavelength band of the second fluorescence,
in a fourth mode different from the first mode and the second mode,
   the control section controls the light source apparatus such that the broadband light and the third narrowband light are emitted to the observation target, and
   the optical element guides the first light flux including the reflected light from the observation target irradiated with the broadband light to the first imaging element, and guides the second light flux including the third fluorescence from the observation target irradiated with the third narrowband light to the second imaging element.

### [Item 10]

The medical observation system according to any one of items 7 to 9, in which
the light source apparatus emits third narrowband light that excites a third substance emitting third fluorescence in a wavelength band that is not included in the first wavelength band and is at least partially different from a wavelength band of the second fluorescence,
in a fifth mode different from the first mode and the second mode,
   the control section controls the light source apparatus such that the broadband light and the first narrowband light are emitted to the observation target in a time division manner and the second narrowband light and the third narrowband light are emitted to the observation target in a time division manner, and
   the optical element sequentially guides the first light flux including the reflected light from the observation target irradiated with the broadband light and the first light flux including the first fluorescence from the observation target irradiated with the first narrowband light to the first imaging element, and sequentially guides the second light flux including the second fluorescence from the observation target irradiated with the second narrowband light and the second light flux including the third fluorescence from the observation target irradiated with the third narrowband light to the second imaging element.

### [Item 11]

The medical observation system according to any one of items 2 to 5, in which
light in the wavelength band of the second fluorescence is partially, substantially, or completely suppressed in the first light flux.

### [Item 12]

The medical observation system according to item 11, in which
the optical element is configured to:
guide the first light flux in which the light of the first wavelength band is partially suppressed to the first imaging element; and
guide the second light flux in which the light of the first wavelength band is partially suppressed to the second imaging element.

### [Item 13]

The medical observation system according to item 12, further including
an image generation section that generates an image on the basis of an image signal from the imaging section, in which
the control section controls the imaging section and the image generation section,
in the first mode,
   the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light and the second light flux including the first fluorescence from the observation target irradiated with the first narrowband light to the second imaging element,
   the control section controls the imaging section and the image generation section such that an image based on reflected light is generated on the basis of an image signal output from the first imaging element that has received the first light flux including the reflected light, and an image based on the first fluorescence is generated on the basis of an image signal output from the second imaging element that has received the second light flux including the first fluorescence,
in the second mode,
   the optical element guides the first light flux including the reflected light from the observation target irradiated with the broadband light to the first imaging element, and sequentially guides the second light flux including the reflected light from the observation target irradiated with the broadband light and the second light flux including the second fluorescence from the observation target irradiated with the second narrowband light to the second imaging element, and
   the control section controls the imaging section and the image generation section such that an image based on reflected light is generated on the basis of an image signal output from the first imaging element that has received the first light flux including the reflected light, and an image based on the second fluorescence is generated on the basis of an image signal output from the second imaging element that has received the second light flux including the second fluorescence.

### [Item 14]

The medical observation system according to item 12 or 13, further including
an image generation section that generates an image on the basis of an image signal from the imaging section, in which
the control section controls the imaging section and the image generation section,
in a third mode different from the first mode and the second mode,
   the control section controls the light source apparatus such that the broadband light, the first narrowband light, and the second narrowband light are emitted to the observation target in a time division manner,
   the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light, the second light flux including the first fluorescence from the observation target irradiated with the first narrowband light, and the second light flux including the second fluorescence from the observation target irradiated with the second narrowband light to the second imaging element, and
   the control section controls the imaging section and the image generation section such that an image based on reflected light is generated on the basis of an image signal output from the first imaging element that has received the first light flux including reflected light, an image based on the first fluorescence is generated on the basis of an image signal output from the second imaging element that has received the second light flux including the first fluorescence, and an image based on the second fluorescence is generated on the basis of an image signal output from the second imaging element that has received the second light flux including the second fluorescence.

### [Item 15]

The medical observation system according to item 11, in which
the optical element is configured to:
guide the first light flux in which the light in the wavelength band of the first fluorescence is partially, substantially, or completely suppressed to the first imaging element; and
guide the second light flux in which light in a wavelength band other than the wavelength band of the first fluorescence in the first wavelength band is partially, substantially, or completely suppressed to the second imaging element.

### [Item 16]

The medical observation system according to item 15, in which
the optical element is configured to:
in the first mode, guide the first light flux including the reflected light from the observation target irradiated with the broadband light to the first imaging element, and guide the second light flux including the first fluorescence from the observation target irradiated with the first narrowband light to the second imaging element; and
in the second mode, guide the first light flux including the reflected light from the observation target irradiated with the broadband light to the first imaging element, and guide the second light flux including the second fluorescence from the observation target irradiated with the second narrowband light to the second imaging element.

### [Item 17]

The medical observation system according to any one of items 16, in which
in a third mode different from the first mode and the second mode,
the control section controls the light source apparatus such that the broadband light is emitted to the observation target and the first narrowband light and the second narrowband light are emitted to the observation target in a time division manner, and
the optical element guides the first light flux including reflected light from the observation target irradiated with the broadband light to the first imaging element, and sequentially guides the second light flux including the first fluorescence from the observation target irradiated with the first narrowband light and the second light flux including the second fluorescence from the observation target irradiated with the second narrowband light to the second imaging element.

### [Item 18]

The medical observation system according to item 4, in which
the second imaging element includes a color filter.

### [Item 19]

The medical observation system according to item 18, in which
light in the wavelength band of the second fluorescence is partially, substantially, or completely suppressed in the first light flux.

### [Item 20]

The medical observation system according to item 19, in which
the optical element is configured to:
guide the first light flux in which the light of the first wavelength band is partially suppressed to the first imaging element; and
guide the second light flux in which the light of the first wavelength band is partially suppressed to the second imaging element.

### [Item 21]

The medical observation system according to any one of items 18 to 20, further including
an image generation section that generates an image on the basis of an image signal from the imaging section, in which
the control section controls the imaging section
and the image generation section,
in the first mode,
   the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light and the second light flux including the first fluorescence from the observation target irradiated with the first narrowband light to the second imaging element,
   the control section is configured to:
      control the imaging section and the image generation section such that an image based on reflected light is generated on the basis of one or both of an image signal output from the first imaging element that has received the first light flux including reflected light and an image signal output from the second imaging element that has received the second light flux including reflected light; and
      control the imaging section and the image generation section such that an image based on the first fluorescence is generated on the basis of an image signal output from the second imaging element that has received the second light flux including the first fluorescence,
in the second mode,
   the optical element guides the first light flux including the reflected light from the observation target irradiated with the broadband light to the first imaging element, and sequentially guides the second light flux including the reflected light from the observation target irradiated with the broadband light and the second light flux including the second fluorescence from the observation target irradiated with the second narrowband light to the second imaging element, and
   the control section controls the imaging section and the image generation section such that an image based on reflected light is generated on the basis of an image signal output from the first imaging element that has received the first light flux including the reflected light, and an image based on the second fluorescence is generated on the basis of an image signal output from the second imaging element that has received the second light flux including the second fluorescence.

### [Item 22]

The medical observation system according to any one of items 18 to 21, further including
an image generation section that generates an image on the basis of an image signal from the imaging section, in which
the control section controls the imaging section and the image generation section,
in a third mode different from the first mode and the second mode,
   the control section controls the light source apparatus such that the broadband light, the first narrowband light, and the second narrowband light are emitted to the observation target in a time division manner,
   the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light, the second light flux including the first fluorescence from the observation target irradiated with the first narrowband light, and the second light flux including the second fluorescence from the observation target irradiated with the second narrowband light to the second imaging element, and
   the control section controls the imaging section and the image generation section such that an image based on reflected light is generated on the basis of an image signal output from the first imaging element that has received the first light flux including reflected light, an image based on the first fluorescence is generated on the basis of an image signal output from the second imaging element that has received the second light flux including the first fluorescence, and an image based on the second fluorescence is generated on the basis of an image signal output from the second imaging element that has received the second light flux including the second fluorescence.

### [Item 23]

The medical observation system according to item 1, further including:
an imaging section including a first imaging element, a second imaging element, and a third imaging element; and
an optical element that separates light from the observation target into a plurality of light fluxes including a first light flux, a second light flux, and a third light flux, guides the first light flux to the first imaging element, guides the second light flux to the second imaging element, and guides the third light flux to the third imaging element.

### [Item 24]

The medical observation system according to item 23, in which
the second imaging element and the third imaging element have higher sensitivity than the first imaging element.

### [Item 25]

The medical observation system according to item 23 or 24, in which
the first imaging element includes a color filter.

### [Item 26]

The medical observation system according to any one of items 23 to 25, in which
the second imaging element and the third imaging element have no color filter.

### [Item 27]

The medical observation system according to any one of items 23 to 26, in which
light in the wavelength band of the second fluorescence is partially, substantially, or completely suppressed in the first light flux,
light in the wavelength band of the second fluorescence is partially, substantially, or completely suppressed in the second light flux, and
light in the first wavelength band is partially, substantially, or completely suppressed in the third light flux.

### [Item 28]

The medical observation system according to any one of items 23 to 27, in which
the optical element is configured to:
in the first mode, guide the first light flux including the reflected light from the observation target irradiated with the broadband light to the first imaging element, and guide the second light flux including the first fluorescence from the observation target irradiated with the first narrowband light to the second imaging element; and
in the second mode, guide the first light flux including the reflected light from the observation target irradiated with the broadband light to the first imaging element, and guide the third light flux including the second fluorescence from the observation target irradiated with the second narrowband light to the third imaging element.

### [Item 29]

The medical observation system according to item 28, in which
in a third mode different from the first mode and the second mode,
the control section controls the light source apparatus such that the broadband light and the first narrowband light are emitted to the observation target in a time division manner and the second narrowband light is emitted to the observation target, and
the optical element guides the first light flux including reflected light from the observation target irradiated with the broadband light to the first imaging element, guides the second light flux including the first fluorescence from the observation target irradiated with the first narrowband light to the second imaging element, and guides the third light flux including the second fluorescence from the observation target irradiated with the second narrowband light to the third imaging element.

### [Item 30]

The medical observation system according to item 28 or 29, in which
the light source apparatus emits third narrowband light that excites a third substance emitting third fluorescence in a wavelength band that is not included in the first wavelength band and is at least partially different from a wavelength band of the second fluorescence,
in a fourth mode different from the first mode and the second mode,
   the control section controls the light source apparatus such that the broadband light and the third narrowband light are emitted to the observation target, and
   the optical element guides the first light flux including reflected light from the observation target irradiated with the broadband light to the first imaging element, and guides the third light flux including the third fluorescence from the observation target irradiated with the third narrowband light to the third imaging element.

### [Item 31]

The medical observation system according to any one of items 28 to 30, in which
the light source apparatus emits third narrowband light that excites a third substance emitting third fluorescence in a wavelength band that is not included in the first wavelength band and is at least partially different from a wavelength band of the second fluorescence,
in a fifth mode different from the first mode and the second mode,
   the control section controls the light source apparatus such that the broadband light and the first narrowband light are emitted to the observation target in a time division manner and the second narrowband light and the third narrowband light are emitted to the observation target in a time division manner, and
   the optical element guides the first light flux including reflected light from the observation target irradiated with the broadband light to the first imaging element, guides the second light flux including the first fluorescence from the observation target irradiated with the first narrowband light to the second imaging element, and sequentially guides the third light flux including the second fluorescence from the observation target irradiated with the second narrowband light and the third light flux including the third fluorescence from the observation target irradiated with the third narrowband light to the third imaging element.

### [Item 32]

The medical observation system according to any one of items 23 to 26, in which
light in the wavelength band of the second fluorescence is partially, substantially, or completely suppressed in the first light flux,
light in the first wavelength band is partially, substantially, or completely suppressed in the second light flux, and
light in the first wavelength band is partially, substantially, or completely suppressed in the third light flux.

### [Item 33]

The medical observation system according to item 32, in which
the optical element is configured to:
in the first mode, sequentially guide the first light flux including the reflected light from the observation target irradiated with the broadband light and the first light flux including the first fluorescence from the observation target irradiated with the first narrowband light to the first imaging element; and
in the second mode, guide the first light flux including the reflected light from the observation target irradiated with the broadband light to the first imaging element, and guide the second light flux including the second fluorescence from the observation target irradiated with the second narrowband light to the second imaging element.

### [Item 34]

The medical observation system according to item 33, in which
in a third mode different from the first mode and the second mode,
the control section controls the light source apparatus such that the broadband light and the first narrowband light are emitted to the observation target in a time division manner and the second narrowband light is emitted to the observation target, and
the optical element sequentially guides the first light flux including the reflected light from the observation target irradiated with the broadband light and the first light flux including the first fluorescence from the observation target irradiated with the first narrowband light to the first imaging element, and guides the second light flux including the second fluorescence from the observation target irradiated with the second narrowband light to the second imaging element.

### [Item 35]

The medical observation system according to item 33 or 34, in which
in a fourth mode different from the first mode and the second mode,
the control section controls the light source apparatus such that the broadband light and the third narrowband light are emitted to the observation target, and
the optical element guides the first light flux including reflected light from the observation target irradiated with the broadband light to the first imaging element, and guides the second light flux including third fluorescence from the observation target irradiated with the third narrowband light to the second imaging element.

### [Item 36]

The medical observation system according to item 33 or 34, in which
the light source apparatus emits third narrowband light that excites a third substance emitting third fluorescence in a wavelength band that is not included in the first wavelength band and is at least partially different from a wavelength band of the second fluorescence,
in a fourth mode different from the first mode and the second mode,
   the control section controls the light source apparatus such that the broadband light and the third narrowband light are emitted to the observation target, and
   the optical element guides the first light flux including reflected light from the observation target irradiated with the broadband light to the first imaging element, and guides the third light flux including the third fluorescence from the observation target irradiated with the third narrowband light to the third imaging element.

### [Item 37]

The medical observation system according to any one of items 33 to 36, further including
an image generation section that generates an image on the basis of an image signal from the imaging section, in which
the control section controls the imaging section and the image generation section,
the light source apparatus emits third narrowband light that excites a third substance emitting third fluorescence in a wavelength band that is not included in the first wavelength band and at least partially coincides with the wavelength band of the second fluorescence,
in a fifth mode different from the first mode and the second mode,
   the control section controls the light source apparatus such that the broadband light is emitted to the observation target and the second narrowband light and the third narrowband light are emitted to the observation target in a time division manner,
   the optical element guides the first light flux including reflected light from the observation target irradiated with the broadband light to the first imaging element, sequentially guides the second light flux including the second fluorescence from the observation target irradiated with the second narrowband light and the second light flux including the third fluorescence from the observation target irradiated with the third narrowband light to the second imaging element, and sequentially guides the third light flux including the second fluorescence from the observation target irradiated with the second narrowband light and the third light flux including the third fluorescence from the observation target irradiated with the third narrowband light to the third imaging element, and
   the control section controls the imaging section and the image generation section such that an image based on reflected light is generated on the basis of an image signal output from the first imaging element that has received the first light flux including reflected light, an image based on the second fluorescence is generated on the basis of an image signal output from the second imaging element that has received the second light flux including the second fluorescence, and image data of the third fluorescence is generated on the basis of an image signal output from the third imaging element that has received the third light flux including the third fluorescence.

### [Item 38]

The medical observation system according to any one of items 33 to 37, further including
an image generation section that generates an image on the basis of an image signal from the imaging section, in which
the control section controls the imaging section and the image generation section,
the light source apparatus emits third narrowband light that excites a third substance emitting third fluorescence in a wavelength band that is not included in the first wavelength band and at least partially coincides with the wavelength band of the second fluorescence,
in a sixth mode different from the first mode and the second mode,
   the control section controls the light source apparatus such that the broadband light and the first narrowband light are emitted to the observation target in a time division manner and the second narrowband light and the third narrowband light are emitted to the observation target in a time division manner,
   the optical element sequentially guides the first light flux including the reflected light from the observation target irradiated with the broadband light and the first light flux including the first fluorescence from the observation target irradiated with the first narrowband light to the first imaging element, sequentially guides the second light flux including the second fluorescence from the observation target irradiated with the second narrowband light and the second light flux including the third fluorescence from the observation target irradiated with the third narrowband light to the second imaging element, and sequentially guides the third light flux including the second fluorescence from the observation target irradiated with the second narrowband light and the third light flux including the third fluorescence from the observation target irradiated with the third narrowband light to the third imaging element, and
   the control section controls the imaging section and the image generation section such that an image based on reflected light is generated on the basis of an image signal output from the first imaging element that has received the first light flux including reflected light, an image based on the first fluorescence is generated on the basis of an image signal output from the first imaging element that has received the first light flux including the first fluorescence, an image based on the second fluorescence is generated on the basis of an image signal output from the second imaging element that has received the second light flux including the second fluorescence, and image data of the third fluorescence is generated on the basis of an image signal output from the third imaging element that has received the third light flux including the third fluorescence.

### [Item 39]

The medical observation system according to item 23 or 24, in which
the first imaging element and the second imaging element include a color filter.

### [Item 40]

The medical observation system according to item 23, 24, or 39, in which
the third imaging element does not have a color filter.

### [Item 41]

The medical observation system according to item 39 or 40, in which
light in the wavelength band of the second fluorescence is partially, substantially, or completely suppressed in the first light flux,
light in the wavelength band of the second fluorescence is partially, substantially, or completely suppressed in the second light flux, and
light in the first wavelength band is partially, substantially, or completely suppressed in the third light flux.

### [Item 42]

The medical observation system according to item 41, further including
an image generation section that generates an image on the basis of an image signal from the imaging section, in which
the control section controls the imaging section and the image generation section,
in the first mode,
   the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light and the second light flux including the first fluorescence from the observation target irradiated with the first narrowband light to the second imaging element,
   the control section is configured to:
      control the imaging section and the image generation section such that an image based on reflected light is generated on the basis of one or both of an image signal output from the first imaging element that has received the first light flux including reflected light and an image signal output from the second imaging element that has received the second light flux including reflected light; and
      control the imaging section and the image generation section such that an image based on the first fluorescence is generated on the basis of an image signal output from the second imaging element that has received the second light flux including the first fluorescence,
in the second mode,
   the optical element guides the first light flux including reflected light from the observation target irradiated with the broadband light to the first imaging element, guides the second light flux including reflected light from the observation target irradiated with the broadband light to the second imaging element, and guides the third light flux including the second fluorescence from the observation target irradiated with the second narrowband light to the third imaging element, and
   the control section is configured to:
      control the imaging section and the image generation section such that an image based on reflected light is generated on the basis of one or both of an image signal output from the first imaging element that has received the first light flux including reflected light and an image signal output from the second imaging element that has received the second light flux including reflected light; and
      control the imaging section and the image generation section such that an image based on the second fluorescence is generated on the basis of an image signal output from the third imaging element that has received the third light flux including the second fluorescence.

### [Item 43]

The medical observation system according to item 39 or 40, further including
an image generation section that generates an image on the basis of an image signal from the imaging section, in which
the control section controls the imaging section and the image generation section,
in a third mode different from the first mode and the second mode,
   the control section controls the light source apparatus such that the broadband light and the first narrowband light are emitted to the observation target in a time division manner and the second narrowband light is emitted to the observation target,
   the optical element sequentially guides the first light flux including the reflected light from the observation target irradiated with the broadband light and the first light flux including the first fluorescence from the observation target irradiated with the first narrowband light to the first imaging element, sequentially guides the second light flux including the reflected light from the observation target irradiated with the broadband light and the second light flux including the first fluorescence from the observation target irradiated with the first narrowband light to the second imaging element, and guides the third light flux including the second fluorescence from the observation target irradiated with the second narrowband light to the third imaging element, and
   the control section is configured to:
      control the imaging section and the image generation section such that an image based on reflected light is generated on the basis of one or both of an image signal output from the first imaging element that has received the first light flux including reflected light and an image signal output from the second imaging element that has received the second light flux including reflected light;
      control the imaging section and the image generation section such that an image based on the first fluorescence is generated on the basis of an image signal output from the second imaging element that has received the second light flux including the first fluorescence; and
      control the imaging section and the image generation section such that an image based on the second fluorescence is generated on the basis of an image signal output from the third imaging element that has received the third light flux including the second fluorescence.

### [Item 44]

The medical observation system according to any one of items 1 to 43, in which the first wavelength band is included in a visible light wavelength band.

### [Item 45]

The medical observation system according to any one of items 1 to 44, in which the wavelength of the second fluorescence is included in an invisible light wavelength band.

### [Item 46]

The medical observation system according to any one of items 2 to 43, in which the first imaging element has a higher resolution than the second imaging element.

### [Item 47]

The medical observation system according to any one of items 1 to 46, further including a filter element that partially, substantially, or completely suppresses light in a wavelength band of the first narrowband light.

### [Item 48]

The medical observation system according to any one of items 1 to 47, further including a filter element that partially, substantially, or completely suppresses light in a wavelength band of the second narrowband light.

### [Item 49]

The medical observation system according to item 21 or 43, further including
an instruction acceptance section that accepts an instruction from a user, in which
the control section determines whether to use one or both of the image signal output from the first imaging element and the image signal output from the second imaging element for generation of the image based on the reflected light on the basis of the instruction from the user received by the instruction receiving section.

### [Item 50]

A medical observation method including:
a step of emitting, from a light source apparatus, at least one of broadband light in a first wavelength band, first narrowband light that excites a first substance that emits first fluorescence in a wavelength band included in the first wavelength band, or second narrowband light that excites a second substance that emits second fluorescence in a wavelength band not included in the first wavelength band, in which
in a first mode, the broadband light and the first narrowband light are emitted from the light source apparatus such that the broadband light and the first narrowband light are emitted to an observation target in a time division manner, and
in a second mode different from the first mode, the broadband light and the second narrowband light are emitted from the light source apparatus such that the observation target is irradiated with the broadband light and the second narrowband light.

### [Item 51]

An image generation apparatus that generates an output image on the basis of a first image that is an image obtained by imaging a first range of an observation target using a first imaging element and a second image that is an image obtained by imaging a second range different from the first range of the observation target using a second imaging element different in type from the first imaging element, in which
the output image is an image obtained by performing processing of replacing or deleting one or more pixel signals among the pixel signals of the first image and the second image on the basis of the first range and the second range.

### [Item 52]

The image generation apparatus according to item 51, in which
the type is a physical size, an image size, or a model number of an imaging element.

### [Item 53]

The image generation apparatus according to item 51 or 52, in which
the image generation apparatus outputting:
a first output image that is the output image; or
a second output image generated by adding one or more pixel signals to an image generated from at least some pixel signals of the pixel signals of the second image.

### [Item 54]

The image generation apparatus according to any one of items 51 to 53, in which
one of the first range and the second range is wider than another.

### [Item 55]

The image generation apparatus according to any one of items 51 to 54, in which
a first image region of the output image is generated on the basis of the first image and the second image.

### [Item 56]

The image generation apparatus according to item 55, in which
the output image includes the first image region and a second image region different from the first image region.

### [Item 57]

The image generation apparatus according to item 56, in which
an image visually distinguishable and identifiable from the image allocated to the first image region is allocated to the second image region.

### [Item 58]

The image generation apparatus according to item 55, in which
the output image includes only the first image region.

### [Item 59]

The image generation apparatus according to any one of items 56 and 57, in which
the output image includes a boundary-enhanced image indicating a boundary between the first image region and the second image region.

### [Item 60]

The image generation apparatus according to any one of items 51 to 59, in which
the second range is included in the first range.

### [Item 61]

The image generation apparatus according to any one of items 51 to 60, in which
the output image is generated on the basis of the second image as a whole.

### [Item 62]

The image generation apparatus according to any one of items 55 to 59, the image generation apparatus being configured to:
enlarge or reduce one or both of the first image and the second image such that the number of pixels of a common region that is an image region of the observation target portion commonly included in the first image and the second image matches between the first image and the second image; and
generate an image allocated to the first image region of the output image on the basis of the image of the common region in the first image and the image of the common region in the second image having the same number of pixels.

### [Item 63]

The image generation apparatus according to any one of items 51 to 62, the image generation apparatus being configured to:
be provided to be connectable to one or a plurality of display apparatuses on which the output image is displayed; and
generate and output the output image corresponding to each characteristic of the one or the plurality of display apparatuses.

### [Item 64]

The image generation apparatus according to any one of items 51 to 63, in which
the first image is acquired by capturing the observation target irradiated with first light in a first wavelength band, and
the second image is acquired by capturing the observation target irradiated with second light in a wavelength band at least partially different from the first wavelength band.

### [Item 65]

The image generation apparatus according to item 64, in which
the first light is visible light,
the second light is excitation light that excites a specific substance so as to emit fluorescence,
the first image includes a captured image based on reflected light of the visible light from the observation target, and
the second image includes a captured image based on the fluorescence from the observation target irradiated with the excitation light.

### [Item 66]

The medical observation system according to any one of item 1 to 49, further including:
an imaging apparatus including a first imaging element and a second imaging element different in type from the first imaging element; and
an image generation apparatus that generates an output image, in which
the image generation apparatus is configured to
   generate the output image on the basis of a first image that is an image obtained by imaging a first range of an observation target using the first imaging element and a second image that is an image obtained by imaging a second range different from the first range of the observation target using the second imaging element, and
the output image is an image obtained by performing processing of replacing or deleting one or more pixel signals among the pixel signals of the first image and the second image on the basis of the first range and the second range.

### [Item 67]

The medical observation system according to item 66, further including
a light source apparatus that emits light with which the observation target is irradiated, in which
the imaging apparatus captures the observation target irradiated with the light and acquires the first image and the second image.

### [Item 68]

The medical observation system according to item 66 or 67, in which
the type is a physical size, an image size, or a model number of the imaging element.

### [Item 69]

The medical observation system according to any one of items 66 to 68, in which
the image generation apparatus outputting:
a first output image that is the output image; or
a second output image generated by adding one or more pixel signals to an image generated from at least some pixel signals of the pixel signals of the second image.

### [Item 70]

The medical observation system according to any one of items 66 to 69, in which
one of the first range and the second range is wider than another.

### [Item 71]

The medical observation system according to any one of items 66 to 70, in which
a first image region of the output image is generated on the basis of the first image and the second image.

### [Item 72]

The medical observation system according to item 71, in which
the output image includes the first image region and a second image region different from the first image region.

### [Item 73]

The medical observation system according to item 72, in which
an image visually distinguishable and identifiable from the image allocated to the first image region is allocated to the second image region.

### [Item 74]

The medical observation system according to item 71, in which
the output image includes only the first image region.

### [Item 75]

The medical observation system according to any one of items 72 and 73, in which
the output image includes a boundary-enhanced image indicating a boundary between the first image region and the second image region.

### [Item 76]

The medical observation system according to any one of items 66 to 75, in which
the second range is included in the first range.

### [Item 77]

The medical observation system according to any one of items 66 to 76, in which
the output image is generated on the basis of the second image as a whole.

### [Item 78]

The medical observation system according to any one of items 71 to 75, the medical observation system being configured to:
enlarge or reduce one or both of the first image and the second image such that the number of pixels of a common region that is the observation target portion commonly included in the first image and the second image matches between the first image and the second image; and
generate an image allocated to the first image region of the output image on the basis of the image of the common region in the first image and the image of the common region in the second image having the same number of pixels.

### [Item 79]

The medical observation system according to any one of items 66 to 78, the medical observation system being configured to:
be provided to be connectable to one or a plurality of display apparatuses on which the output image is displayed; and
generate and output the output image corresponding to each characteristic of the one or the plurality of display apparatuses.

### [Item 80]

The medical observation system according to any one of items 66 to 79, in which
the first image is acquired by capturing the observation target irradiated with first light in a first wavelength band, and
the second image is acquired by capturing the observation target irradiated with second light in a wavelength band at least partially different from the first wavelength band.

### [Item 81]

The medical observation system according to item 80, in which
the first light is visible light,
the second light is excitation light that excites a specific substance so as to emit fluorescence,
the first image includes a captured image based on reflected light of the visible light from the observation target, and
the second image includes a captured image based on the fluorescence from the observation target irradiated with the excitation light.

### [Item 82]

A medical observation method according to item 50, the medical observation method including
a step of generating an output image on the basis of a first image that is an image obtained by imaging a first range of an observation target using a first imaging element and a second image that is an image obtained by imaging a second range different from the first range of the observation target using a second imaging element different in type from the first imaging element, in which
the output image is an image obtained by performing processing of replacing or deleting one or more pixel signals among the pixel signals of the first image and the second image on the basis of the first range and the second range.

### [Item 83]

The medical observation method according to item 82, further including:
a size adjustment processing step of adjusting a size of both or one of the first image and the second image; and
a step of generating the output image on the basis of the first image and the second image after the size adjustment processing step.

### [Item 84]

The medical observation method according to item 82, further including:
a step of extracting an image of a range in which the observation target is captured in the first image;
a size adjustment processing step of adjusting a size of both or one of the first image and the second image extracted; and
a step of generating the output image on the basis of the first image and the second image extracted after the size adjustment processing step.

### [Item 85]

The medical observation method according to any one of items 82 to 84, in which
the type is a physical size, an image size, or a model number of the imaging element.

### [Item 86]

The medical observation method according to any one of items 82 to 85, in which
the image generation apparatus outputting:
a first output image that is the output image; or
a second output image generated by adding one or more pixel signals to an image generated from at least some pixel signals of the pixel signals of the second image.

### [Item 87]

The medical observation method according to any one of items 82 to 86, in which
one of the first range and the second range is wider than another.

### [Item 88]

The medical observation method according to any one of items 82 to 87, in which
a first image region of the output image is generated on the basis of the first image and the second image.

### [Item 89]

The medical observation method according to item 88, in which
the output image includes the first image region and a second image region different from the first image region.

### [Item 90]

The medical observation method according to item 89, in which
an image visually distinguishable and identifiable from the image allocated to the first image region is allocated to the second image region.

### [Item 91]

The medical observation method according to item 88, in which
the output image includes only the first image region.

### [Item 92]

The medical observation method according to any one of items 89 and 90, in which
the output image includes a boundary-enhanced image indicating a boundary between the first image region and the second image region.

### [Item 93]

The medical observation method according to any one of items 82 to 92, in which
the second range is included in the first range.

### [Item 94]

The medical observation method according to any one of items 82 to 93, in which
the output image is generated on the basis of the second image as a whole.

### [Item 95]

The medical observation method according to any one of items 88 to 92, the medical observation method including:
enlarging or reducing one or both of the first image and the second image such that the number of pixels of a common region that is the observation target portion commonly included in the first image and the second image matches between the first image and the second image; and
generating an image allocated to the first image region of the output image on the basis of the image of the common region in the first image and the image of the common region in the second image having the same number of pixels.

### [Item 96]

The medical observation method according to any one of items 82 to 95, the medical observation method being configured to:
be provided to be connectable to one or a plurality of display apparatuses on which the output image is displayed; and
generate and output the output image corresponding to each characteristic of the one or the plurality of display apparatuses.

### [Item 97]

The medical observation method according to any one of items 82 to 96, in which
the first image is acquired by capturing the observation target irradiated with first light in a first wavelength band, and
the second image is acquired by capturing the observation target irradiated with second light in a wavelength band at least partially different from the first wavelength band.

### [Item 98]

The medical observation method according to item 97, in which
the first light is visible light,
the second light is excitation light that excites a specific substance so as to emit fluorescence,
the first image includes a captured image based on reflected light of the visible light from the observation target, and
the second image includes a captured image based on the fluorescence from the observation target irradiated with the excitation light.

### [Item 99]

The medical observation system according to any one of items 1 to 49 and 66 to 81, further including
an image generation section that generates an image on the basis of an image signal from an imaging section that captures an image of the observation target, in which
in a case where a mode is switched from one of the first mode and the second mode to another mode under control of the control section, the image generation section uses at least some of parameters used in the one mode while maintaining the another mode.

### [Item 100]

The medical observation system according to item 99, in which
the parameter is a parameter related to at least one of a white balance, a color tone, or a color mode.

### [Item 101]

The image generation apparatus according to any one of items 51 to 65, in which
in a case where a mode is switched from one of the first mode and the second mode to another mode under control of the control section, at least some of parameters used in the one mode are used while being maintained in the another mode.

### [Item 102]

The image generation apparatus according to item 101, in which
the parameter is a parameter related to at least one of a white balance, a color tone, or a color mode.

### [Item 103]

The medical observation method according to any one of items 50 and 82 to 98, in which
an image generation section that generates an image on the basis of an image signal from an imaging section that images the observation target uses at least some of parameters used in one mode while maintaining another mode in a case where the one mode is switched from one of the first mode and the second mode to the another mode under control of the control section.

### [Item 104]

The medical observation method according to item 103, in which
the parameter is a parameter related to at least one of a white balance, a color tone, or a color mode.

### REFERENCE SIGNS LIST

- 10: Light source apparatus
- 11: Broadband light source
- 12: First narrowband light source
- 13: Second narrowband light source
- 14: Third narrowband light source
- 15: Optical element
- 20: Insertion device
- 21: Insertion portion
- 21a: Distal end portion
- 22: Optical connection portion
- 23: Imaging connection portion
- 30: Light guide
- 40: Lens optical system
- 41: Mirror optical system
- 50: Camera head
- 51: Lens unit
- 52: Imaging section
- 53: Communication section
- 60: Ring light
- 70: Display apparatus
- 80: Transmission cable
- 90: Control apparatus
- 91: Communication section
- 92: Memory
- 93: Image generation section
- 94: Control section
- 95: Input section
- 96: Output section
- 97: Storage section
- 100: Medical observation system
- 110: Microscope apparatus
- 123: Transmission cable
- 124: Support portion
- 125: Microscope portion
- 126: Base portion
- 210: Output image
- 211: Observation target image
- 213: First identification target image
- 214: Second identification target image
- 220: Main image
- 221: First reduced display image
- 222: Second reduced display image
- 521: Light incident section
- 522: Imaging element
- 522a: First imaging element
- 522b: Second imaging element
- 522c: Third imaging element
- 523: Signal processing section
- 930: Memory controller
- 931: Image processing section
- 934: Superimposed image generation section
- 935: Display control section
- Bs: Branching optical system
- Bs1: First branching optical system
- Bs2: Second branching optical system
- CF: Color filter
- FC: Excitation light cut filter
- FL: Wavelength selection filter
- FL1: First wavelength selection filter
- FL2: Second wavelength selection filter
- L1: Broadband light
- L2: First narrowband light
- L3: Second narrowband light
- L4: Third narrowband light
- Lf: Observation light
- Lf1: First light flux
- Lf2: Second light flux
- Lf3: Third light flux
- Lw1: Broadband reflected light
- Lw2: First fluorescence
- Lw3: Second fluorescence
- Lw4: Third fluorescence
- PR: Color separation prism
- S: Observation target

## Claims

1. A medical observation system comprising:
a light source apparatus that emits broadband light of a first wavelength band, first narrowband light that excites a first substance that emits first fluorescence of a wavelength band included in the first wavelength band, and second narrowband light that excites a second substance that emits second fluorescence of a wavelength band not included in the first wavelength band; and
a control section that controls the light source apparatus, wherein
the control section is configured to:
control the light source apparatus such that the broadband light and the first narrowband light are emitted to an observation target in a time division manner in a first mode; and
control the light source apparatus such that the broadband light and the second narrowband light are emitted to the observation target in a second mode different from the first mode.

2. The medical observation system according to claim 1, further comprising:
an imaging section including a first imaging element and a second imaging element; and
an optical element that separates light from the observation target into a plurality of light fluxes including a first light flux and a second light flux, guides the first light flux to the first imaging element, and guides the second light flux to the second imaging element.

3. The medical observation system according to claim 2, wherein
the second imaging element has higher sensitivity than the first imaging element.

4. The medical observation system according to claim 2, wherein
the first imaging element includes a color filter.

5. The medical observation system according to claim 2, wherein
the second imaging element has no color filter.

6. The medical observation system according to claim 2, wherein
light in a wavelength band of the second fluorescence is partially, substantially, or completely suppressed in the first light flux, and
light in the first wavelength band is partially, substantially, or completely suppressed in the second light flux.

7. The medical observation system according to claim 6, wherein
the optical element is configured to:
in the first mode, sequentially guide the first light flux including reflected light from the observation target irradiated with the broadband light and the first light flux including the first fluorescence from the observation target irradiated with the first narrowband light to the first imaging element; and
in the second mode, guide the first light flux including the reflected light from the observation target irradiated with the broadband light to the first imaging element, and guide the second light flux including the second fluorescence from the observation target irradiated with the second narrowband light to the second imaging element.

8. The medical observation system according to claim 7, wherein
in a third mode different from the first mode and the second mode,
the control section controls the light source apparatus such that the broadband light and the first narrowband light are emitted to the observation target in a time division manner and the second narrowband light is emitted to the observation target, and
the optical element sequentially guides the first light flux including the reflected light from the observation target irradiated with the broadband light and the first light flux including the first fluorescence from the observation target irradiated with the first narrowband light to the first imaging element, and guides the second light flux including the second fluorescence from the observation target irradiated with the second narrowband light to the second imaging element.

9. The medical observation system according to claim 7, wherein
the light source apparatus emits third narrowband light that excites a third substance emitting third fluorescence in a wavelength band that is not included in the first wavelength band and is at least partially different from a wavelength band of the second fluorescence,
in a fourth mode different from the first mode and the second mode,
the control section controls the light source apparatus such that the broadband light and the third narrowband light are emitted to the observation target, and
the optical element guides the first light flux including the reflected light from the observation target irradiated with the broadband light to the first imaging element, and guides the second light flux including the third fluorescence from the observation target irradiated with the third narrowband light to the second imaging element.

10. The medical observation system according to claim 7, wherein
the light source apparatus emits third narrowband light that excites a third substance emitting third fluorescence in a wavelength band that is not included in the first wavelength band and is at least partially different from a wavelength band of the second fluorescence,
in a fifth mode different from the first mode and the second mode,
the control section controls the light source apparatus such that the broadband light and the first narrowband light are emitted to the observation target in a time division manner and the second narrowband light and the third narrowband light are emitted to the observation target in a time division manner, and
the optical element sequentially guides the first light flux including the reflected light from the observation target irradiated with the broadband light and the first light flux including the first fluorescence from the observation target irradiated with the first narrowband light to the first imaging element, and sequentially guides the second light flux including the second fluorescence from the observation target irradiated with the second narrowband light and the second light flux including the third fluorescence from the observation target irradiated with the third narrowband light to the second imaging element.

11. The medical observation system according to claim 2, wherein
light in the wavelength band of the second fluorescence is partially, substantially, or completely suppressed in the first light flux.

12. The medical observation system according to claim 11, wherein
the optical element is configured to:
guide the first light flux in which the light of the first wavelength band is partially suppressed to the first imaging element; and
guide the second light flux in which the light of the first wavelength band is partially suppressed to the second imaging element.

13. The medical observation system according to claim 12, further comprising
an image generation section that generates an image on a basis of an image signal from the imaging section, wherein
the control section controls the imaging section and the image generation section,
in the first mode,
the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light and the second light flux including the first fluorescence from the observation target irradiated with the first narrowband light to the second imaging element,
the control section controls the imaging section and the image generation section such that an image based on reflected light is generated on a basis of an image signal output from the first imaging element that has received the first light flux including the reflected light, and an image based on the first fluorescence is generated on a basis of an image signal output from the second imaging element that has received the second light flux including the first fluorescence,
in the second mode,
the optical element guides the first light flux including the reflected light from the observation target irradiated with the broadband light to the first imaging element, and sequentially guides the second light flux including the reflected light from the observation target irradiated with the broadband light and the second light flux including the second fluorescence from the observation target irradiated with the second narrowband light to the second imaging element, and
the control section controls the imaging section and the image generation section such that an image based on reflected light is generated on a basis of an image signal output from the first imaging element that has received the first light flux including the reflected light, and an image based on the second fluorescence is generated on a basis of an image signal output from the second imaging element that has received the second light flux including the second fluorescence.

14. The medical observation system according to claim 12, further comprising
an image generation section that generates an image on a basis of an image signal from the imaging section, wherein
the control section controls the imaging section and the image generation section,
in a third mode different from the first mode and the second mode,
the control section controls the light source apparatus such that the broadband light, the first narrowband light, and the second narrowband light are emitted to the observation target in a time division manner,
the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light, the second light flux including the first fluorescence from the observation target irradiated with the first narrowband light, and the second light flux including the second fluorescence from the observation target irradiated with the second narrowband light to the second imaging element, and
the control section controls the imaging section and the image generation section such that an image based on reflected light is generated on a basis of an image signal output from the first imaging element that has received the first light flux including reflected light, an image based on the first fluorescence is generated on a basis of an image signal output from the second imaging element that has received the second light flux including the first fluorescence, and an image based on the second fluorescence is generated on a basis of an image signal output from the second imaging element that has received the second light flux including the second fluorescence.

15. The medical observation system according to claim 11, wherein
the optical element is configured to:
guide the first light flux in which the light in the wavelength band of the first fluorescence is partially, substantially, or completely suppressed to the first imaging element; and
guide the second light flux in which light in a wavelength band other than the wavelength band of the first fluorescence in the first wavelength band is partially, substantially, or completely suppressed to the second imaging element.

16. The medical observation system according to claim 15, wherein
the optical element is configured to:
in the first mode, guide the first light flux including the reflected light from the observation target irradiated with the broadband light to the first imaging element, and guide the second light flux including the first fluorescence from the observation target irradiated with the first narrowband light to the second imaging element; and
in the second mode, guide the first light flux including the reflected light from the observation target irradiated with the broadband light to the first imaging element, and guide the second light flux including the second fluorescence from the observation target irradiated with the second narrowband light to the second imaging element.

17. The medical observation system according to claim 16, wherein
in a third mode different from the first mode and the second mode,
the control section controls the light source apparatus such that the broadband light is emitted to the observation target and the first narrowband light and the second narrowband light are emitted to the observation target in a time division manner, and
the optical element guides the first light flux including reflected light from the observation target irradiated with the broadband light to the first imaging element, and sequentially guides the second light flux including the first fluorescence from the observation target irradiated with the first narrowband light and the second light flux including the second fluorescence from the observation target irradiated with the second narrowband light to the second imaging element.

18. The medical observation system according to claim 4, wherein
the second imaging element includes a color filter.

19. The medical observation system according to claim 18, wherein
light in the wavelength band of the second fluorescence is partially, substantially, or completely suppressed in the first light flux.

20. The medical observation system according to claim 19, wherein
the optical element is configured to:
guide the first light flux in which the light of the first wavelength band is partially suppressed to the first imaging element; and
guide the second light flux in which the light of the first wavelength band is partially suppressed to the second imaging element.

21. The medical observation system according to claim 18, further comprising
an image generation section that generates an image on a basis of an image signal from the imaging section, wherein
the control section controls the imaging section and the image generation section,
in the first mode,
the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light and the second light flux including the first fluorescence from the observation target irradiated with the first narrowband light to the second imaging element,
the control section is configured to:
control the imaging section and the image generation section such that an image based on reflected light is generated on a basis of one or both of an image signal output from the first imaging element that has received the first light flux including reflected light and an image signal output from the second imaging element that has received the second light flux including reflected light; and
control the imaging section and the image generation section such that an image based on the first fluorescence is generated on a basis of an image signal output from the second imaging element that has received the second light flux including the first fluorescence,
in the second mode,
the optical element guides the first light flux including the reflected light from the observation target irradiated with the broadband light to the first imaging element, and sequentially guides the second light flux including the reflected light from the observation target irradiated with the broadband light and the second light flux including the second fluorescence from the observation target irradiated with the second narrowband light to the second imaging element, and
the control section controls the imaging section and the image generation section such that an image based on reflected light is generated on a basis of an image signal output from the first imaging element that has received the first light flux including the reflected light, and an image based on the second fluorescence is generated on a basis of an image signal output from the second imaging element that has received the second light flux including the second fluorescence.

22. The medical observation system according to claim 18, further comprising
an image generation section that generates an image on a basis of an image signal from the imaging section, wherein
the control section controls the imaging section and the image generation section,
in a third mode different from the first mode and the second mode,
the control section controls the light source apparatus such that the broadband light, the first narrowband light, and the second narrowband light are emitted to the observation target in a time division manner,
the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light, the second light flux including the first fluorescence from the observation target irradiated with the first narrowband light, and the second light flux including the second fluorescence from the observation target irradiated with the second narrowband light to the second imaging element, and
the control section controls the imaging section and the image generation section such that an image based on reflected light is generated on a basis of an image signal output from the first imaging element that has received the first light flux including reflected light, an image based on the first fluorescence is generated on a basis of an image signal output from the second imaging element that has received the second light flux including the first fluorescence, and an image based on the second fluorescence is generated on a basis of an image signal output from the second imaging element that has received the second light flux including the second fluorescence.

23. The medical observation system according to claim 1, further comprising
an imaging section including a first imaging element, a second imaging element, and a third imaging element; and
an optical element that separates light from the observation target into a plurality of light fluxes including a first light flux, a second light flux, and a third light flux, guides the first light flux to the first imaging element, guides the second light flux to the second imaging element, and guides the third light flux to the third imaging element.

24. The medical observation system according to claim 23, wherein
the second imaging element and the third imaging element have higher sensitivity than the first imaging element.

25. The medical observation system according to claim 23, wherein
the first imaging element includes a color filter.

26. The medical observation system according to claim 23, wherein
the second imaging element and the third imaging element have no color filter.

27. The medical observation system according to claim 23, wherein
light in the wavelength band of the second fluorescence is partially, substantially, or completely suppressed in the first light flux,
light in the wavelength band of the second fluorescence is partially, substantially, or completely suppressed in the second light flux, and
light in the first wavelength band is partially, substantially, or completely suppressed in the third light flux.

28. The medical observation system according to claim 23, wherein
the optical element is configured to:
in the first mode, guide the first light flux including the reflected light from the observation target irradiated with the broadband light to the first imaging element, and guide the second light flux including the first fluorescence from the observation target irradiated with the first narrowband light to the second imaging element; and
in the second mode, guide the first light flux including the reflected light from the observation target irradiated with the broadband light to the first imaging element, and guide the third light flux including the second fluorescence from the observation target irradiated with the second narrowband light to the third imaging element.

29. The medical observation system according to claim 28, wherein
in a third mode different from the first mode and the second mode,
the control section controls the light source apparatus such that the broadband light and the first narrowband light are emitted to the observation target in a time division manner and the second narrowband light is emitted to the observation target, and
the optical element guides the first light flux including reflected light from the observation target irradiated with the broadband light to the first imaging element, guides the second light flux including the first fluorescence from the observation target irradiated with the first narrowband light to the second imaging element, and guides the third light flux including the second fluorescence from the observation target irradiated with the second narrowband light to the third imaging element.

30. The medical observation system according to claim 28, wherein
the light source apparatus emits third narrowband light that excites a third substance emitting third fluorescence in a wavelength band that is not included in the first wavelength band and is at least partially different from a wavelength band of the second fluorescence,
in a fourth mode different from the first mode and the second mode,
the control section controls the light source apparatus such that the broadband light and the third narrowband light are emitted to the observation target, and
the optical element guides the first light flux including reflected light from the observation target irradiated with the broadband light to the first imaging element, and guides the third light flux including the third fluorescence from the observation target irradiated with the third narrowband light to the third imaging element.

31. The medical observation system according to claim 28, wherein
the light source apparatus emits third narrowband light that excites a third substance emitting third fluorescence in a wavelength band that is not included in the first wavelength band and is at least partially different from a wavelength band of the second fluorescence,
in a fifth mode different from the first mode and the second mode,
the control section controls the light source apparatus such that the broadband light and the first narrowband light are emitted to the observation target in a time division manner and the second narrowband light and the third narrowband light are emitted to the observation target in a time division manner, and
the optical element guides the first light flux including reflected light from the observation target irradiated with the broadband light to the first imaging element, guides the second light flux including the first fluorescence from the observation target irradiated with the first narrowband light to the second imaging element, and sequentially guides the third light flux including the second fluorescence from the observation target irradiated with the second narrowband light and the third light flux including the third fluorescence from the observation target irradiated with the third narrowband light to the third imaging element.

32. The medical observation system according to claim 23, wherein
light in the wavelength band of the second fluorescence is partially, substantially, or completely suppressed in the first light flux,
light in the first wavelength band is partially, substantially, or completely suppressed in the second light flux, and
light in the first wavelength band is partially, substantially, or completely suppressed in the third light flux.

33. The medical observation system according to claim 32, wherein
the optical element is configured to:
in the first mode, sequentially guide the first light flux including the reflected light from the observation target irradiated with the broadband light and the first light flux including the first fluorescence from the observation target irradiated with the first narrowband light to the first imaging element; and
in the second mode, guide the first light flux including the reflected light from the observation target irradiated with the broadband light to the first imaging element, and guide the second light flux including the second fluorescence from the observation target irradiated with the second narrowband light to the second imaging element.

34. The medical observation system according to claim 33, wherein
in a third mode different from the first mode and the second mode,
the control section controls the light source apparatus such that the broadband light and the first narrowband light are emitted to the observation target in a time division manner and the second narrowband light is emitted to the observation target, and
the optical element sequentially guides the first light flux including the reflected light from the observation target irradiated with the broadband light and the first light flux including the first fluorescence from the observation target irradiated with the first narrowband light to the first imaging element, and guides the second light flux including the second fluorescence from the observation target irradiated with the second narrowband light to the second imaging element.

35. The medical observation system according to claim 33, wherein
in a fourth mode different from the first mode and the second mode,
the control section controls the light source apparatus such that the broadband light and the third narrowband light are emitted to the observation target, and
the optical element guides the first light flux including reflected light from the observation target irradiated with the broadband light to the first imaging element, and guides the second light flux including third fluorescence from the observation target irradiated with the third narrowband light to the second imaging element.

36. The medical observation system according to claim 33, wherein
the light source apparatus emits third narrowband light that excites a third substance emitting third fluorescence in a wavelength band that is not included in the first wavelength band and is at least partially different from a wavelength band of the second fluorescence,
in a fourth mode different from the first mode and the second mode,
the control section controls the light source apparatus such that the broadband light and the third narrowband light are emitted to the observation target, and
the optical element guides the first light flux including reflected light from the observation target irradiated with the broadband light to the first imaging element, and guides the third light flux including the third fluorescence from the observation target irradiated with the third narrowband light to the third imaging element.

37. The medical observation system according to claim 33, further comprising
an image generation section that generates an image on a basis of an image signal from the imaging section, wherein
the control section controls the imaging section and the image generation section,
the light source apparatus emits third narrowband light that excites a third substance emitting third fluorescence in a wavelength band that is not included in the first wavelength band and at least partially coincides with the wavelength band of the second fluorescence,
in a fifth mode different from the first mode and the second mode,
the control section controls the light source apparatus such that the broadband light is emitted to the observation target and the second narrowband light and the third narrowband light are emitted to the observation target in a time division manner,
the optical element guides the first light flux including reflected light from the observation target irradiated with the broadband light to the first imaging element, sequentially guides the second light flux including the second fluorescence from the observation target irradiated with the second narrowband light and the second light flux including the third fluorescence from the observation target irradiated with the third narrowband light to the second imaging element, and sequentially guides the third light flux including the second fluorescence from the observation target irradiated with the second narrowband light and the third light flux including the third fluorescence from the observation target irradiated with the third narrowband light to the third imaging element, and
the control section controls the imaging section and the image generation section such that an image based on reflected light is generated on a basis of an image signal output from the first imaging element that has received the first light flux including reflected light, an image based on the second fluorescence is generated on a basis of an image signal output from the second imaging element that has received the second light flux including the second fluorescence, and image data of the third fluorescence is generated on a basis of an image signal output from the third imaging element that has received the third light flux including the third fluorescence.

38. The medical observation system according to claim 33, further comprising
an image generation section that generates an image on a basis of an image signal from the imaging section, wherein
the control section controls the imaging section and the image generation section,
the light source apparatus emits third narrowband light that excites a third substance emitting third fluorescence in a wavelength band that is not included in the first wavelength band and at least partially coincides with the wavelength band of the second fluorescence,
in a sixth mode different from the first mode and the second mode,
the control section controls the light source apparatus such that the broadband light and the first narrowband light are emitted to the observation target in a time division manner and the second narrowband light and the third narrowband light are emitted to the observation target in a time division manner,
the optical element sequentially guides the first light flux including the reflected light from the observation target irradiated with the broadband light and the first light flux including the first fluorescence from the observation target irradiated with the first narrowband light to the first imaging element, sequentially guides the second light flux including the second fluorescence from the observation target irradiated with the second narrowband light and the second light flux including the third fluorescence from the observation target irradiated with the third narrowband light to the second imaging element, and sequentially guides the third light flux including the second fluorescence from the observation target irradiated with the second narrowband light and the third light flux including the third fluorescence from the observation target irradiated with the third narrowband light to the third imaging element, and
the control section controls the imaging section and the image generation section such that an image based on reflected light is generated on a basis of an image signal output from the first imaging element that has received the first light flux including reflected light, an image based on the first fluorescence is generated on a basis of an image signal output from the first imaging element that has received the first light flux including the first fluorescence, an image based on the second fluorescence is generated on a basis of an image signal output from the second imaging element that has received the second light flux including the second fluorescence, and image data of the third fluorescence is generated on a basis of an image signal output from the third imaging element that has received the third light flux including the third fluorescence.

39. The medical observation system according to claim 23, wherein
the first imaging element and the second imaging element include a color filter.

40. The medical observation system according to claim 23, wherein
the third imaging element does not have a color filter.

41. The medical observation system according to claim 39, wherein
light in the wavelength band of the second fluorescence is partially, substantially, or completely suppressed in the first light flux,
light in the wavelength band of the second fluorescence is partially, substantially, or completely suppressed in the second light flux, and
light in the first wavelength band is partially, substantially, or completely suppressed in the third light flux.

42. The medical observation system according to claim 41, further comprising
an image generation section that generates an image on a basis of an image signal from the imaging section, wherein
the control section controls the imaging section and the image generation section,
in the first mode,
the optical element sequentially guides the first light flux including the reflected light from the observation target irradiated with the broadband light and the first light flux including the first fluorescence from the observation target irradiated with the first narrowband light to the first imaging element, and sequentially guides the second light flux including the reflected light from the observation target irradiated with the broadband light and the second light flux including the first fluorescence from the observation target irradiated with the first narrowband light to the second imaging element,
the control section is configured to:
control the imaging section and the image generation section such that an image based on reflected light is generated on a basis of one or both of an image signal output from the first imaging element that has received the first light flux including reflected light and an image signal output from the second imaging element that has received the second light flux including reflected light; and
control the imaging section and the image generation section such that an image based on the first fluorescence is generated on a basis of an image signal output from the second imaging element that has received the second light flux including the first fluorescence,
in the second mode,
the optical element guides the first light flux including reflected light from the observation target irradiated with the broadband light to the first imaging element, guides the second light flux including reflected light from the observation target irradiated with the broadband light to the second imaging element, and guides the third light flux including the second fluorescence from the observation target irradiated with the second narrowband light to the third imaging element, and
the control section is configured to:
control the imaging section and the image generation section such that an image based on reflected light is generated on a basis of one or both of an image signal output from the first imaging element that has received the first light flux including reflected light and an image signal output from the second imaging element that has received the second light flux including reflected light; and
control the imaging section and the image generation section such that an image based on the second fluorescence is generated on a basis of an image signal output from the third imaging element that has received the third light flux including the second fluorescence.

43. The medical observation system according to claim 39, further comprising
an image generation section that generates an image on a basis of an image signal from the imaging section, wherein
the control section controls the imaging section and the image generation section,
in a third mode different from the first mode and the second mode,
the control section controls the light source apparatus such that the broadband light and the first narrowband light are emitted to the observation target in a time division manner and the second narrowband light is emitted to the observation target,
the optical element sequentially guides the first light flux including the reflected light from the observation target irradiated with the broadband light and the first light flux including the first fluorescence from the observation target irradiated with the first narrowband light to the first imaging element, sequentially guides the second light flux including the reflected light from the observation target irradiated with the broadband light and the second light flux including the first fluorescence from the observation target irradiated with the first narrowband light to the second imaging element, and guides the third light flux including the second fluorescence from the observation target irradiated with the second narrowband light to the third imaging element, and
the control section is configured to:
control the imaging section and the image generation section such that an image based on reflected light is generated on a basis of one or both of an image signal output from the first imaging element that has received the first light flux including reflected light and an image signal output from the second imaging element that has received the second light flux including reflected light;
control the imaging section and the image generation section such that an image based on the first fluorescence is generated on a basis of an image signal output from the second imaging element that has received the second light flux including the first fluorescence; and
control the imaging section and the image generation section such that an image based on the second fluorescence is generated on a basis of an image signal output from the third imaging element that has received the third light flux including the second fluorescence.

44. The medical observation system according to claim 1, wherein the first wavelength band is included in a visible light wavelength band.

45. The medical observation system according to claim 1, wherein a wavelength of the second fluorescence is included in an invisible light wavelength band.

46. The medical observation system according to claim 2, wherein the first imaging element has a higher resolution than the second imaging element.

47. The medical observation system according to claim 1, further comprising a filter element that partially, substantially, or completely suppresses light in a wavelength band of the first narrowband light.

48. The medical observation system according to claim 1, further comprising a filter element that partially, substantially, or completely suppresses light in a wavelength band of the second narrowband light.

49. The medical observation system according to claim 21, further comprising
an instruction acceptance section that accepts an instruction from a user, wherein
the control section determines whether to use one or both of the image signal output from the first imaging element and the image signal output from the second imaging element for generation of the image based on the reflected light on a basis of the instruction from the user received by the instruction receiving section.

50. The medical observation system according to claim 43, further comprising
an instruction acceptance section that accepts an instruction from a user, wherein
the control section determines whether to use one or both of the image signal output from the first imaging element and the image signal output from the second imaging element for generation of the image based on the reflected light on a basis of the instruction from the user received by the instruction receiving section.

51. The medical observation system according to claim 1, further comprising
an image generation section that generates an image on a basis of an image signal from an imaging section that captures an image of the observation target, wherein
in a case where a mode is switched from one of the first mode and the second mode to another mode under control of the control section, the image generation section uses at least some of parameters used in the one mode while maintaining the another mode.

52. The medical observation system according to claim 51, wherein
the parameter is a parameter related to at least one of a white balance, a color tone, or a color mode.

53. A medical observation method comprising:
a step of emitting, from a light source apparatus, at least one of broadband light in a first wavelength band, first narrowband light that excites a first substance that emits first fluorescence in a wavelength band included in the first wavelength band, or second narrowband light that excites a second substance that emits second fluorescence in a wavelength band not included in the first wavelength band, wherein
in a first mode, the broadband light and the first narrowband light are emitted from the light source apparatus such that the broadband light and the first narrowband light are emitted to an observation target in a time division manner, and
in a second mode different from the first mode, the broadband light and the second narrowband light are emitted from the light source apparatus such that the observation target is irradiated with the broadband light and the second narrowband light.
